# EUROPEAN PATENT APPLICATION

(11) **EP 3 933 401 A2**
(43) Date of publication of application: **05.01.2022**
(21) Application number: 21167425.4
(22) Date of filing: 26.03.2014
(51) Int. Cl.: G01N 33/00, G01N 33/53

(54) **USE OF BIOMARKERS FOR ASSESSING TREATMENT OF GASTROINTESTINAL INFLAMMATORY DISORDERS WITH BETA7 INTEGRIN ANTAGONISTS**

(30) Priority: 27.03.2013 US 201361805860 P; 11.12.2013 US 201361914619 P
(62) Divisional of application: 18191598.4
(71) Applicant: F. Hoffmann-La Roche AG, 4070 Basel (CH)
(72) Inventor: DIEHL, Lauri, South San Francisco, 94080 (US); KEIR, Mary, South San Francisco, 94080 (US); TANG, Meina Tao, South San Francisco, 94080 (US); WEI, Xiaohui, South San Francisco, 94080 (US); WILLIAMS, Marna B., Palo Alto, 94306 (US)
(74) Representative: Bernard, Guillaume

(57) **Abstract**

Methods of assessing or monitoring the effect, efficacy, responsiveness to treatment, and/or determining a dose or dosing regimen of therapeutic agents, such as integrin beta7 antagonists, for the treatment of gastrointestinal inflammatory disorders are provided. In certain aspects, methods of using integrin beta7 subunit-containing receptor occupancy by the integrin beta7 antagonist on colonic lymphocytes as an indicator ("biomarker") of the effect, efficacy, or responsiveness to treatment, and/or as a means to determine dosing or dosing regimens of therapeutic agents such as beta7 integrin antagonists for the treatment of gastrointestinal inflammatory disorders are provided. In certain aspects, methods of assessing the effect, efficacy, or responsiveness to beta7 integrin antagonist treatment by measuring gene expression levels of one or more integrin receptor ligands, lymphocyte genes, cytokine genes, or the number of alphaE-positive cells in intestinal crypt epithelium are provided.

## Description

### CROSS REFERENCE TO RELATED APPLICATIONS

This application claims the benefit of priority of provisional U.S. Application No. 61/914,619 filed December 11, 2013 and provisional U.S. Application No. 61/805,860 filed March 27, 2013, both of which are hereby incorporated by reference in their entirety.

### SEQUENCE LISTING

The instant application contains a Sequence Listing which has been submitted via EFS-Web and is hereby incorporated by reference in its entirety. Said ASCII copy, created on March 17, 2014, is named P5599RlWO_PCTSequenceListing.txt and is 20,255 bytes in size.

### FIELD

Methods of assessing or monitoring the effect, efficacy, responsiveness to treatment, and/or determining a dose or dosing regimen of therapeutic agents, such as integrin beta7 antagonists, for the treatment of gastrointestinal inflammatory disorders, e.g., inflammatory bowel disease, are provided. In certain aspects, methods of using integrin beta7 subunit-containing receptor occupancy by the integrin beta7 antagonist on colonic lymphocytes as an indicator ("biomarker") of the effect, efficacy, or responsiveness to treatment, and/or as a means to determine dosing or dosing regimens of therapeutic agents such as beta7 integrin antagonists for the treatment of gastrointestinal inflammatory disorders are provided. In certain aspects, methods of assessing the effect, efficacy, or responsiveness to beta7 integrin antagonist treatment by measuring gene expression levels of one or more integrin receptor ligands, lymphocyte genes, cytokine genes, or the number of alphaE-positive cells in intestinal crypt epithelium are provided.

### BACKGROUND

Inflammatory bowel disease (IBD) is a chronic inflammatory autoimmune condition of the gastrointestinal (GI) tract, which presents clinically as either ulcerative colitis (UC) or Crohn's disease (CD). CD is a chronic transmural inflammatory disease with the potential to affect any part of the entire GI tract, and UC is a mucosal inflammation of the colon. Both conditions are characterized clinically by frequent bowel motions, malnutrition, and dehydration, with disruption in the activities of daily living. CD is frequently complicated by the development of malabsorption, strictures, and fistulae and may require repeated surgery. UC, less frequently, may be complicated by severe bloody diarrhea and toxic megacolon, also requiring surgery. Both IBD conditions are associated with an increased risk for malignancy of the GI tract. The etiology of IBD is complex, and many aspects of the pathogenesis remain unclear.

The treatment of moderate to severe IBD poses significant challenges to treating physicians, because conventional therapy with corticosteroids and immunomodulator therapy (e.g., azathioprine, 6 mercaptopurine, and methotrexate) is associated with side effects and intolerance and has not shown proven benefit in maintenance therapy (steroids). Monoclonal antibodies targeting tumor necrosis factor alpha (TNF-α), such as infliximab (a chimeric antibody) and adalimumab (a fully human antibody), are currently used in the management of CD. Infliximab has also shown efficacy and has been approved for use in UC. However, approximately 10%-20% of patients with CD are primary nonresponders to anti TNF therapy, and another ∼20%-30% of CD patients lose response over time (Schnitzler et al., Gut 58:492-500 (2009)). Other adverse events (AEs) associated with anti TNFs include elevated rates of bacterial infection, including tuberculosis, and, more rarely, lymphoma and demyelination (Chang et al., Nat Clin Pract Gastroenterol Hepatology 3:220 (2006); Hoentjen et al., World J. Gastroenterol. 15(17):2067 (2009)). No currently available therapy achieves sustained remission in more than 20%-30% of IBD patients with chronic disease (Hanauer et al., Lancet 359:1541-49 (2002); Sandborn et al., N Engl J Med 353:1912-25 (2005)). In addition, most patients do not achieve sustained steroid-free remission and mucosal healing, clinical outcomes that correlate with true disease modification. Therefore, there is a need to develop more targeted therapy in IBD that is optimized for chronic use: an improved safety profile with sustained remission, particularly steroid-free remission and prevention of long-term complications in a greater proportion of patients, including those patients who either never respond to an anti TNF therapeutic agent or lose response over time.

The integrins are alpha/beta heterodimeric cell surface glycoprotein receptors that play a role in numerous cellular processes including leukocyte adhesion, signaling, proliferation, and migration, as well as in gene regulation (Hynes, R. O., Cell, 1992, 69:11-25; and Hemler, M. E., Annu. Rev. Immunol., 1990, 8:365-368). They are composed of two heterodimeric, non-covalently interacting α and β transmembrane subunits that bind specifically to distinct cell adhesion molecules (CAMs) on endothelia, epithelia, and extracellular matrix proteins. In this manner, integrins can function as tissue-specific cell adhesion receptors aiding in the recruitment of leukocytes from blood into nearly all tissue sites in a highly regulated manner, playing a role in the homing of leukocytes to normal tissue and to sites of inflammation (von Andrian et al., N Engl J Med 343:1020-34 (2000)). In the immune system, integrins are involved in leukocyte trafficking, adhesion and infiltration during inflammatory processes (Nakajima, H. et al., J. Exp. Med., 1994, 179:1145-1154). Differential expression of integrins regulates the adhesive properties of cells and different integrins are involved in different inflammatory responses. (Butcher, E. C. et al., Science, 1996, 272:60-66). The beta7 containing integrins (*i.e.,* alpha4beta7 and alphaEbeta7) are expressed primarily on monocytes, lymphocytes, eosinophils, basophils, and macrophages but not on neutrophils (Elices, M. J. et al., Cell, 1990, 60:577-584).

The α4β7 integrin is a leukocyte-homing receptor that is important in the migration of cells to the intestinal mucosa and associated lymphoid tissues, such as Peyer's patches in the small intestine, lymphoid follicles in the large intestine, and mesenteric lymph nodes. In the gut, leukocyte rolling and firm adhesion to the mucosal endothelium is initiated by signals from chemokines and is mediated via mucosal addressin cell adhesion molecule (MAdCAM)-1-associated sialyl Lewis X. Chemokine signaling induces the α4β7 integrin to undergo a change from low to high MAdCAM-1 binding affinity. The leukocyte then arrests and begins the process of extravasation through the vascular endothelium to underlying tissue. This extravasation process is believed to occur in both the normal immune cell recirculation state and in inflammatory conditions (von Andrian et al., *supra*)*.* The numbers of α4β7⁺ cells in infiltrates and the expression of the ligand MAdCAM-1 are higher at sites of chronic inflammation such as in the intestinal tract of patients with UC or CD (Briskin et al., Am J Pathol 151:97-110 (1997); Souza et al., Gut 45:856-63 (1999)). α4β7 binds preferentially to high endothelial venules expressing MAdCAM-1 and vascular cell adhesion molecule (VCAM)-1, as well as to the extracellular matrix molecule fibronectin fragment CS-1 (Chan et al., J Biol Chem 267:8366-70 (1992); Ruegg et al., J Cell Biol 17:179-89 (1992); Berlin et al., Cell 74:185-95 (1993)). Together with constitutively expressed MAdCAM-1 in gut mucosal vessels, the α4β7 integrin plays a selective role in leukocyte gut tropism but does not seem to contribute to homing of leukocytes to the peripheral tissue or the CNS. Instead, peripheral lymphoid trafficking has been associated with α4β1 interaction with VCAM-1 (Yednock et al., Nature 356:63-6 (1992); Rice et al., Neurology 64:1336-42 (2005)).

Another member of the β7 integrin family, expressed exclusively on T lymphocytes and associated with mucosal tissues, is the αEβ7 integrin, otherwise known as CD103. The αEβ7 integrin binds selectively to E-cadherin on epithelial cells and has been proposed to play a role in the retention of T cells in the mucosal tissue in the intraepithelial lymphocyte compartment (Cepek et al., J Immunol 150:3459-70 (1993); Karecla et al. Eur J Immunol 25:852-6 (1995)). The αEβ7⁺ cells in the lamina propria have been reported to exhibit cytotoxicity against stressed or infected epithelial cells (Hadley et al., J Immunol 159:3748-56 (1997); Buri et al., J Pathol 206:178-85 (2005)). The expression of αEβ7 is increased in CD (Elewaut et al., Acta Gastroenterol Belg 61:288-94 (1998); Oshitani et al., Int J Mol Med 12:715-9 (2003)), and anti-αEβ7 antibody treatment has been reported to attenuate experimental colitis in mice, implicating a role for αEβ7⁺ lymphocytes in experimental models of IBD (Ludviksson et al., J Immunol 162:4975-82 (1999)).

Administration of monoclonal antibodies against alphaE beta7 reportedly prevents and ameliorates immunization induced colitis in IL-2 ^{-/-} mice, suggesting that the onset and maintenance of inflammatory bowel disease depends on colonic localization of lamina propria CD4⁺ lymphocytes expressing alphaEbeta7 (Ludviksson et al., J Immunol. 1999, 162(8):4975-82). An anti-α4 antibody (natalizumab) reportedly has efficacy in treatment of patients with CD (Sandborn et al., N Engl J Med 2005;353:1912-25) and an anti-α4β7 antibody (MLN-02, MLN0002, vedolizumab) reportedly is effective in patients with UC (Feagan et al., N Engl J Med 2005;352:2499-507). These findings validate α4β7 as a therapeutic target and support the idea that the interaction between α4β7 and MAdCAM-1 mediates the pathogenesis of IBD. Thus, antagonists of beta7 integrin are of great potential as a therapeutic agent in treating IBD.

Humanized monoclonal antibodies targeted against the β7 integrin subunit have been described previously. See, e.g., Intn'l Patent Pub. No. WO2006/026759. One such antibody, rhuMAb Beta7 (etrolizumab) is derived from the rat anti-mouse/human monoclonal antibody FIB504 (Andrew et al. 1994). It was engineered to include human IgG1-heavy chain and κ1-light chain frameworks. Intn'l Patent Pub. No. WO2006/026759.

RhuMAb Beta7 binds α4β7 (Holzmann et al., Cell 56:37-46 (1989); Hu et al., Proc Natl Acad Sci USA 89:8254-8 (1992)) and αEβ7 (Cepek et al., J Immunol 150:3459-70 (1993)), which regulate trafficking and retention of lymphocyte subsets, respectively, in the intestinal mucosa. Clinical studies have demonstrated the efficacy of an anti-α4 antibody (natalizumab) for the treatment of CD (Sandborn et al., N Engl J Med 353:1912-25 (2005)), and encouraging results have been reported for anti α4β7 antibody (LDP02/MLN02/MLN0002/vedolizumab) in the treatment of UC (Feagan et al., N Engl J Med 352:2499-507 (2005)). These findings help to validate α4β7 as a potential therapeutic target and support the hypothesis that the interaction between α4β7 and mucosal addressin cell adhesion molecule 1 (MAdCAM 1) contributes to the pathogenesis of inflammatory bowel disease (IBD).

Unlike natalizumab, which binds α4 and thus binds both α4β1 and α4β7, rhuMAb Beta7 binds specifically to the β7 subunit of α4β7 and αEβ7 and does not bind to α4 or β1 integrin individual subunits. This was demonstrated by the inability of the antibody to inhibit adhesion of v4β1+α4β7- Ramos cells to vascular cell adhesion molecule 1 (VCAM 1) at concentrations as high as 100 nM. Importantly, this characteristic of rhuMAb Beta7 indicates selectivity: T cell subsets expressing α4β1 but not β7 should not be directly affected by rhuMAb Beta7.

Support for the gut-specific effects of rhuMAb Beta7 on leukocyte homing comes from several in vivo nonclinical studies. In severe combined immunodeficient (SCID) mice reconstituted with CD45RB^{high}CD4+ T cells (an animal model of colitis), rhuMAb Beta7 blocked radiolabeled lymphocyte homing to the inflamed colon but did not block homing to the spleen, a peripheral lymphoid organ. See, e.g., Intn'l Patent Pub. No. WO2006/026759. In addition, the rat-mouse chimeric anti-murine β7 (anti β7, muFIB504) was unable to reduce the histologic degree of central nervous system (CNS) inflammation or improve disease survival in myelin basic protein T cell receptor (MBP-TCR) transgenic mice with experimental autoimmune encephalitis (EAE), an animal model of multiple sclerosis. *Id.* Furthermore, in two safety studies in cynomolgus monkeys, rhuMAb Beta7 induced a moderate increase in peripheral blood lymphocyte numbers that was largely due to a marked (approximately three- to six-fold) increase in CD45RA⁻β7^{high} peripheral blood T cells, a subset that is phenotypically similar to gut-homing memory/effector T cells in humans. See, e.g., Intn'l Patent Pub. No. WO2009/140684; Stefanich et al., Br. J. Pharmacol. 162:1855-1870 (2011). In contrast, rhuMAb Beta7 had minimal to no effect on the number of CD45RA+β7intermediate peripheral blood T cells, a subset that is phenotypically similar to naive T cells in humans, and no effect on the number of CD45RA⁻β7^{low} peripheral blood T cells, a subset that is phenotypically similar to peripheral homing memory/effector T cells in humans, confirming the specificity of rhuMAb Beta7 for the gut homing lymphocyte subpopulation. Intn'l Patent Pub. No. WO2009/140684; Stefanich et al., Br. J. Pharmacol. 162:1855-1870 (2011).

To design therapies with a desired effect and/or efficacy, it is important to assess a patient's responsiveness to treatment with a therapeutic agent, such as a beta7 integrin antagonist. It is also important to determine optimal doses and dosing regimens of beta7 integrin antagonists that will provide or are likely to provide efficacy. Therefore, it is desirable to develop a biomarker that can be used to accurately track or monitor the responsiveness of a patient to treatment with a therapeutic agent. Such a biomarker would be particularly useful for designing effective treatment and dosing regimens for human patients in clinical trial studies and for disease treatment.

The invention described herein meets certain of the above-described needs and provides other benefits.

All references cited herein, including patent applications and publications, are incorporated by reference in their entirety for any purpose.

### SUMMARY

The methods of the invention are based, at least in part, on the discovery that receptor occupancy and cell surface expression of integrin beta7-subunit containing receptors, including αEβ7, on lymphocytes obtained from colon tissue of integrin beta7 antagonist (e.g., anti-beta7 antibody)-treated patients are capable of being assessed by flow cytometry methods. In addition, surprisingly, anti-beta7 antibody serum concentrations that were capable of saturating lymphocyte beta7 receptors in the periphery of treated patients were essentially the same as the anti-beta7 antibody serum concentrations that were capable of saturating lymphocyte beta7 receptors at the site of disease (in the colon). Accordingly, beta7 receptor occupancy in the peripheral blood is a surrogate indicator of beta7 receptor occupancy in colonic tissue. Additionally, the methods of the invention are based, at least in part, on the discovery that levels of gene expression of integrin receptor ligands, lymphocyte genes, and cytokine genes, as well as the numbers of alphaE-positive cells in intestinal crypt epithelium change after treatment with an integrin beta7 antagonist.

In one aspect, methods of determining or monitoring the efficacy or of aiding in determining or monitoring the efficacy of an integrin beta7 antagonist for treatment of a gastrointestinal inflammatory disorder in a patient are provided. In certain embodiments, the methods comprise comparing the amount of a biomarker in a sample obtained from the patient after or during treatment with the antagonist, to an amount of the biomarker in a sample obtained from the patient before the treatment, where a change in the amount of the biomarker after or during the treatment, as compared to before the treatment, is indicative of the efficacy of the antagonist for treatment of the gastrointestinal disorder in the patient, and where the biomarker is integrin beta7 subunit-containing receptor occupancy by the antagonist on colonic lymphocytes. In certain embodiments, the biomarker is selected from gene expression levels of one or more integrin receptor ligands, gene expression levels of one or more lymphocyte genes, gene expression levels of one or more cytokines, and the number of alphaE-positive cells in intestinal crypt epithelium. In certain embodiments, combinations of the above biomarkers are assessed. In certain embodiments, the afore-mentioned methods using one or more of the biomarkers selected from integrin beta7 subunit-containing receptor occupancy by the antagonist on colonic lymphocytes, gene expression levels of one or more integrin receptor ligands, gene expression levels of one or more lymphocyte genes, gene expression levels of one or more cytokines, and the number of alphaE-positive cells in intestinal crypt epithelium are combined with one or more additional biomarkers of efficacy. In certain embodiments, the one or more additional biomarkers of efficacy are one or more clinical biomarkers selected from clinical remission at week 6, clinical remission at week 10, clinical response at week 6, clinical response at week 10, endoscopy score and rectal bleeding score of 0 at week 6, endoscopy and rectal bleeding score of 0 at week 10, and time to flare of UC after achieving response or remission. In certain embodiments, the integrin beta7 subunit-containing receptor is αEβ7 receptor or α4β7 receptor. In one embodiment, the integrin beta7 subunit-containing receptor occupancy on colonic lymphocytes is determined by measuring integrin beta7 subunit-containing receptor occupancy on peripheral blood lymphocytes, where the integrin beta7 subunit-containing receptor occupancy on peripheral blood lymphocytes was previously determined to be essentially the same as the integrin beta7 subunit-containing receptor occupancy on colonic lymphocytes. In certain embodiments, the occupancy of the integrin beta7 subunit-containing receptor is determined by a method comprising incubating the lymphocytes with labeled anti-beta7 antibody, where the labeled anti-beta7 antibody binds to the same epitope as the integrin beta7 antagonist, washing the lymphocytes, and measuring the percentage of labeled lymphocytes by flow cytometry. In certain embodiments, the label is selected from fluorescein isothiocyanate (FITC), rhodamine, phycoerythrin (PE), allophycocyanin (APC), peridinin chlorophyll protein (PerCP), PE-Cy7, APC-Cy7 and APC-H7. In one embodiment, the integrin beta7 antagonist is etrolizumab and the labeled anti-beta7 antibody is etrolizumab or FIB504. In some embodiments, the gene expression level of the integrin receptor ligand, MadCAM-1, is measured. In some embodiments, the level of the integrin receptor ligand, E-Cadherin, is measured. In some embodiments, the level of gene expression of one or more lymphocyte genes selected from CD19, CD8, and CD3epsilon is measured. In some embodiments, the level of gene expression of one or more cytokines selected from IL,-1β, IL-6, IL-12-p40, IL-17A, IL-17-F, IL-23A, IPNγ and TNFα is measured. In certain embodiments, the level of gene expression is measured in colonic biopsy tissue. In certain embodiments, the level of gene expression is measured by qPCR. In certain embodiments, the change in the biomarker is an increase or a decrease. In certain embodiments, the biomarker is measured within 100 days after receiving a first dose of the antagonist. In certain embodiments, the biomarker is measured at day 43 and at day 71 after receiving a first dose of the antagonist or the biomarker is measured at week 6 and at week 10 after receiving a first dose of the antagonist. In one embodiment, the gastrointestinal inflammatory disorder is an inflammatory bowel disease. Exemplary inflammatory bowel diseases include ulcerative colitis and Crohn's disease. In certain embodiments, the patient is human.

In another aspect, methods of determining or monitoring the responsiveness or of aiding in determining or monitoring the responsiveness of a patient having a gastrointestinal inflammatory disorder to treatment with an integrin beta7 antagonist are provided. In certain embodiments, the methods comprise comparing the amount of a biomarker in a sample obtained from the patient after or during treatment with the antagonist, to the amount of the biomarker in a sample obtained from the patient before the treatment, where a change in the amount of the biomarker after or during the treatment, as compared to before the treatment is indicative of the responsiveness of the patient to treatment with the antagonist, and where the biomarker is integrin beta7 subunit-containing receptor occupancy by the antagonist on colonic lymphocytes. In certain embodiments, the biomarker is selected from gene expression levels of one or more integrin receptor ligands, gene expression levels of one or more lymphocyte genes, gene expression levels of one or more cytokines, and the number of alphaE-positive cells in intestinal crypt epithelium. In certain embodiments, combinations of the above biomarkers are assessed. In certain embodiments, the afore-mentioned methods using one or more of the biomarkers selected from integrin beta7 subunit-containing receptor occupancy by the antagonist on colonic lymphocytes, gene expression levels of one or more integrin receptor ligands, gene expression levels of one or more lymphocyte genes, gene expression levels of one or more cytokines, and the number of alphaE-positive cells in intestinal crypt epithelium are combined with one or more additional biomarkers of efficacy. In certain embodiments, the one or more additional biomarkers of efficacy are one or more clinical biomarkers selected from clinical remission at week 6, clinical remission at week 10, clinical response at week 6, clinical response at week 10, endoscopy score and rectal bleeding score of 0 at week 6, endoscopy and rectal bleeding score of 0 at week 10, and time to flare of UC after achieving response or remission. In certain embodiments, the integrin beta7 subunit-containing receptor is αEβ7 receptor or α4β7 receptor. In one embodiment, the integrin beta7 subunit-containing receptor occupancy on colonic lymphocytes is determined by measuring integrin beta7 subunit-containing receptor occupancy on peripheral blood lymphocytes, where the integrin beta7 subunit-containing receptor occupancy on peripheral blood lymphocytes was previously determined to be essentially the same as the integrin beta7 subunit-containing receptor occupancy on colonic lymphocytes. In certain embodiments, the occupancy of the integrin beta7 subunit-containing receptor is determined by a method comprising incubating the lymphocytes with labeled anti-beta7 antibody, where the labeled anti-beta7 antibody binds to the same epitope as the integrin beta7 antagonist, washing the lymphocytes, and measuring the percentage of labeled lymphocytes by flow cytometry. In certain embodiments, the label is selected from fluorescein isothiocyanate (FITC), rhodamine, phycoerythrin (PE), allophycocyanin (APC), peridinin chlorophyll protein (PerCP), PE-Cy7, APC-Cy7 and APC-H7. In one embodiment, the integrin beta7 antagonist is etrolizumab and the labeled anti-beta7 antibody is etrolizumab or FIB504. In some embodiments, the gene expression level of the integrin receptor ligand, MadCAM-1, is measured. In some embodiments, the level of the integrin receptor ligand, E-Cadherin, is measured. In some embodiments, the level of gene expression of one or more lymphocyte genes selected from CD19, CD8, and CD3epsilon is measured. In some embodiments, the level of gene expression of one or more cytokines selected from IL,-1β, IL-6, IL-12-p40, IL-17A, IL-17-F, IL-23A, IPNγ and TNFα is measured. In certain embodiments, the level of gene expression is measured in colonic biopsy tissue. In certain embodiments, the level of gene expression is measured by qPCR. In certain embodiments, the change in the biomarker is an increase or a decrease. In certain embodiments, the biomarker is measured within 100 days after receiving a first dose of the antagonist. In certain embodiments, the biomarker is measured at day 43 and at day 71 after receiving a first dose of the antagonist or the biomarker is measured at week 6 and at week 10 after receiving a first dose of the antagonist. In one embodiment, the gastrointestinal inflammatory disorder is an inflammatory bowel disease. Exemplary inflammatory bowel diseases include ulcerative colitis and Crohn's disease. In certain embodiments, the patient is human.

In yet another aspect, methods of determining or monitoring the efficacy of an integrin beta7 antagonist for treatment of a gastrointestinal inflammatory disorder in an antagonist-treated patient in a placebo-controlled clinical trial are provided. In certain embodiments, the methods comprise comparing the amount of a biomarker in a sample obtained from the patient after or during treatment with the antagonist, to an amount of the biomarker in a sample obtained from a placebo-treated patient, where a change in the amount of the biomarker in the antagonist-treated patient after or during treatment, as compared to the amount of the biomarker in the placebo-treated patient, is indicative of the efficacy of the antagonist for treatment of the gastrointestinal disorder in the antagonist-treated patient, and where the biomarker is integrin beta7 subunit-containing receptor occupancy by the antagonist on colonic lymphocytes. In certain embodiments, the biomarker is selected from gene expression levels of one or more integrin receptor ligands, gene expression levels of one or more lymphocyte genes, gene expression levels of one or more cytokines, and the number of alphaE-positive cells in intestinal crypt epithelium. In certain embodiments, the integrin beta7 subunit-containing receptor is αEβ7 receptor or α4β7 receptor. In one embodiment, the integrin beta7 subunit-containing receptor occupancy on colonic lymphocytes is determined by measuring integrin beta7 subunit-containing receptor occupancy on peripheral blood lymphocytes, where the integrin beta7 subunit-containing receptor occupancy on peripheral blood lymphocytes was previously determined to be essentially the same as the integrin beta7 subunit-containing receptor occupancy on colonic lymphocytes. In certain embodiments, the occupancy of the integrin beta7 subunit-containing receptor is determined by a method comprising incubating the lymphocytes with labeled anti-beta7 antibody, where the labeled anti-beta7 antibody binds to the same epitope as the integrin beta7 antagonist, washing the lymphocytes, and measuring the percentage of labeled lymphocytes by flow cytometry. In certain embodiments, the label is selected from fluorescein isothiocyanate (FITC), rhodamine, phycoerythrin (PE), allophycocyanin (APC), peridinin chlorophyll protein (PerCP), PE-Cy7, APC-Cy7 and APC-H7. In one embodiment, the integrin beta7 antagonist is etrolizumab and the labeled anti-beta7 antibody is etrolizumab or FIB504. In some embodiments, the gene expression level of the integrin receptor ligand, MadCAM-1, is measured. In some embodiments, the level of the integrin receptor ligand, E-Cadherin, is measured. In some embodiments, the level of gene expression of one or more lymphocyte genes selected from CD19, CD8, and CD3epsilon is measured. In some embodiments, the level of gene expression of one or more cytokines selected from IL,-1β, IL-6, IL-12-p40, IL-17A, IL-17-F, IL-23A, IPNγ and TNFα is measured. In certain embodiments, the level of gene expression is measured in colonic biopsy tissue. In certain embodiments, the level of gene expression is measured by qPCR. In certain embodiments, the change in the biomarker is an increase or a decrease. In certain embodiments, the biomarker is measured within 100 days after receiving a first dose of the antagonist. In certain embodiments, the biomarker is measured at day 43 and at day 71 after receiving a first dose of the antagonist or the biomarker is measured at week 6 and at week 10 after receiving a first dose of the antagonist. In certain embodiments, combinations of the above biomarkers are assessed. In certain embodiments, the methods further comprise assessing one or more clinical biomarkers of efficacy selected from clinical remission at week 6, clinical remission at week 10, clinical response at week 6, clinical response at week 10, endoscopy score and rectal bleeding score of 0 at week 6, endoscopy and rectal bleeding score of 0 at week 10, and time to flare of UC after achieving response or remission. In one embodiment, the gastrointestinal inflammatory disorder is an inflammatory bowel disease. Exemplary inflammatory bowel diseases include ulcerative colitis and Crohn's disease. In certain embodiments, the patient is human.

In yet still another aspect, methods of determining or monitoring the responsiveness of a patient having a gastrointestinal inflammatory disorder to treatment with an integrin beta7 antagonist, where the patient is in a placebo-controlled clinical trial are provided. In certain embodiments, the methods comprise comparing the amount of a biomarker in a sample obtained from the patient after or during treatment with the antagonist, to an amount of the biomarker in a sample obtained from a placebo-treated patient, wherein a change in the amount of the biomarker in the antagonist-treated patient after or during treatment, as compared to the amount of the biomarker in the placebo-treated patient, is indicative of the responsiveness of the patient to treatment with the antagonist, and wherein the biomarker is integrin beta7 subunit-containing receptor occupancy by the antagonist on colonic lymphocytes. In certain embodiments, the biomarker is selected from gene expression levels of one or more integrin receptor ligands, gene expression levels of one or more lymphocyte genes, gene expression levels of one or more cytokines, and the number of alphaE-positive cells in intestinal crypt epithelium. In certain embodiments, the integrin beta7 subunit-containing receptor is αEβ7 receptor or α4β7 receptor. In one embodiment, the integrin beta7 subunit-containing receptor occupancy on colonic lymphocytes is determined by measuring integrin beta7 subunit-containing receptor occupancy on peripheral blood lymphocytes, where the integrin beta7 subunit-containing receptor occupancy on peripheral blood lymphocytes was previously determined to be essentially the same as the integrin beta7 subunit-containing receptor occupancy on colonic lymphocytes. In certain embodiments, the occupancy of the integrin beta7 subunit-containing receptor is determined by a method comprising incubating the lymphocytes with labeled anti-beta7 antibody, where the labeled anti-beta7 antibody binds to the same epitope as the integrin beta7 antagonist, washing the lymphocytes, and measuring the percentage of labeled lymphocytes by flow cytometry. In certain embodiments, the label is selected from fluorescein isothiocyanate (FITC), rhodamine, phycoerythrin (PE), allophycocyanin (APC), peridinin chlorophyll protein (PerCP), PE-Cy7, APC-Cy7 and APC-H7. In one embodiment, the integrin beta7 antagonist is etrolizumab and the labeled anti-beta7 antibody is etrolizumab or FIB504. In some embodiments, the gene expression level of the integrin receptor ligand, MadCAM-1, is measured. In some embodiments, the level of the integrin receptor ligand, E-Cadherin, is measured. In some embodiments, the level of gene expression of one or more lymphocyte genes selected from CD19, CD8, and CD3epsilon is measured. In some embodiments, the level of gene expression of one or more cytokines selected from IL,-1β, IL-6, IL-12-p40, IL-17A, IL-17-F, IL-23A, IPNγ and TNFα is measured. In certain embodiments, the level of gene expression is measured in colonic biopsy tissue. In certain embodiments, the level of gene expression is measured by qPCR. In certain embodiments, the change in the biomarker is an increase or a decrease. In certain embodiments, the biomarker is measured within 100 days after receiving a first dose of the antagonist. In certain embodiments, the biomarker is measured at day 43 and at day 71 after receiving a first dose of the antagonist or the biomarker is measured at week 6 and at week 10 after receiving a first dose of the antagonist. In certain embodiments, combinations of the above biomarkers are assessed. In certain embodiments, the methods further comprise assessing one or more clinical biomarkers of efficacy selected from clinical remission at week 6, clinical remission at week 10, clinical response at week 6, clinical response at week 10, endoscopy score and rectal bleeding score of 0 at week 6, endoscopy and rectal bleeding score of 0 at week 10, and time to flare of UC after achieving response or remission. In one embodiment, the gastrointestinal inflammatory disorder is an inflammatory bowel disease. Exemplary inflammatory bowel diseases include ulcerative colitis and Crohn's disease. In certain embodiments, the patient is human.

In yet another aspect, methods of determining the dosing of an integrin beta7 antagonist for treatment of a gastrointestinal inflammatory disorder in a patient are provided. In certain embodiments, the methods comprise adjusting the dose of the antagonist based on a comparison of the amount of a biomarker in a sample obtained from the patient after or during treatment with a dose or dosing regimen of the antagonist, to an amount of the biomarker in a sample obtained from the patient before the treatment, where a change in the amount of the biomarker after or during the treatment, as compared to before the treatment, is indicative of the efficacy of or responsiveness to the dose or dosing regimen of the antagonist for treatment of the gastrointestinal disorder in the patient, and where the biomarker is integrin beta7 subunit-containing receptor occupancy by the antagonist on colonic lymphocytes. In certain embodiments, the integrin beta7 subunit-containing receptor is αEβ7 receptor or α4β7 receptor. In one embodiment, the integrin beta7 subunit-containing receptor occupancy on colonic lymphocytes is determined by measuring integrin beta7 subunit-containing receptor occupancy on peripheral blood lymphocytes, where the integrin beta7 subunit-containing receptor occupancy on peripheral blood lymphocytes was previously determined to be essentially the same as the integrin beta7 subunit-containing receptor occupancy on colonic lymphocytes. In certain embodiments, the occupancy of the integrin beta7 subunit-containing receptor is determined by a method comprising incubating the lymphocytes with labeled anti-beta7 antibody, where the labeled anti-beta7 antibody binds to the same epitope as the integrin beta7 antagonist, washing the lymphocytes, and measuring the percentage of labeled lymphocytes by flow cytometry. In certain embodiments, the label is selected from fluorescein isothiocyanate (FITC), rhodamine, phycoerythrin (PE), allophycocyanin (APC), peridinin chlorophyll protein (PerCP), PE-Cy7, APC-Cy7 and APC-H7. In one embodiment, the integrin beta7 antagonist is etrolizumab and the labeled anti-beta7 antibody is etrolizumab or FIB504. In one embodiment, the change in the occupancy is an increase or decrease. In one embodiment, the occupancy is measured within 100 days after receiving a first dose of the antagonist. In one embodiment, the occupancy is measured at day 43 and at day 71. In one embodiment, the gastrointestinal inflammatory disorder is an inflammatory bowel disease. Exemplary inflammatory bowel diseases include ulcerative colitis and Crohn's disease. In certain embodiments, the patient is human. In one embodiment, the antagonist is an anti-beta7 antibody and the dosing or dosing regimen determined as indicative of the efficacy of or responsiveness to the dose or dosing regimen comprises subcutaneous administration of a first loading dose of 420 mg anti-beta7 antibody followed two weeks later by subcutaneous administration of a first maintenance dose of 315 mg anti-beta7 antibody (or nominal dose of 300 mg) followed by subcutaneous administration of one or more subsequent maintenance doses of 315 mg anti-beta7 antibody (or nominal dose of 300 mg), where each subsequent maintenance dose is administered four weeks after the prior maintenance dose. In one embodiment, the dosing or dosing regimen determined as indicative of the efficacy of or responsiveness to the dose or dosing regimen comprises subcutaneous administration of 105 mg anti-beta7 antibody (or nominal dose of 100 mg) every four weeks or 50 mg anti-beta7 antibody (nominal dose) every two weeks. In one embodiment, the anti-beta7 antibody is etrolizumab.

In still yet another aspect, methods of determining the dosing regimen of an integrin beta7 antagonist for treatment of a gastrointestinal inflammatory disorder in a patient are provided. In certain embodiments, the methods comprise adjusting the dose regimen of the antagonist based on a comparison of the amount of a biomarker in a sample obtained from the patient after or during treatment with a dosing regimen of the antagonist, to an amount of the biomarker in a sample obtained from the patient before the treatment, where a change in the amount of the biomarker after or during the treatment, as compared to before the treatment, is indicative of the efficacy of or responsiveness to the dose or dosing regimen of the antagonist for treatment of the gastrointestinal disorder in the patient, and where the biomarker is integrin beta7 subunit-containing receptor occupancy by the antagonist on colonic lymphocytes. In certain embodiments, the integrin beta7 subunit-containing receptor is αEβ7 receptor or α4β7 receptor. In one embodiment, the integrin beta7 subunit-containing receptor occupancy on colonic lymphocytes is determined by measuring integrin beta7 subunit-containing receptor occupancy on peripheral blood lymphocytes, where the integrin beta7 subunit-containing receptor occupancy on peripheral blood lymphocytes was previously determined to be essentially the same as the integrin beta7 subunit-containing receptor occupancy on colonic lymphocytes. In certain embodiments, the occupancy of the integrin beta7 subunit-containing receptor is determined by a method comprising incubating the lymphocytes with labeled anti-beta7 antibody, where the labeled anti-beta7 antibody binds to the same epitope as the integrin beta7 antagonist, washing the lymphocytes, and measuring the percentage of labeled lymphocytes by flow cytometry. In certain embodiments, the label is selected from fluorescein isothiocyanate (FITC), rhodamine, phycoerythrin (PE), allophycocyanin (APC), peridinin chlorophyll protein (PerCP), PE-Cy7, APC-Cy7 and APC-H7. In one embodiment, the integrin beta7 antagonist is etrolizumab and the labeled anti-beta7 antibody is etrolizumab or FIB504. In one embodiment, the change in the occupancy is an increase or decrease. In one embodiment, the occupancy is measured within 100 days after receiving a first dose of the antagonist. In one embodiment, the occupancy is measured at day 43 and at day 71. In one embodiment, the gastrointestinal inflammatory disorder is an inflammatory bowel disease. Exemplary inflammatory bowel diseases include ulcerative colitis and Crohn's disease. In certain embodiments, the patient is human. In one embodiment, the antagonist is an anti-beta7 antibody and the dosing or dosing regimen determined as indicative of the efficacy of or responsiveness to the dose or dosing regimen comprises subcutaneous administration of a first loading dose of 420 mg anti-beta7 antibody followed two weeks later by subcutaneous administration of a first maintenance dose of 315 mg anti-beta7 antibody (or nominal dose of 300 mg) followed by subcutaneous administration of one or more subsequent maintenance doses of 315 mg anti-beta7 antibody (or nominal dose of 300 mg), where each subsequent maintenance dose is administered four weeks after the prior maintenance dose. In one embodiment, the dosing or dosing regimen determined as indicative of the efficacy of or responsiveness to the dose or dosing regimen comprises subcutaneous administration of 105 mg anti-beta7 antibody (or nominal dose of 100 mg) every four weeks or 50 mg anti-beta7 antibody (nominal dose) every two weeks. In one embodiment, the anti-beta7 antibody is etrolizumab.

In certain aspects of the above-described methods, the integrin beta7 antagonist is a monoclonal anti-beta7 antibody. In certain such embodiments, the anti-beta7 antibody is selected from a chimeric antibody, a human antibody, and a humanized antibody. In certain embodiments, the anti-beta7 antibody is an antibody fragment. In certain embodiments, the anti-beta7 antibody comprises six hypervariable regions (HVRs), wherein:
(i) HVR-L1 comprises amino acid sequence A1-A11, wherein A1-A11 is RASESVDTYLH (SEQ ID NO:1); RASESVDSLLH (SEQ ID NO:7), RASESVDTLLH (SEQ ID NO:8), or RASESVDDLLH (SEQ ID NO:9) or a variant of SEQ ID NOs:1, 7, 8 or 9 (SEQ ID NO:26) wherein amino acid A2 is selected from the group consisting of A, G, S, T, and V and/or amino acid A3 is selected from the group consisting of S, G, I, K, N, P, Q, R, and T, and/or A4 is selected from the group consisting of E, V, Q, A, D, G, H, I, K, L, N, and R, and/or amino acid A5 is selected from the group consisting of S, Y, A, D, G, H, I, K, N, P, R, T, and V, and/or amino acid A6 is selected from the group consisting of V, R, I, A, G, K, L, M, and Q, and/or amino acid A7 is selected from the group consisting of D, V, S, A, E, G, H, I, K, L, N, P, S, and T, and/or amino acid A8 is selected from the group consisting of D, G, N, E, T, P and S, and/or amino acid A9 is selected from the group consisting of L, Y, I and M, and/or amino acid A10 is selected from the group consisting of L, A, I, M, and V and/or amino acid A11 is selected from the group consisting of H, Y, F, and S;
(ii) HVR-L2 comprises amino acid sequence B1-B8, wherein B1-B8 is KYASQSIS (SEQ ID NO:2), RYASQSIS (SEQ ID NO:20), or XaaYASQSIS (SEQ ID NO:21, where Xaa represents any amino acid) or a variant of SEQ ID NOs:2, 20 or 21 (SEQ ID NO:27) wherein amino acid B1 is selected from the group consisting of K, R, N, V, A, F, Q, H, P, I, L, Y and Xaa (where Xaa represents any amino acid), and/or amino acid B4 is selected from the group consisting of S and D, and/or amino acid B5 is selected from the group consisting of Q and S, and/or amino acid B6 is selected from the group consisting of S, D, L, and R, and/or amino acid B7 is selected from the group consisting of I, V, E, and K;
(iii) HVR-L3 comprises amino acid sequence C1-C9, wherein C1-C9 is QQGNSLPNT (SEQ ID NO:3) or a variant of SEQ ID NO:3 (SEQ ID NO:28) wherein amino acid C8 is selected from the group consisting of N, V, W, Y, R, S, T, A, F, H, I L, and M;
(iv) HVR-H1 comprises amino acid sequence D1-D10 wherein D1-D10 is GFFITNNYWG (SEQ ID NO:4);
(v) HVR-H2 comprises amino acid sequence E1-E17 wherein E1-E17 is GYISYSGSTSYNPSLKS (SEQ ID NO:5), or a variant of SEQ ID NO:5 (SEQ ID NO:29) wherein amino acid E2 is selected from the group consisting of Y, F, V, and D, and/or amino acid E6 is selected from the group consisting of S and G, and/or amino acid E10 is selected from the group consisting of S and Y, and/or amino acid E12 is selected from the group consisting of N, T, A, and D, and/or amino acid 13 is selected from the group consisting of P, H, D, and A, and/or amino acid E15 is selected from the group consisting of L and V, and/or amino acid E17 is selected from the group consisting of S and G; and
(vi) HVR-H3 comprises amino acid sequence F2-F11 wherein F2 -F11 is MTGSSGYFDF (SEQ ID NO:6) or RTGSSGYFDF (SEQ ID NO: 19); or comprises amino acid sequence F1-F11, wherein F1-F11 is AMTGSSGYFDF (SEQ ID NO: 16), ARTGSSGYFDF (SEQ ID NO:17), or AQTGSSGYFDF (SEQ ID NO:18), or a variant of SEQ ID NOs:6, 16, 17, 18, or 19 (SEQ ID NO:30) wherein amino acid F2 is R, M, A, E, G, Q, S, and/or amino acid F11 is selected from the group consisting of F and Y.

In certain embodiments, the anti-beta7 antibody comprises three heavy chain hypervariable region (HVR-H1-H3) sequences and three light chain hypervariable region (HVR-L1-L3) sequences, wherein:
(i) HVR-L1 comprises SEQ ID NO:7, SEQ ID NO:8 or SEQ ID NO:9;
(ii) HVR-L2 comprises SEQ ID NO:2;
(iii) HVR-L3 comprises SEQ ID NO:3;
(iv) HVR-H1 comprises SEQ ID NO:4;
(v) HVR-H2 comprises SEQ ID NO:5; and
(vi) HVR-H3 comprises SEQ ID NO:6 or SEQ ID NO:16 or SEQ ID NO:17 or SEQ ID NO:19. In certain embodiments, the anti-beta7 antibody comprises a variable light chain comprising the amino acid sequence of SEQ ID NO:31 and a variable heavy chain comprising the amino acid sequence of SEQ ID NO:32.

In certain embodiments the anti-beta7 antibody is etrolizumab, also referred to as rhuMAb Beta7.

### BRIEF DESCRIPTION OF THE DRAWINGS

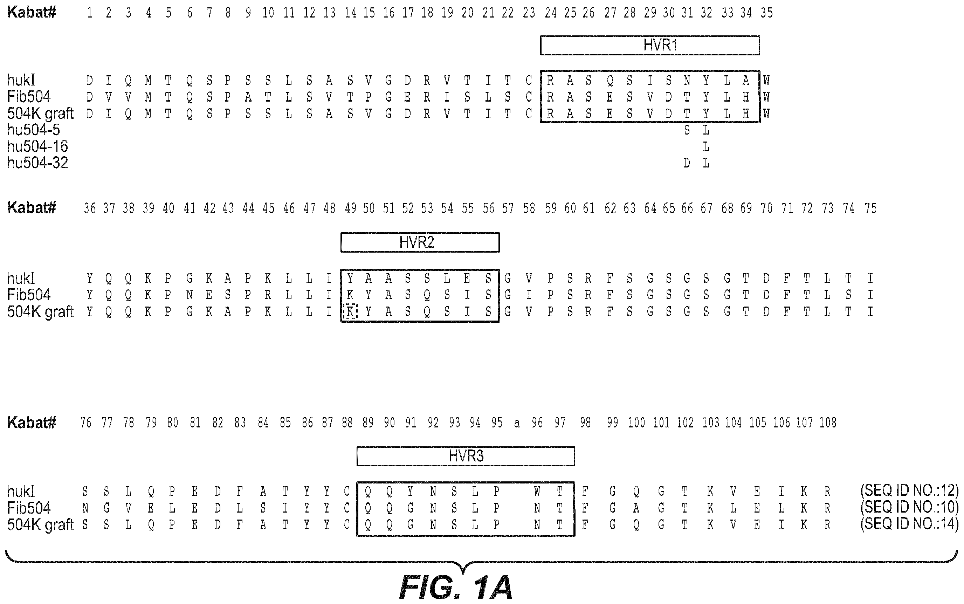

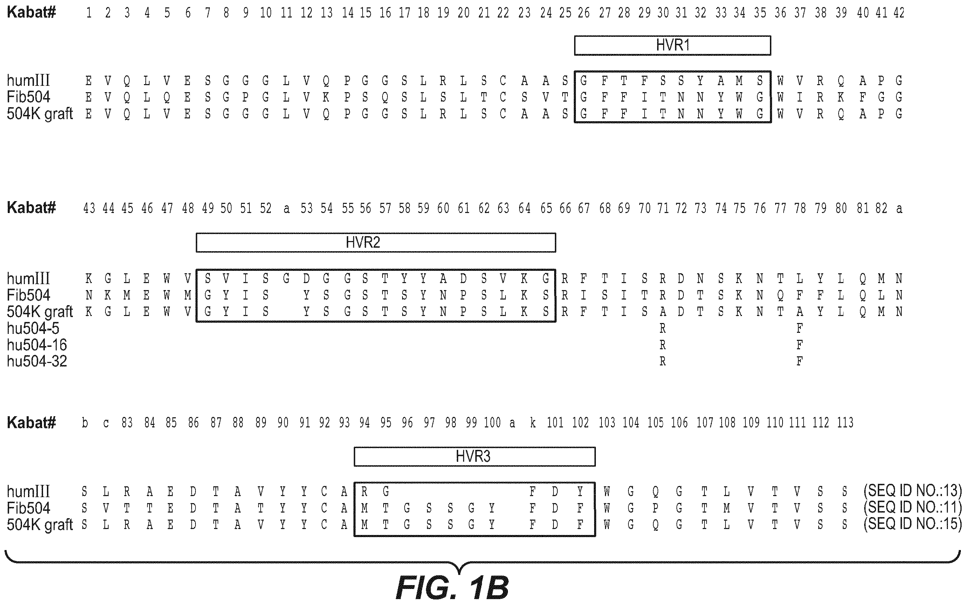

Figure 1A and 1B shows alignment of sequences of the variable light and heavy chains for the following consensus sequences and anti-beta7 subunit antibody sequences: light chain human subgroup kappa I consensus sequence (FIG. 1A, SEQ ID NO: 12), heavy chain human subgroup III consensus sequence (FIG. 1B, SEQ ID NO:13), rat anti-mouse beta7 antibody (Fib504) variable light chain (FIG. 1A, SEQ ID NO:10), rat anti-mouse beta7 antibody (Fib504) variable heavy chain (FIG. 1B, SEQ ID NO:11), and humanized antibody variants: Humanized hu504Kgraft variable light chain (FIG. 1A, SEQ ID NO: 14), humanized hu504K graft variable heavy chain (FIG. 1B, SEQ ID NO: 15), variants hu504-5, hu504-16, and hu504-32 (amino acid variations from humanized hu504K graft are indicated in FIG. 1A) (light chain) (SEQ ID NOS:22-24, respectively, in order of appearance) and FIG. 1B (heavy chain) for variants hu504-5, hu504-16, and hu504-32 (SEQ ID NO:25).

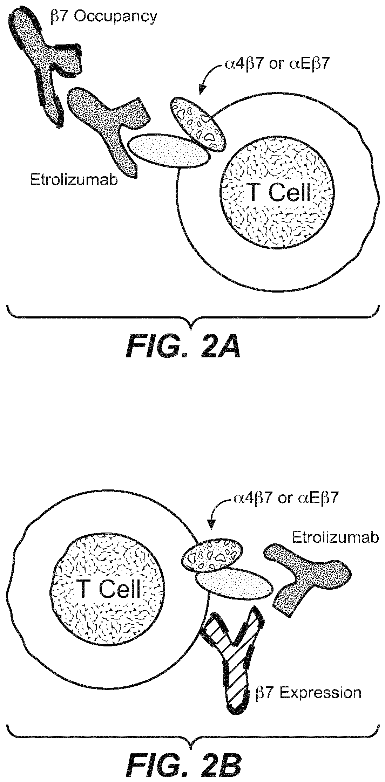

Figure 2A shows a schematic drawing of the occupancy assay described in Example 1.
Figure 2B shows a schematic drawing of the expression assay (also referred to as the MOA assay) described in Example 1.

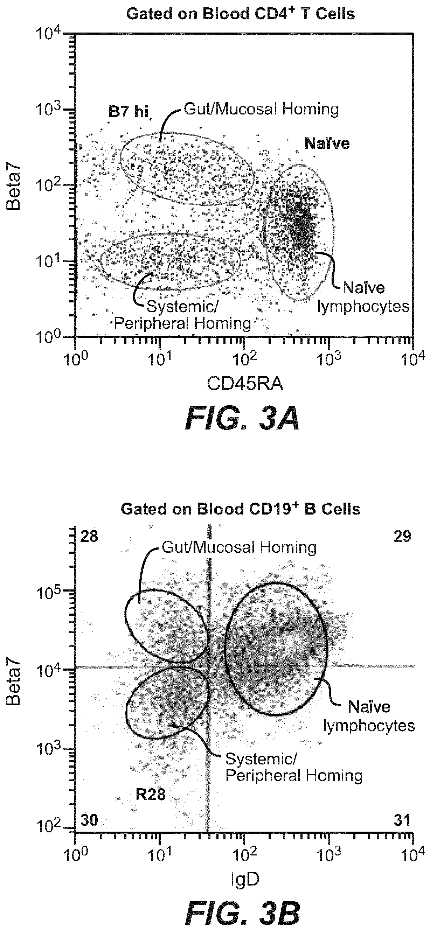

Figure 3A shows the phenotypic subdivision of peripheral blood T cells as described in Example 1.
Figure 3B shows the phenotypic subdivision of peripheral blood B cells as described in Example 1.

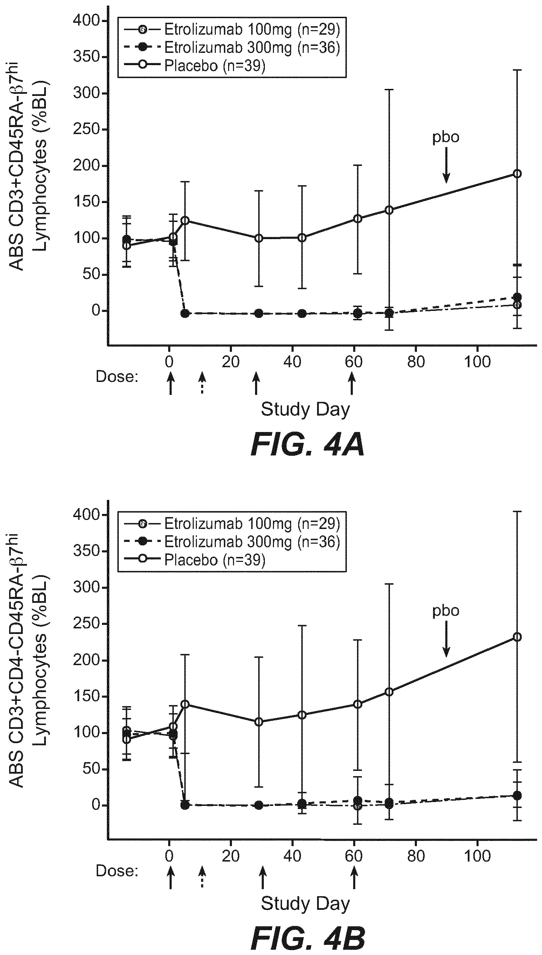

Figure 4A shows integrin beta7 occupancy on peripheral blood T cells (CD3+, CD4+, CD45RA-, beta7high) in patient samples following placebo (pbo) or etrolizumab administration according to two different dosing regimens as described in Example 1. Group mean absolute counts expressed as a percentage of baseline (%BL) are shown with error bars representing standard deviation from the mean. Solid line with open circles (pbo), dotdashed line with stippled circles (100 mg etrolizumab), dashed line with closed circles (300mg + LD etrolizumab). The solid arrows denote etrolizumab or pbo administration according to the treatment arm; the dashed arrow denotes placebo administration in all arms.
Figure 4B shows integrin beta7 occupancy on peripheral blood T cells (CD3+, CD4-, CD45RA-, beta7high) in patient samples following placebo (pbo) or etrolizumab administration according to two different dosing regimens as described in Example 1. Group mean absolute counts expressed as a percentage of baseline (%BL) are shown with error bars representing standard deviation from the mean. Solid line with open circles (pbo), dotdashed line with stippled circles (100 mg etrolizumab), dashed line with closed circles (300mg + LD etrolizumab). The solid arrows denote etrolizumab or pbo administration according to the treatment arm; the dashed arrow denotes placebo administration in all arms.

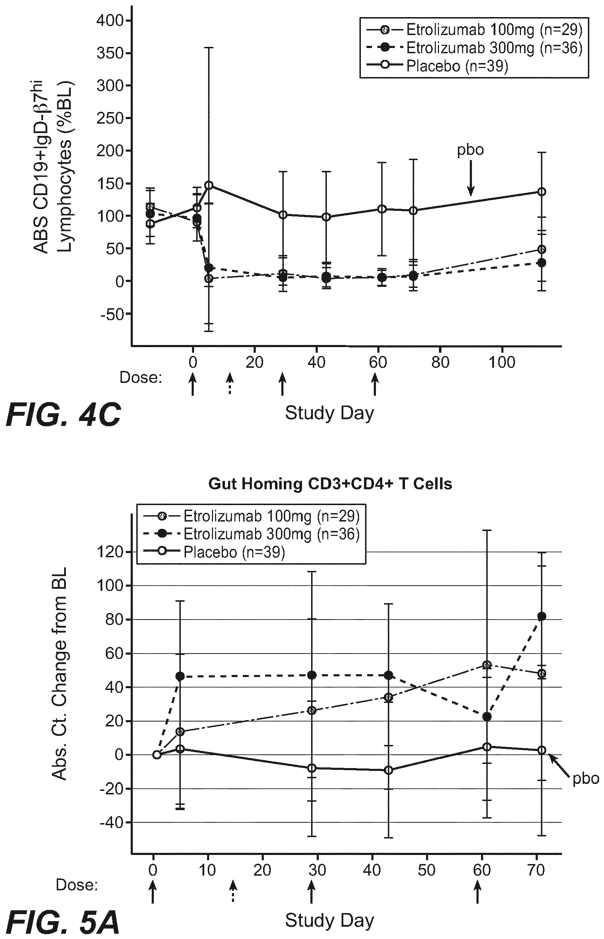

Figure 4C shows integrin beta7 occupancy on peripheral blood B cells (CD19+, IgD-, beta7high) in patient samples following placebo (pbo) or etrolizumab administration according to two different dosing regimens as described in Example 1. Group mean absolute counts expressed as a percentage of baseline (%BL) are shown with error bars representing standard deviation from the mean. Solid line with open circles (pbo), dotdashed line with stippled circles (100 mg etrolizumab), dashed line with closed circles (300mg + LD etrolizumab). The solid arrows denote etrolizumab or pbo administration according to the treatment arm; the dashed arrow denotes placebo administration in all arms.

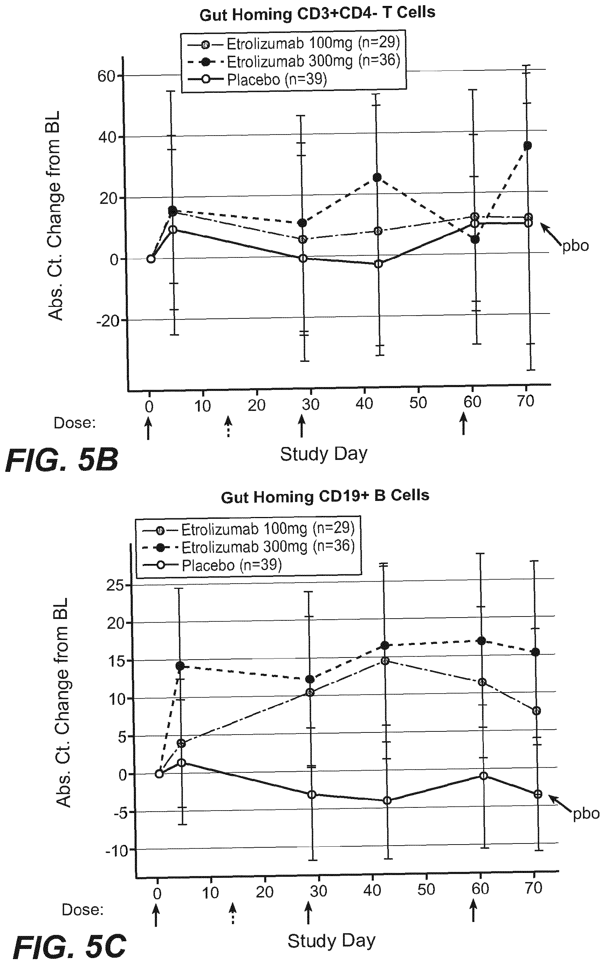

Figure 5 shows integrin beta7 expression on peripheral blood mucosal (gut) homing T and B cells in patient samples following placebo (pbo) or etrolizumab administration according to two different dosing regimens as described in Example 1. Group median absolute counts expressed as a change from baseline are shown, with error bars representing the absolute deviation from the median. (A) Mucosal (gut) homing CD3+CD4+ T cells; (B) Mucosal (gut) homing CD3+ CD4- T cells; (C) Mucosal (gut) homing CD19+ B cells. Solid line with open circles (pbo), dotdashed line with stippled circles (100 mg etrolizumab), dashed line with closed circles (300mg + LD etrolizumab). The solid arrows denote etrolizumab or pbo administration according to the treatment arm; the dashed arrow denotes placebo administration in all arms.

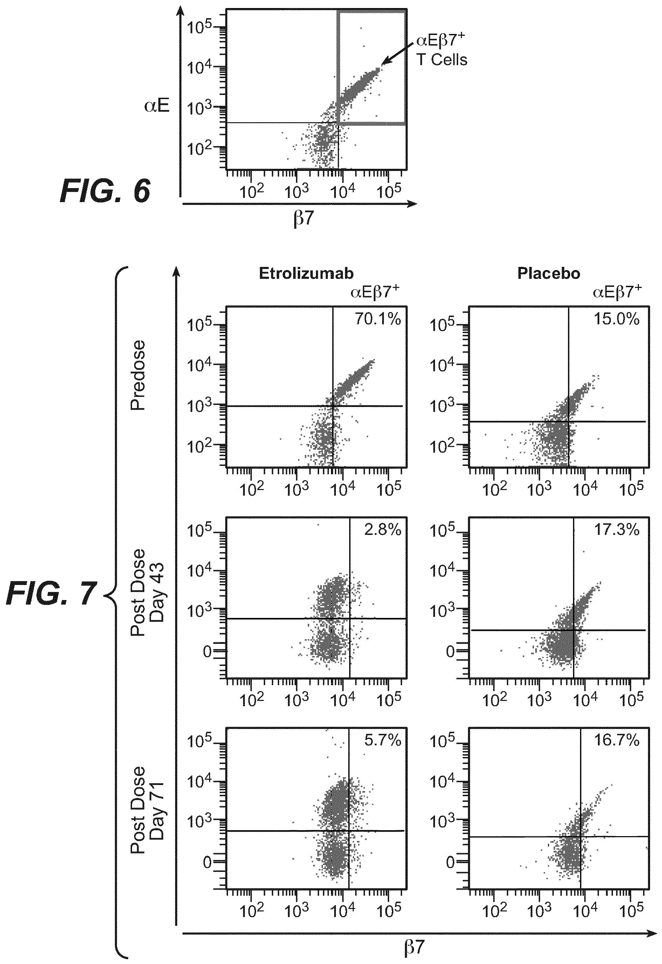

Figure 6 shows a representative FACS dot plot of cell surface αEβ7 expression on CD45+, CD3+, CD4- T lymphocytes obtained from a colonic biopsy sample from a patient before treatment with etrolizumab as described in Example 1. FACS plot of T lymphocytes from the patient prior to dosing with etrolizumab: αE levels are shown on the vertical axis, β7 levels as determined using labeled FIB504 antibody (competing antibody) are shown on the horizontal axis, the boxed upper right quadrant shows the signal from cells that are both αE+ and β7+.
Figure 7 shows representative FACS dot plots of cell surface αEβ7 occupancy on CD45+, CD3+, CD4- T lymphocytes obtained from colonic biopsy samples obtained from patients before and after treatment with a single dose of etrolizumab at 100 mg or placebo as described in Example 1. αE levels are shown on the vertical axis, β7 levels as determined using labeled FIB504 antibody (competing antibody) are shown on the horizontal axis, the upper right quadrant shows the signal from cells that are both αE+ and β7+; the percentage of cells staining positive for both markers is indicated. The plots on the left show FACS dot plots for a patient dosed with etrolizumab; the plots on the right show FACS dot plots for a patient dosed with placebo. The upper plots show prior to dosing; the middle plots show day 43, and the lower plots show day 71. Similar results as shown here were obtained from other patients dosed with etrolizumab or placebo.

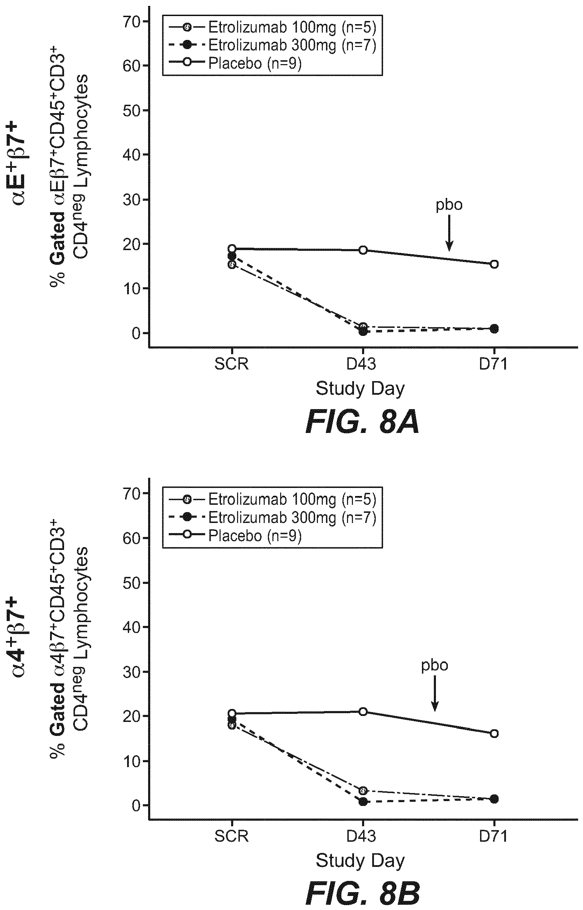

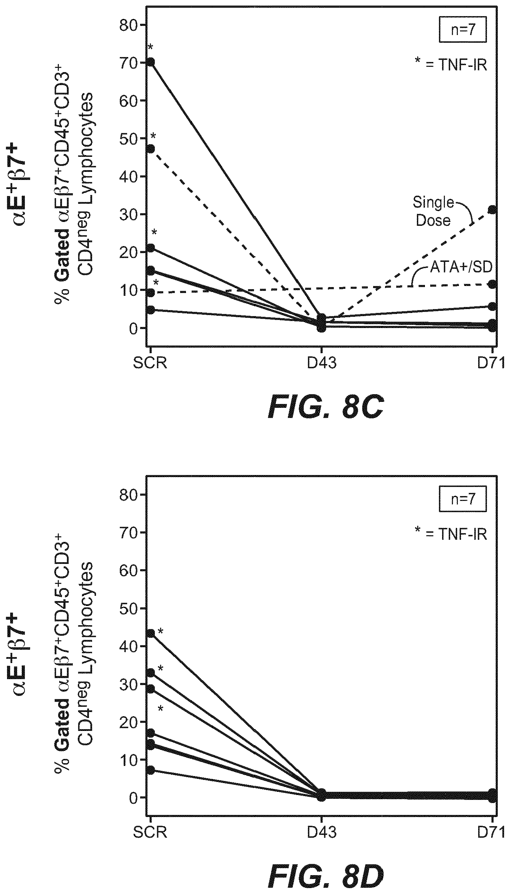

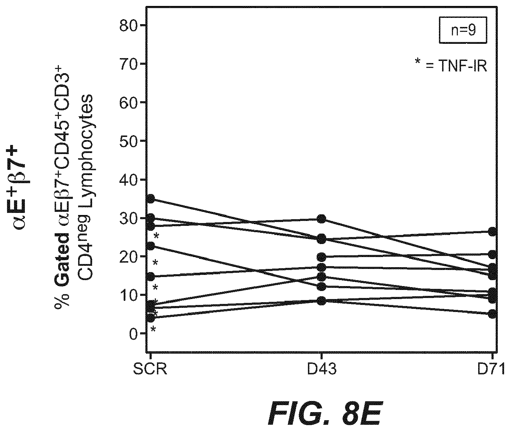

Figure 8A-E shows beta7 receptor occupancy on CD45+, CD3+, CD4- T lymphocytes obtained from colonic biopsy samples obtained from patients before, during and/or after treatment with etrolizumab or placebo as described in Example 1. Loss of detectable T lymphocytes indicates occupancy. (A) Cohort median percentages of detectable αEβ7+, CD45+, CD3+, CD4- T lymphocytes for patients treated in the "100 mg" dose arm, the "300 mg + LD" dose arm or placebo, as indicated, solid line with open circles (pbo), dotdashed line with stippled circles (100 mg etrolizumab), dashed line with closed circles (300mg + LD etrolizumab); (B) Cohort median percentages of detectable α4β7+, CD45+, CD3+, CD4- T lymphocytes for patients treated in the "100 mg" dose arm, the "300 mg + LD" dose arm or placebo, as indicated, solid line with open circles (pbo), dotdashed line with stippled circles (100 mg etrolizumab), dashed line with closed circles (300mg + LD etrolizumab); (C) Percentages of detectable αEβ7+, CD45+, CD3+, CD4- T lymphocytes for each of seven patients in the "100 mg" dose of etrolizumab arm; two patients who received only a single dose (SD) are indicated by dashed lines, one of whom had an anti-therapeutic antibody (ATA) response; (D) Percentages of detectable αEβ7+, CD45+, CD3+, CD4- T lymphocytes for each of seven patients in the "300 mg + LD" arm; and (E) Percentages of detectable αEβ7+, CD45+, CD3+, CD4- T lymphocytes for each of nine patients in the placebo arm. In each of (C)-(E), TNF-IR patients are denoted with an (*).

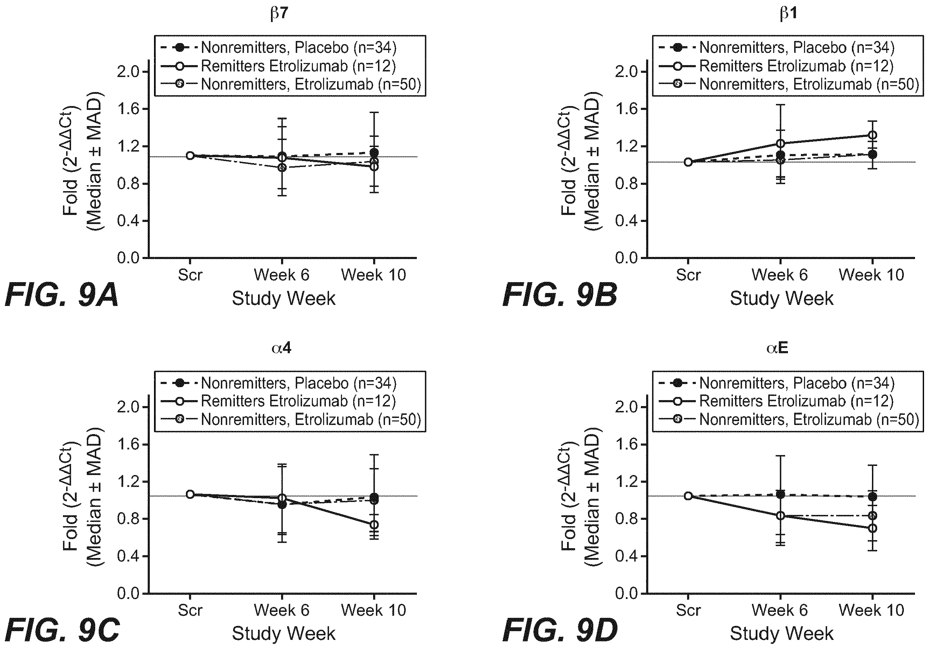

Figure 9 shows integrin expression in colonic biopsy in patients with clinical remission compared with patients without clinical remission before and after treatment with etrolizumab or placebo as described in Example 1. (A) Integrin-β7 expression by qPCR at screening (Scr), week 6 and week 10; (B) Integrin-β1 expression by qPCR at screening (Scr), week 6 and week 10; (C) Integrin-α4 expression by qPCR at screening (Scr), week 6 and week 10; (D) Integrin-αE expression by qPCR at screening (Scr), week 6 and week 10. Data are represented as fold change (2^{-ΔΔCt}) from baseline as group median ± median absolute deviation. Dashed lines with solid circles, placebo nonremitters; solid lines with open circles, etrolizumab-treated remitters; dotdashed lines with stippled circles, etrolizumab-treated nonremitters.

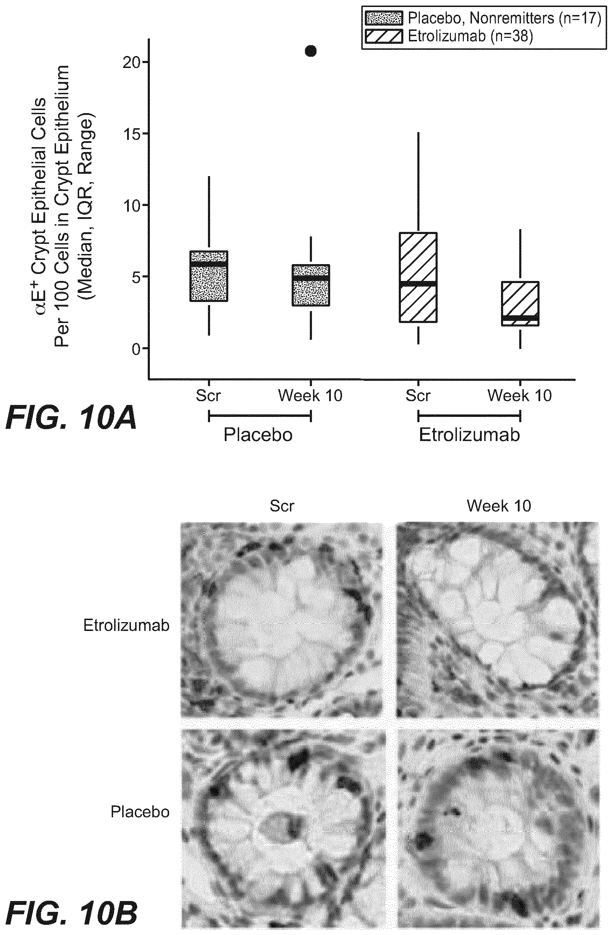

Figure 10 shows the effect of etrolizumab on αE+ cells in the intestinal crypt epithelium as described in Example 1. (A) Median αE+ cells in the intestinal crypt epithelium before and after treatment with etrolizumab (striped boxes) or placebo (stippled boxes); (B) Representative IHC stains of αE⁺ cells in intestinal crypt epithelium.

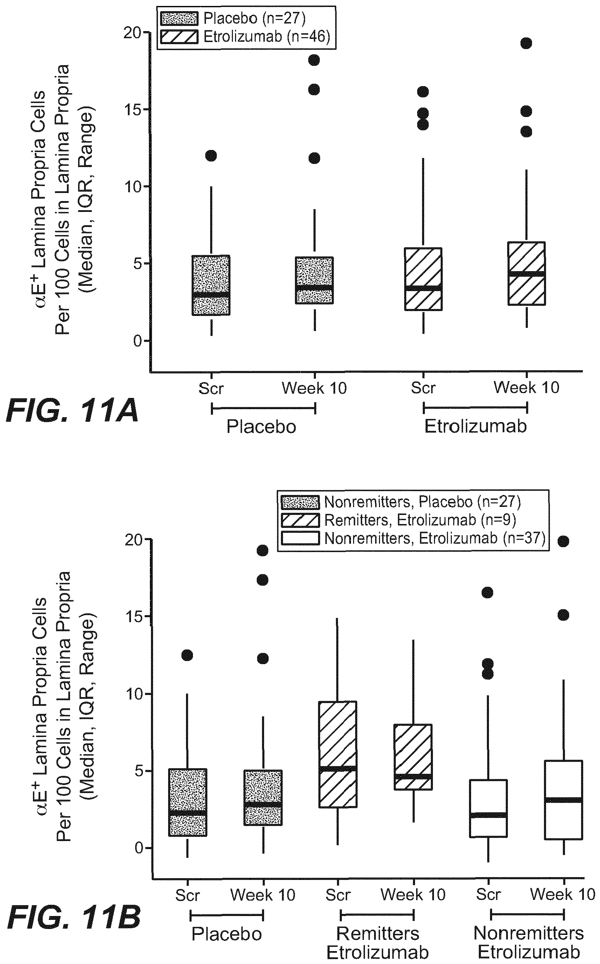

Figure 11 shows the effect of etrolizumab on αE+ cells in intestinal lamina propria as described in Example 1. (A) Mean αE+ cells in the intestinal lamina propria in all patients before and after treatment with etrolizumab (striped boxes) or placebo (stippled boxes); (B) Mean αE+ cells in the intestinal lamina propria before and after treatment with etrolizumab or placebo in patients with clinical remission compared with patients who did not achieve clinical remission. Mean, interquartile ranges (IQR) and ranges are shown on box plots: stippled boxes, placebo nonremitters; striped boxes, etrolizumab-treated remitters; open boxes, etrolizumab-treated nonremitters.

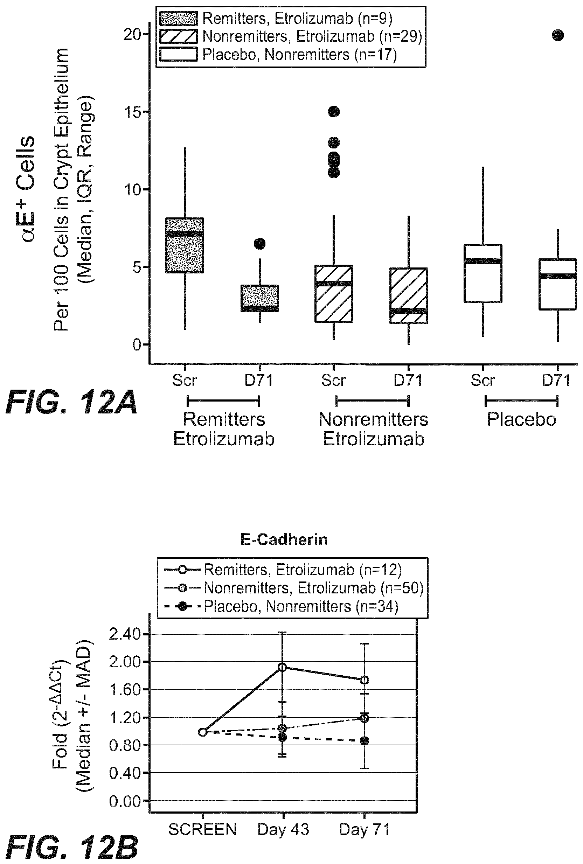

Figure 12A shows the immunohistochemistry quantification of αE+ cells in the intestinal crypt epithelium in remitters compared with nonremitters treated with etrolizumab or placebo; Mean, interquartile ranges (IQR) and ranges are shown on box plots: stippled boxes, etrolizumab-treated remitters; striped boxes, etrolizumab-treated nonremitters; open boxes, placebo nonremitters; Figure 12B shows E-cadherin levels in colonic tissue before and after treatment with etrolizumab or placebo by clinical remission status, as described in Example 1; dashed lines with solid circles, placebo nonremitters; solid lines with open circles, etrolizumab-treated remitters; dotdashed lines with stippled circles, etrolizumab-treated nonremitters.

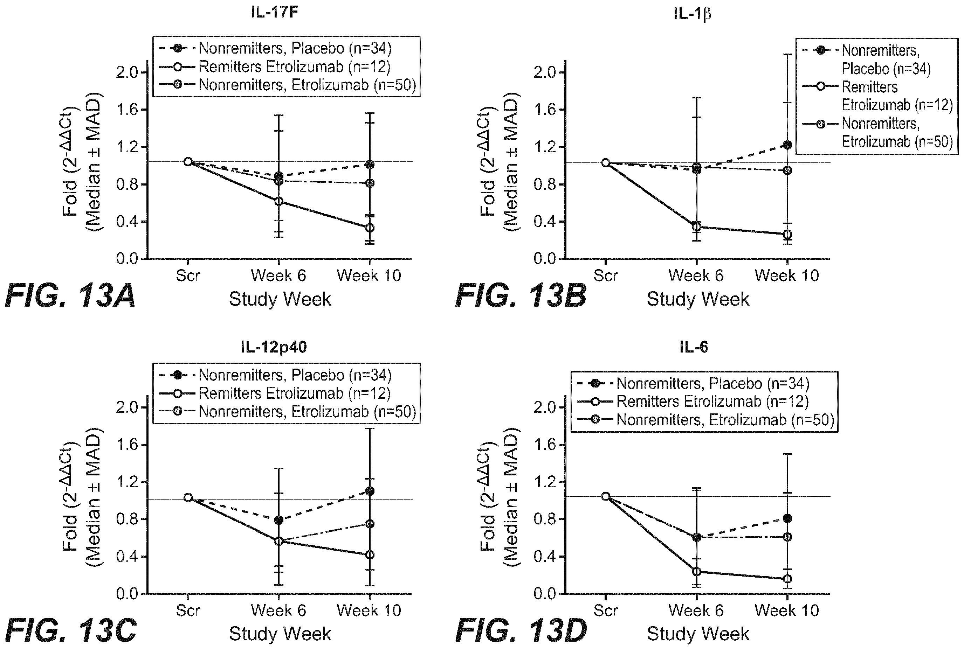

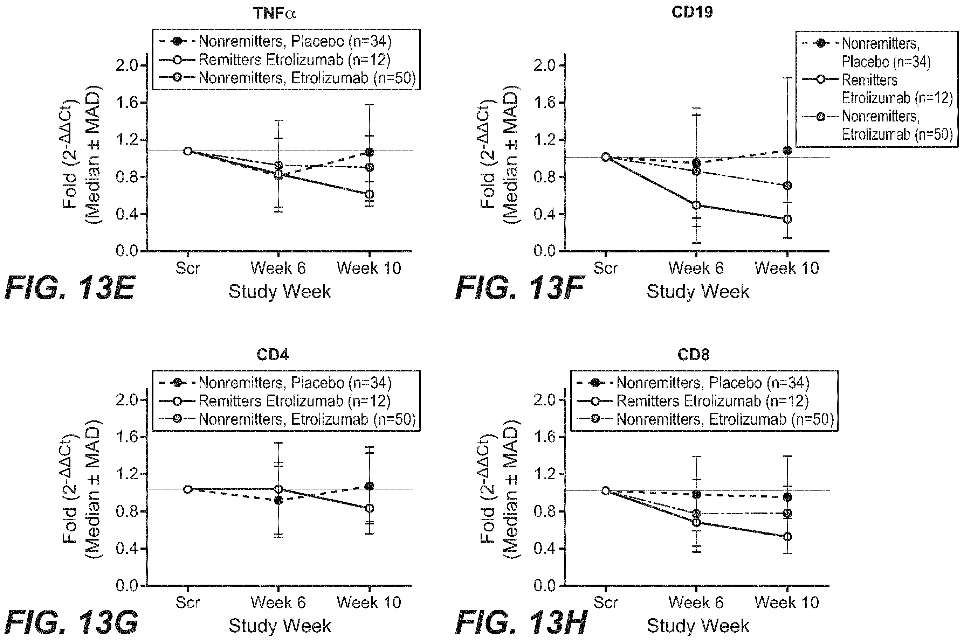

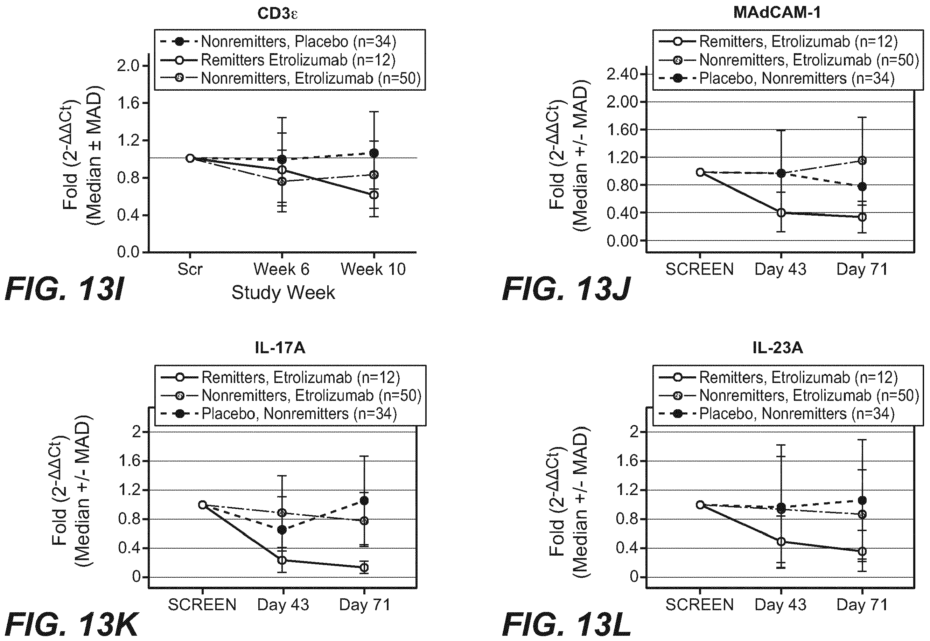

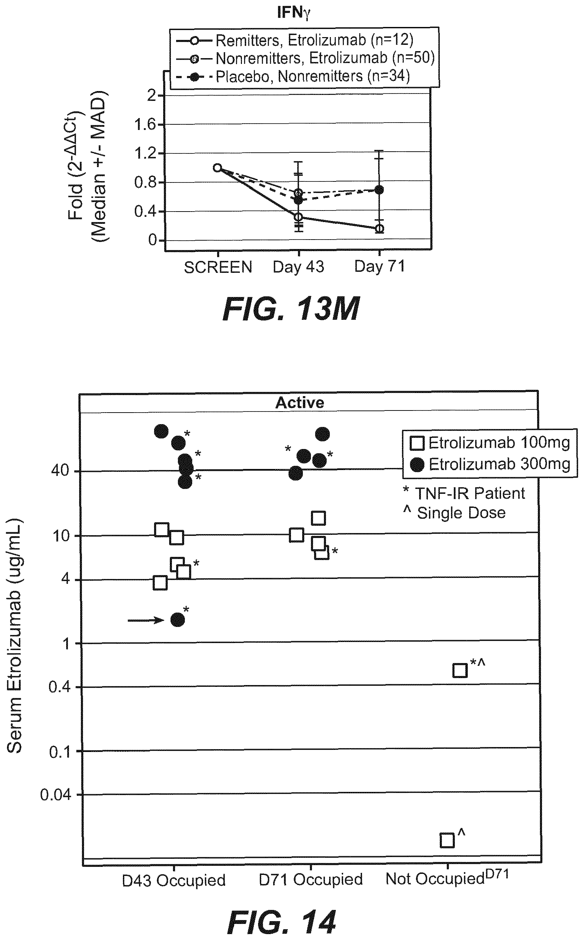

Figure 13 shows the expression of MAdCAM-1, cytokines and markers for lymphocyte subsets in colonic biopsy in patients with clinical remission compared with patients without clinical remission before and after treatment with etrolizumab or placebo as described in Example 1. Expression was quantified by qPCR, data are presented as fold change (2^{-ΔΔCt}) group median + median absolute deviation (MAD). (A) IL-17F; (B) IL-1β; (C) IL-12p40; (D) IL-6; (E) TNFα; (F) CD19; (G) CD4; (H) CD8; (I) CD3ε; (J) MAdCAM-1; (K) IL-17A; (L) IL-23A; (M) IFNγ. Dashed lines with solid circles, placebo nonremitters; solid lines with open circles, etrolizumab-treated remitters; dotdashed lines with stippled circles, etrolizumab-treated nonremitters.
Figure 14 shows the serum concentration of etrolizumab compared to colonic lymphocyte beta7 receptor occupancy in patients treated with 100 mg etrolizumab q4w or 300 mg etrolizumab q4w plus a loading dose at days 43 and 71 as described in Example 1. TNF-IR patients are indicated as are two patients who received only a single dose. The arrow identifies one patient who received only two doses.

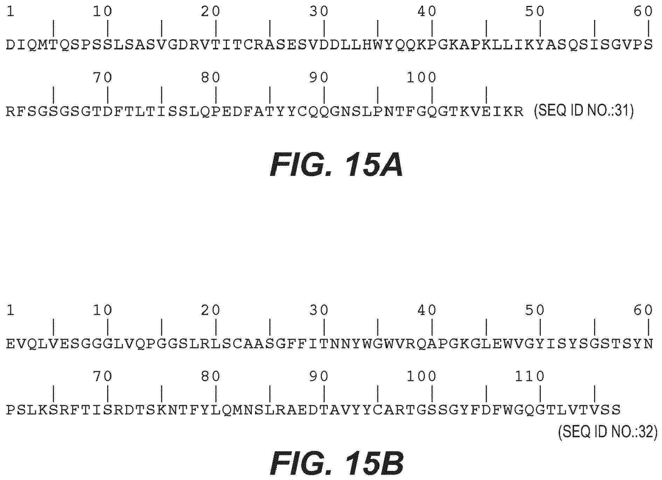

Figure 15 shows the variable light chain region (A) (SEQ ID NO:31) and the variable heavy chain region (B) (SEQ ID NO:32) of etrolizumab.

### DETAILED DESCRIPTION

Unless defined otherwise, technical and scientific terms used herein have the same meaning as commonly understood by one of ordinary skill in the art to which this invention belongs. Singleton et al., Dictionary of Microbiology and Molecular Biology 2nd ed., J. Wiley & Sons (New York, N.Y. 1994), and March, Advanced Organic Chemistry Reactions, Mechanisms and Structure 4th ed., John Wiley & Sons (New York, N.Y. 1992), provide one skilled in the art with a general guide to many of the terms used in the present application.

### I. Certain Definitions

As used in this specification and the appended claims, the singular forms "a," "an" and "the" include plural referents unless the context clearly dictates otherwise. Thus, for example, reference to "a protein" includes a plurality of proteins; reference to "a cell" includes mixtures of cells, and the like.

Ranges provided in the specification and appended claims include both end points and all points between the end points. Thus, for example, a range of 2.0 to 3.0 includes 2.0, 3.0, and all points between 2.0 and 3.0.

"Treatment" refers to clinical intervention in an attempt to alter the natural course of the individual or cell being treated, and can be performed either for prophylaxis or during the course of clinical pathology. Desirable effects of treatment include preventing occurrence or recurrence of disease, alleviation of symptoms, diminishment of any direct or indirect pathological consequences of the disease, decreasing the rate of disease progression, amelioration or palliation of the disease state, and remission or improved prognosis.

"Treatment regimen" refers to a combination of dosage, frequency of administration, or duration of treatment, with or without addition of a second medication.

"Effective treatment regimen" refers to a treatment regimen that will offer beneficial response to a patient receiving the treatment.

"Modifying a treatment" refers to changing the treatment regimen including, changing dosage, frequency of administration, or duration of treatment, and/or addition of a second medication.

"Patient response" or "patient responsiveness" can be assessed using any endpoint indicating a benefit to the patient, including, without limitation, (1) inhibition, to some extent, of disease progression, including slowing down and complete arrest; (2) reduction in the number of disease episodes and/or symptoms; (3) reduction in lesional size; (4) inhibition (*i.e.,* reduction, slowing down or complete stopping) of disease cell infiltration into adjacent peripheral organs and/or tissues; (5) inhibition (*i.e.,* reduction, slowing down or complete stopping) of disease spread; (6) decrease of auto-immune, immune, or inflammatory response, which may, but does not have to, result in the regression or ablation of the disease lesion; (7) relief, to some extent, of one or more symptoms associated with the disorder; (8) increase in the length of disease-free presentation following treatment; and/or (9) decreased mortality at a given point of time following treatment. The term "responsiveness" refers to a measurable response, including complete response (CR) and partial response (PR).

By "complete response" or "CR" is intended the disappearance of all signs of inflammation or remission in response to treatment. This does not always mean the disease has been cured.

"Partial response" or "PR" refers to a decrease of at least 50% in the severity of inflammation, in response to treatment.

A "beneficial response" of a patient to treatment with an integrin beta7 antagonist and similar wording refers to the clinical or therapeutic benefit imparted to a patient at risk for or suffering from a gastrointestinal inflammatory disorder from or as a result of the treatment with the antagonist, such as an anti-beta7 integrin antibody. Such benefit includes cellular or biological responses, a complete response, a partial response, a stable disease (without progression or relapse), or a response with a later relapse of the patient from or as a result of the treatment with the antagonist.

"A patient maintains responsiveness to a treatment" when the patient' responsiveness does not decrease with time during the course of a treatment.

As used herein, "non-response" or "lack of response" or similar wording means an absence of a complete response, a partial response, or a beneficial response to treatment with an integrin beta7 antagonist.

The term "monitoring the efficacy of an integrin beta7 antagonist therapy" is used to indicate that a sample is obtained at least once, including serially, from a patient before, during, and/or after therapy with an integrin beta7 antagonist and that a biomarker selected from integrin beta7 subunit-containing receptor occupancy by the antagonist on colonic lymphocytes, gene expression levels of one or more integrin receptor ligands, gene expression levels of one or more lymphocyte genes, gene expression levels of one or more cytokines, and the number of alphaE-positive cells in intestinal crypt epithelium is measured in such samples, and the results of before therapy, during therapy, and/or after therapy are compared, to obtain an indication whether the therapy is efficacious or not. In the monitoring of the efficacy of a therapy the level of a biomarker selected from integrin beta7 subunit-containing receptor occupancy by the antagonist on colonic lymphocytes, gene expression levels of one or more integrin receptor ligands, gene expression levels of one or more lymphocyte genes, gene expression levels of one or more cytokines, and the number of alphaE-positive cells in intestinal crypt epithelium is measured and in one embodiment compared to a reference value for the same biomarker, or, in a further embodiment, it is compared to the level of the same biomarker in a sample obtained from the same patient at an earlier point in time, either while the patient was under therapy or before start of the therapy. In one embodiment, a decreased level or an increased level of one or more biomarkers selected from integrin beta7 subunit-containing receptor occupancy by the antagonist on colonic lymphocytes, gene expression levels of one or more integrin receptor ligands, gene expression levels of one or more lymphocyte genes, gene expression levels of one or more cytokines, and the number of alphaE-positive cells in intestinal crypt epithelium, a decreased level or an increased level depending upon the particularly biomarker assessed as described further herein, of a biomarker as compared to the level of the same biomarker in a sample obtained from the same patient at an earlier point in time, either while the patient was already under therapy or before start of the therapy indicates that the patient is responsive to the therapy.

The term "diagnosis" is used herein to refer to the identification or classification of a molecular or pathological state, disease or condition. For example, "diagnosis" may refer to identification of a particular type of gastrointestinal inflammatory disorder, and more particularly, the classification of a particular sub-type of gastrointestinal inflammatory disorder, by tissue/organ involvement (e.g., inflammatory bowel disease), or by other features *(e.g.,* a patient subpopulation characterized by responsiveness to a treatment, such as to a treatment with an integrin beta7 antagonist).

The term "prognosis" is used herein to refer to the prediction of the likelihood of disease symptoms, including, for example, recurrence, flaring, and drug resistance, of a gastrointestinal inflammatory disorder.

The term "sample" or "test sample", as used herein, refers to a composition that is obtained or derived from a subject of interest that contains a cellular and/or other molecular entity that is to be characterized and/or identified, for example based on physical, biochemical, chemical and/or physiological characteristics. For example, the phrase "disease sample" and variations thereof refers to any sample obtained from a subject of interest that would be expected or is known to contain the cellular and/or molecular entity that is to be characterized. The sample can be obtained from a tissue for the subject of interest or from peripheral blood of the subject.

A "reference sample," as used herein, refers to any sample, standard, or level that is used for comparison purposes. In one embodiment, a reference sample is obtained from a healthy and/or non-diseased part of the body (e.g., tissue or cells) of the same subject or patient. In another embodiment, a reference sample is obtained from an untreated tissue and/or cell of the body of the same subject or patient. In yet another embodiment, a reference sample is obtained from a healthy and/or non-diseased part of the body (e.g., tissues or cells) of an individual who is not the subject or patient. In even another embodiment, a reference sample is obtained from an untreated tissue and/or cell part of the body of an individual who is not the subject or patient.

"A beta7 integrin antagonist" or "beta7 antagonist" refers to any molecule that inhibits one or more biological activities or blocking binding of beta7 integrin with one or more of its associated molecules. Antagonists of the invention can be used to modulate one or more aspects of beta7 associated effects, including but not limited to association with alpha4 integrin subunit, association with alphaE integrin subunit, binding of alpha4beta7 integrin to MAdCAM, VCAM-1 or fibronectin and binding of alphaEbeta7 integrin to E-cadherin. These effects can be modulated by any biologically relevant mechanism, including disruption of ligand binding to beta7 subunit or to the alpha4beta7 or alphaEbeta7 dimeric integrin, and/or by disrupting association between the alpha and beta integrin subunits such that formation of the dimeric integrin is inhibited. In one embodiment of the invention, the beta7 antagonist is an anti-beta7 integrin antibody (or anti-beta7 antibody). In one embodiment, the anti-beta7 integrin antibody is a humanized anti-beta7 integrin antibody and more particularly a recombinant humanized monoclonal anti-beta7 antibody (or rhuMAb beta7 also referred to as etrolizumab). In some embodiments, the anti-beta7 antibodies of the present invention are anti-integrin beta7 antagonistic antibodies that inhibit or block the binding of beta7 subunit with alpha4 integrin subunit, association with alphaE integrin subunit, binding of alpha4beta7 integrin to MAdCAM, VCAM-1 or fibronectin and binding of alphaEbeta7 integrin to E-cadherin.

By "beta7 subunit" or "β7 subunit" is meant the human β7 integrin subunit (Erle et al., (1991) J. Biol. Chem. 266:11009-11016). The beta7 subunit associates with alpha4 integrin subunit, such as the human .alpha.4 subunit (Kilger and Holzmann (1995) J. Mol. Biol. 73:347-354). The alpha4beta7 integrin is reportedly expressed on a majority of mature lymphocytes, as well as a small population of thymocytes, bone marrow cells and mast cells. (Kilshaw and Murant (1991) Eur. J. Immunol. 21:2591-2597; Gurish *et al.,* (1992) 149: 1964-1972; and Shaw, S. K. and Brenner, M. B. (1995) Semin. Immunol. 7:335). The beta7 subunit also associates with the alphaE subunit, such as the human alphaE integrin subunit (Cepek, K. L, et al. (1993) J. Immunol. 150:3459). The alphaEbeta7 integrin is expressed on intra-intestinal epithelial lymphocytes (iIELs) (Cepek, K. L. (1993) *supra*)*.*

By "alphaE subunit" or "alphaE integrin subunit" or "αE subunit" or "αE integrin subunit" or "CD103" is meant an integrin subunit found to be associated with beta7 integrin on intra-epithelial lymphocytes, which alphaEbeta7 integrin mediates binding of the iELs to intestinal epithelium expressing E-cadherin (Cepek, K. L. et al. (1993) J. Immunol. 150:3459; Shaw, S. K. and Brenner, M. B. (1995) Semin. Immunol. 7:335).

"MAdCAM" or "MAdCAM-1" are used interchangeably in the context of the present invention and refer to the protein mucosal addressin cell adhesion molecule-1, which is a single chain polypeptide comprising a short cytoplasmic tail, a transmembrane region and an extracellular sequence composed of three immunoglobulin-like domains. The cDNAs for murine, human and macaque MAdCAM-1 have been cloned (Briskin, et al., (1993) Nature, 363:461-464; Shyjan et al., (1996) J. Immunol. 156:2851-2857).

"VCAM-1" or "vascular cell adhesion molecule-1" "CD106" refers to a ligand of alpha4beta7 and alpha4beta1, expressed on activated endothelium and important in endothelial-leukocyte interactions such as binding and transmigration of leukocytes during inflammation.

"CD45" refers to a protein of the protein tyrosine phosphatase (PTP) family. PTPs are known to be signaling molecules that regulate a variety of cellular processes including cell growth, differentiation, mitotic cycle, and oncogenic transformation. This PTP contains an extracellular domain, a single transmembrane segment and two tandem intracytoplasmic catalytic domains, and thus belongs to receptor type PTP. This gene is specifically expressed in hematopoietic cells. This PTP has been shown to be an essential regulator of T- and B-cell antigen receptor signaling. It functions through either direct interaction with components of the antigen receptor complexes, or by activating various Src family kinases required for the antigen receptor signaling. This PTP also suppresses JAK kinases, and thus functions as a regulator of cytokine receptor signaling. Four alternatively spliced transcripts variants of this gene, which encode distinct isoforms, have been reported. (Tchilian EZ, Beverley PC (2002). "CD45 in memory and disease." Arch. Immunol. Ther. Exp. (Warsz.) 50 (2): 85-93. Ishikawa H, Tsuyama N, Abroun S, et al. (2004). "Interleukin-6, CD45 and the src-kinases in myeloma cell proliferation." Leuk. Lymphoma 44 (9):1477-81.

Various isoforms of CD45 exist: CD45RA, CD45RB, CD45RC, CD45RAB, CD45RAC, CD45RBC, CD45RO, CD45R (ABC). CD45 is also highly glycosylated. CD45R is the longest protein and migrates at 200 kDa when isolated from T cells. B cells also express CD45R with heavier glycosylation, bringing the molecular weight to 220 kDa, hence the name B220; B cell isoform of 220 kDa. B220 expression is not restricted to B cells and can also be expressed on activated T cells, on a subset of dendritic cells and other antigen presenting cells. Stanton T, Boxall S, Bennett A, et al. (2004). "CD45 variant alleles: possibly increased frequency of a novel exon 4 CD45 polymorphism in HIV seropositive Ugandans." Immunogenetics 56 (2): 107-10.

"Gut-homing lymphocytes" refer to a subgroup of lymphocytes having the characteristic of selectively homing to intestinal lymph nodes and tissues but not homing to peripheral lymph nodes and tissues. This subgroup of lymphocytes is characterized by an unique expression pattern of a combination of multiples cell surface molecules, including, but not limited to, the combination of CD4, CD45RA and Beta7. Typically, at least two subsets of peripheral blood CD4⁺ lymphocytes can be subdivided based on the markers of CD45RA and Beta7, CD45RA⁻β7^{high}, and CD45RA⁻β7^{low} CD4⁺ cells. CD45RA⁻β7^{high} CD4⁺ cells home preferentially to intestinal lymph nodes and tissues, whereas CD45RA ⁻β7^{low} CD4 ⁺ cells home preferentially to peripheral lymph nodes and tissues (Rott *et al.* 1996; Rott *et al.* 1997; Williams *et al.* 1998; Rose *et al.* 1998; Williams and Butcher 1997; Butcher *et al.* 1999). Gut-homing lymphocytes are therefore a distinctive subgroup of lymphocytes identified as CD45RA⁻β7^{high} CD4⁺ in a flow cytometry assay. The methods of identifying this group of lymphocytes are well-known in the art and also disclosed in detail in Examples of the present application.

As used herein with respect to a cell surface marker, the symbol "+" indicates a positive expression of a cell surface marker. For instance, CD4⁺ lymphocytes are a group of lymphocytes having CD4 expressed on their cell surfaces.

As used herein with respect to a cell surface marker, the symbol "-" indicates a negative expression of a cell surface marker. For instance, CD45RA⁻lymphocytes are a group of lymphocytes having no CD45RA expressed on their cell surfaces.

As used herein with respect to the expression of a cell surface marker, the symbol "low" indicates a relatively low level of expression of a cell surface marker on lymphocytes, while "high" indicates a relatively high level of expression of a cell surface marker on lymphocytes. In a flow cytometry, the intensity of β7^{high} is at least about 10 or 100 fold higher than that of β7^{low}. Thus, as provided herein in exemplary embodiments, the CD45RA⁻β7^{low} CD4 ⁺ and CD45RA⁻β7^{high} CD4 ⁺ lymphocytes locate in distinct portions of a dot plot or histogram of a flow cytometry analysis where X-axis is the intensity of expression of CD45AR and Y-axis is the intensity of the expression of Beta7.

"Peripheral-homing lymphocytes" refer to a subgroup of lymphocytes having the characteristic of homing to peripheral lymph nodes and tissues and not homing to intestinal lymph nodes and tissues. In an exemplary embodiment, as explained above, Peripheral-homing lymphocytes are a distinctive group of lymphocytes identified as CD45RA⁻β7^{low} CD4⁺ cells in a flow cytometry assay. The methods of identifying this group of lymphocytes are known in the art and disclosed in detail in the present application.

An "amount" or "level" of biomarker can be determined using methods known in the art and disclosed herein, such as flow cytometry analysis.

A "change in the amount or level of a biomarker" is as compared to a reference/comparator amount of the biomarker. In certain embodiments, the change is greater than about 10%, or greater than about 30%, or greater than about 50%, or greater than about 100%, or greater than about 300% as a function of the value for the reference or comparator amount. For example, a reference or comparator amount can be the amount of a biomarker before treatment and more particularly, can be the baseline or pre-dose amount.

The phrase "essentially the same as" as used herein, denotes an insignificant degree of change such that one of skill in the art would not consider the change to be of statistical significance or a biologically meaningful change within the context of the biological characteristic measured by said values *(e.g.,* the drug serum level needed to saturate the drug target receptors). For example, serum drug concentrations needed to saturate receptors that are less than about two fold different, or less than about three fold different, or less than about four fold different from each other are considered essentially the same.

"Gastrointestinal inflammatory disorders" are a group of chronic disorders that cause inflammation and/or ulceration in the mucous membrane. These disorders include, for example, inflammatory bowel disease (e.g., Crohn's disease, ulcerative colitis, indeterminate colitis and infectious colitis), mucositis (e.g., oral mucositis, gastrointestinal mucositis, nasal mucositis and proctitis), necrotizing enterocolitis and esophagitis. In a preferred embodiment, the gastrointestinal inflammatory disorder is a inflammatory bowel disease.

"Inflammatory Bowel Disease" or "IBD" is used interchangeably herein to refer to diseases of the bowel that cause inflammation and/or ulceration and includes without limitation Crohn's disease and ulcerative colitis.

"Crohn's disease (CD)" or "ulcerative colitis (UC)" are chronic inflammatory bowel diseases of unknown etiology. Crohn's disease, unlike ulcerative colitis, can affect any part of the bowel. The most prominent feature Crohn's disease is the granular, reddish-purple edematous thickening of the bowel wall. With the development of inflammation, these granulomas often lose their circumscribed borders and integrate with the surrounding tissue. Diarrhea and obstruction of the bowel are the predominant clinical features. As with ulcerative colitis, the course of Crohn's disease may be continuous or relapsing, mild or severe, but unlike ulcerative colitis, Crohn's disease is not curable by resection of the involved segment of bowel. Most patients with Crohn's disease require surgery at some point, but subsequent relapse is common and continuous medical treatment is usual.

Crohn's disease may involve any part of the alimentary tract from the mouth to the anus, although typically it appears in the ileocolic, small-intestinal or colonic-anorectal regions. Histopathologically, the disease manifests by discontinuous granulomatomas, crypt abscesses, fissures and aphthous ulcers. The inflammatory infiltrate is mixed, consisting of lymphocytes (both T and B cells), plasma cells, macrophages, and neutrophils. There is a disproportionate increase in IgM- and IgG-secreting plasma cells, macrophages and neutrophils.

Anti-inflammatory drugs sulfasalazine and 5-aminosalisylic acid (5-ASA) are useful for treating mildly active colonic Crohn's disease and are commonly prescribed to maintain remission of the disease. Metroidazole and ciprofloxacin are similar in efficacy to sulfasalazine and appear to be particularly useful for treating perianal disease. In more severe cases, corticosteroids are effective in treating active exacerbations and can even maintain remission. Azathioprine and 6-mercaptopurine have also shown success in patients who require chronic administration of corticosteroids. It is also possible that these drugs may play a role in the long-term prophylaxis. Unfortunately, there can be a very long delay (up to six months) before onset of action in some patients. Antidiarrheal drugs can also provide symptomatic relief in some patients. Nutritional therapy or elemental diet can improve the nutritional status of patients and induce symptomatic improvement of acute disease, but it does not induce sustained clinical remissions. Antibiotics are used in treating secondary small bowel bacterial overgrowth and in treatment of pyogenic complications.

"Ulcerative colitis (UC)" afflicts the large intestine. The course of the disease may be continuous or relapsing, mild or severe. The earliest lesion is an inflammatory infiltration with abscess formation at the base of the crypts of Lieberkuhn. Coalescence of these distended and ruptured crypts tends to separate the overlying mucosa from its blood supply, leading to ulceration. Symptoms of the disease include cramping, lower abdominal pain, rectal bleeding, and frequent, loose discharges consisting mainly of blood, pus and mucus with scanty fecal particles. A total colectomy may be required for acute, severe or chronic, unremitting ulcerative colitis.

The clinical features of UC are highly variable, and the onset may be insidious or abrupt, and may include diarrhea, tenesmus and relapsing rectal bleeding. With fulminant involvement of the entire colon, toxic megacolon, a life-threatening emergency, may occur. Extraintestinal manifestations include arthritis, pyoderma gangrenoum, uveitis, and erythema nodosum.

Treatment for UC includes sulfasalazine and related salicylate-containing drugs for mild cases and corticosteroid drugs in severe cases. Topical administration of either salicylates or corticosteroids is sometimes effective, particularly when the disease is limited to the distal bowel, and is associated with decreased side effects compared with systemic use. Supportive measures such as administration of iron and antidiarrheal agents are sometimes indicated. Azathioprine, 6-mercaptopurine and methotrexate are sometimes also prescribed for use in refractory corticosteroid-dependent cases.

An "effective dosage" refers to an amount effective, at dosages and for periods of time necessary, to achieve the desired therapeutic or prophylactic result.

As used herein, the term "patient" refers to any single animal, more preferably a mammal (including such non-human animals as, for example, dogs, cats, horses, rabbits, zoo animals, cows, pigs, sheep, and non-human primates) for which treatment is desired. Most preferably, the patient herein is a human.

The term "non-human subject" refers to any single non-human animal, more preferably a mammal (including such non-human animals as, for example, dogs, cats, horses, rabbits, zoo animals, cows, pigs, sheep, and non-human primates).

The terms "antibody" and "immunoglobulin" are used interchangeably in the broadest sense and include monoclonal antibodies (for example, full length or intact monoclonal antibodies), polyclonal antibodies, multivalent antibodies, multispecific antibodies (e.g., bispecific antibodies so long as they exhibit the desired biological activity) and may also include certain antibody fragments (as described in greater detail herein). An antibody can be human, humanized and/or affinity matured.

"Antibody fragments" comprise only a portion of an intact antibody, wherein the portion preferably retains at least one, preferably most or all, of the functions normally associated with that portion when present in an intact antibody. In one embodiment, an antibody fragment comprises an antigen binding site of the intact antibody and thus retains the ability to bind antigen. In another embodiment, an antibody fragment, for example one that comprises the Fc region, retains at least one of the biological functions normally associated with the Fc region when present in an intact antibody, such as FcRn binding, antibody half life modulation, ADCC function and complement binding. In one embodiment, an antibody fragment is a monovalent antibody that has an *in vivo* half life substantially similar to an intact antibody. For example, such an antibody fragment may comprise on antigen binding arm linked to an Fc sequence capable of conferring *in vivo* stability to the fragment.

The term "monoclonal antibody" as used herein refers to an antibody obtained from a population of substantially homogeneous antibodies, *i.e.,* the individual antibodies comprising the population are identical except for possible naturally occurring mutations that may be present in minor amounts. Monoclonal antibodies are highly specific, being directed against a single antigen. Furthermore, in contrast to polyclonal antibody preparations that typically include different antibodies directed against different determinants (epitopes), each monoclonal antibody is directed against a single determinant on the antigen.

The monoclonal antibodies herein specifically include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Patent No. 4,816,567; and Morrison et al., Proc. Natl. Acad. Sci. USA 81:6851-6855 (1984)).

"Humanized" forms of non-human (e.g., murine) antibodies are chimeric antibodies that contain minimal sequence derived from non-human immunoglobulin. For the most part, humanized antibodies are human immunoglobulins (recipient antibody) in which residues from a hypervariable region of the recipient are replaced by residues from a hypervariable region of a non-human species (donor antibody) such as mouse, rat, rabbit or nonhuman primate having the desired specificity, affinity, and capacity. In some instances, framework region (FR) residues of the human immunoglobulin are replaced by corresponding non-human residues. Furthermore, humanized antibodies may comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FRs are those of a human immunoglobulin lo sequence. The humanized antibody optionally will also comprise at least a portion of an immunoglobulin constant region (Fc), typically that of a human immunoglobulin. For further details, see Jones et al., Nature 321:522-525 (1986); Riechmann et al., Nature 332:323-329 (1988); and Presta, Curr. Op. Struct. Biol. 2:593-596 (1992). See also the following review articles and references cited therein: Vaswani and Hamilton, Ann. Allergy, Asthma & Immunol. 1: 105-115 (1998); Harris, Biochem. Soc. Transactions 23:1035-1038 (1995); Hurle and Gross, Curr. Op. Biotech. 5:428-433 (1994).

A "human antibody" is one which comprises an amino acid sequence corresponding to that of an antibody produced by a human and/or has been made using any of the techniques for making human antibodies as disclosed herein. Such techniques include screening human-derived combinatorial libraries, such as phage display libraries (see, *e.g.,* Marks et al., J. Mol. Biol., 222: 581-597 (1991) and Hoogenboom et al., Nucl. Acids Res., 19: 4133-4137 (1991)); using human myeloma and mouse-human heteromyeloma cell lines for the production of human monoclonal antibodies (see, *e.g.,* Kozbor J. Immunol., 133: 3001 (1984); Brodeur et al., Monoclonal Antibody Production Techniques and Applications, pp. 55-93 (Marcel Dekker, Inc., New York, 1987); and Boerner et al., J. Immunol., 147: 86 (1991)); and generating monoclonal antibodies in transgenic animals (e.g., mice) that are capable of producing a full repertoire of human antibodies in the absence of endogenous immunoglobulin production (see, *e.g.,* Jakobovits et al., Proc. Natl. Acad. Sci USA, 90: 2551 (1993); Jakobovits et al., Nature, 362: 255 (1993); Bruggermann et al.,Year in Immunol., 7: 33 (1993)). This definition of a human antibody specifically excludes a humanized antibody comprising antigen-binding residues from a non-human animal.

An "isolated" antibody is one which has been identified and separated and/or recovered from a component of its natural environment. Contaminant components of its natural environment are materials which would interfere with diagnostic or therapeutic uses for the antibody, and may include enzymes, hormones, and other proteinaceous or nonproteinaceous solutes. In preferred embodiments, the antibody will be purified (1) to greater than 95% by weight of antibody as determined by the Lowry method, and most preferably more than 99% by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue or, preferably, silver stain. Isolated antibody includes the antibody in situ within recombinant cells since at least one component of the antibody's natural environment will not be present. Ordinarily, however, isolated antibody will be prepared by at least one purification step.

The term "hypervariable region," "HVR," or "HV," when used herein refers to the regions of an antibody variable domain which are hypervariable in sequence and/or form structurally defined loops. Generally, antibodies comprise six hypervariable regions; three in the VH (HI, H2, H3), and three in the VL (L1, L2, L3). A number of hypervariable region delineations are in use and are encompassed herein. The Kabat Complementarity Determining Regions (CDRs) are based on sequence variability and are the most commonly used (Kabat et al., Sequences of Proteins of Immunological Interest, 5th Ed. Public Health Service, National Institutes of Health, Bethesda, Md. (1991)). Chothia refers instead to the location of the structural loops (Chothia and Lesk J. Mol. Biol. 196:901-917 (1987)). The AbM hypervariable regions represent a compromise between the Kabat CDRs and Chothia structural loops, and are used by Oxford Molecular's AbM antibody modeling software. The "contact" hypervariable regions are based on an analysis of the available complex crystal structures. The residues from each of these HVRs are noted below.

| Loop | Kabat | AbM | Chothia | Contact |
|---|---|---|---|---|
| L1 | L24-L34 | L24-L34 | L26-L32 | L30-L36 |
| L2 | L50-L56 | L50-L56 | L50-L52 | L46-L55 |
| L3 | L89-L97 | L89-L97 | L91-L96 | L89-L96 |
| H1 | H31-H35B | H26-H35B | H26-H32 | H30-H35B (Kabat Numbering) |
| H1 | H31-H35 | H26-H3 5 | H26-H32 | H30-H35 (Chothia Numbering) |
| H2 | H50-H65 | H50-H58 | H53-H55 | H47-H58 |
| H3 | H95-H102 | H95-H102 | H96-H101 | H93-H101 |

Hypervariable regions may comprise "extended hypervariable regions" as follows: 24-36 or 24-34 (L1), 46-56 or 49-56 or 50-56 or 52-56 (L2) and 89-97 (L3) in the VL and 26-35 (HI), 50-65 or 49-65 (H2) and 93-102, 94-102 or 95-102 (H3) in the VH. The variable domain residues are numbered according to Kabat *et al., supra* for each of these definitions.

"Framework" or "FR" residues are those variable domain residues other than the hypervariable region residues as herein defined.

A "human consensus framework" is a framework which represents the most commonly occurring amino acid residue in a selection of human immunoglobulin VL or VH framework sequences. Generally, the selection of human immunoglobulin VL or VH sequences is from a subgroup of variable domain sequences. Generally, the subgroup of sequences is a subgroup as in Kabat *et al.* In one embodiment, for the VL, the subgroup is subgroup kappa I as in Kabat *et al.* In one embodiment, for the VH, the subgroup is subgroup III as in Kabat *et al.*

An "affinity matured" antibody is one with one or more alterations in one or more CDRs thereof which result in an improvement in the affinity of the antibody for antigen, compared to a parent antibody which does not possess those alteration(s). Preferred affinity matured antibodies will have nanomolar or even picomolar affinities for the target antigen. Affinity matured antibodies are produced by procedures known in the art. Marks et al. Bio/Technology 10:779-783 (1992) describes affinity maturation by VH and VL domain shuffling. Random mutagenesis of CDR and/or framework residues is described by: Barbas et al. Proc Nat. Acad. Sci, USA 91:3809-3813 (1994); Schier et al. Gene 169:147-155 (1996); Yelton et al. J. Immunol. 155:1994-2004 (1995); Jackson et al., J. Immunol. 154(7):3310-9 (1995); and Hawkins et al. J. Mol. Biol. 226:889-896 (1992).

"Binding affinity" generally refers to the strength of the sum total of noncovalent interactions between a single binding site of a molecule *(e.g.,* an antibody) and its binding partner *(e.g.,* an antigen). Unless indicated otherwise, as used herein, "binding affinity" refers to intrinsic binding affinity which reflects a 1:1 interaction between members of a binding pair (e.g., antibody and antigen). The affinity of a molecule X for its partner Y can generally be represented by the dissociation constant (Kd). Affinity can be measured by common methods known in the art, including those described herein. Low-affinity antibodies generally bind antigen slowly and tend to dissociate readily, whereas high-affinity antibodies generally bind antigen faster and tend to remain bound longer. A variety of methods of measuring binding affinity are known in the art, any of which can be used for purposes of the present invention.

The term "variable" refers to the fact that certain portions of the variable domains differ extensively in sequence among antibodies and are used in the binding and specificity of each particular antibody for its particular antigen. However, the variability is not evenly distributed throughout the variable domains of antibodies. It is concentrated in three segments called hypervariable regions both in the light chain and the heavy chain variable domains. The more highly conserved portions of variable domains are called the framework regions (FRs). The variable domains of native heavy and light chains each comprise four FRs, largely adopting a β-sheet configuration, connected by three hypervariable regions, which form loops connecting, and in some cases forming part of, the β-sheet structure. The hypervariable regions in each chain are held together in close proximity by the FRs and, with the hypervariable regions from the other chain, contribute to the formation of the antigen-binding site of antibodies (see Kabat et al., Sequences of Proteins of Immunological Interest, 5th Ed. Public Health Service, National Institutes of Health, Bethesda, MD. (1991)). The constant domains are not involved directly in binding an antibody to an antigen, but exhibit various effector functions, such as participation of the antibody in antibody dependent cellular cytotoxicity (ADCC).

Papain digestion of antibodies produces two identical antigen-binding fragments, called "Fab" fragments, each with a single antigen-binding site, and a residual "Fc" fragment, whose name reflects its ability to crystallize readily. Pepsin treatment yields an F(ab')₂ fragment that has two antigen-binding sites and is still capable of cross-linking antigen.

"Fv" is the minimum antibody fragment which contains a complete antigen-recognition and antigen-binding site. This region consists of a dimer of one heavy chain and one light chain variable domain in tight, non-covalent association. It is in this configuration that the three hypervariable regions of each variable domain interact to define an antigen-binding site on the surface of the V_{H}-V_{L} dimer. Collectively, the six hypervariable regions confer antigen-binding specificity to the antibody. However, even a single variable domain (or half of an Fv comprising only three hypervariable regions specific for an antigen) has the ability to recognize and bind antigen, although at a lower affinity than the entire binding site.

The Fab fragment also contains the constant domain of the light chain and the first constant domain (CH1) of the heavy chain. Fab= fragments differ from Fab fragments by the addition of a few residues at the carboxy terminus of the heavy chain CH1 domain including one or more cysteines from the antibody hinge region. Fab'-SH is the designation herein for Fab' in which the cysteine residue(s) of the constant domains bear at least one free thiol group. F(ab')₂ antibody fragments originally were produced as pairs of Fab' fragments which have hinge cysteines between them. Other chemical couplings of antibody fragments are also known.

The "light chains" of antibodies from any vertebrate species can be assigned to one of two clearly distinct types, called kappa (κ) and lambda (λ), based on the amino acid sequences of their constant domains.

Depending on the amino acid sequences of the constant domains of their heavy chains, antibodies (immunoglobulins) can be assigned to different classes. There are five major classes of immunoglobulins: IgA, IgD, IgE, IgG, and IgM, and several of these may be further divided into subclasses (isotypes), e.g., IgG₁, IgG₂, IgG₃, IgG₄, IgA₁, and IgA₂. The heavy-chain constant domains that correspond to the different classes of immunoglobulins are called α, δ, ε, γ, and µ, respectively. The subunit structures and three-dimensional configurations of different classes of immunoglobulins are well known and described generally in, for example, Abbas et al. Cellular and Mol. Immunology, 4th ed. (W. B. Saunders, Co., 2000). An antibody may be part of a larger fusion molecule, formed by covalent or non-covalent association of the antibody with one or more other proteins or peptides.

The terms "full-length antibody," "intact antibody," and "whole antibody" are used herein interchangeably to refer to an antibody in its substantially intact form, not antibody fragments as defined below. The terms particularly refer to an antibody with heavy chains that contain an Fc region.

A "naked antibody" for the purposes herein is an antibody that is not conjugated to a cytotoxic moiety or radiolabel.

The term "Fc region" herein is used to define a C-terminal region of an immunoglobulin heavy chain, including native sequence Fc regions and variant Fc regions. Although the boundaries of the Fc region of an immunoglobulin heavy chain might vary, the human IgG heavy chain Fc region is usually defined to stretch from an amino acid residue at position Cys226, or from Pro230, to the carboxyl-terminus thereof. The C-terminal lysine (residue 447 according to the EU numbering system) of the Fc region may be removed, for example, during production or purification of the antibody, or by recombinantly engineering the nucleic acid encoding a heavy chain of the antibody. Accordingly, a composition of intact antibodies may comprise antibody populations with all K447 residues removed, antibody populations with no K447 residues removed, and antibody populations having a mixture of antibodies with and without the K447 residue.

Unless indicated otherwise, herein the numbering of the residues in an immunoglobulin heavy chain is that of the EU index as in Kabat et al., Sequences of Proteins of Immunological Interest, 5th Ed. Public Health Service, National Institutes of Health, Bethesda, MD (1991), expressly incorporated herein by reference. The "EU index as in Kabat" refers to the residue numbering of the human IgG1 EU antibody.

A "functional Fc region" possesses an "effector function" of a native sequence Fc region. Exemplary "effector functions" include C1q binding; complement dependent cytotoxicity; Fc receptor binding; antibody-dependent cell-mediated cytotoxicity (ADCC); phagocytosis; down regulation of cell surface receptors *(e.g.,* B cell receptor; BCR), etc. Such effector functions generally require the Fc region to be combined with a binding domain *(e.g.,* an antibody variable domain) and can be assessed using various assays as herein disclosed, for example.

A "native sequence Fc region" comprises an amino acid sequence identical to the amino acid sequence of an Fc region found in nature. Native sequence human Fc regions include a native sequence human IgG1 Fc region (non-A and A allotypes); native sequence human IgG2 Fc region; native sequence human IgG3 Fc region; and native sequence human IgG4 Fc region as well as naturally occurring variants thereof.

A "variant Fc region" comprises an amino acid sequence which differs from that of a native sequence Fc region by virtue of at least one amino acid modification, preferably one or more amino acid substitution(s). Preferably, the variant Fc region has at least one amino acid substitution compared to a native sequence Fc region or to the Fc region of a parent polypeptide, *e.g*., from about one to about ten amino acid substitutions, and preferably from about one to about five amino acid substitutions in a native sequence Fc region or in the Fc region of the parent polypeptide. The variant Fc region herein will preferably possess at least about 80% homology with a native sequence Fc region and/or with an Fc region of a parent polypeptide, and most preferably at least about 90% homology therewith, more preferably at least about 95% homology therewith.

Depending on the amino acid sequence of the constant domain of their heavy chains, intact antibodies can be assigned to different "classes." There are five major classes of intact antibodies: IgA, IgD, IgE, IgG, and IgM, and several of these may be further divided into "subclasses" (isotypes), *e.g.*, IgG1, IgG2, IgG3, IgG4, IgA, and IgA2. The heavy-chain constant domains that correspond to the different classes of antibodies are called α, δ, ε, γ, and µ, respectively. The subunit structures and three-dimensional configurations of different classes of immunoglobulins are well known.

"Antibody-dependent cell-mediated cytotoxicity" and "ADCC" refer to a cell-mediated reaction in which nonspecific cytotoxic cells that express Fc receptors (FcRs) (*e.g.* Natural Killer (NK) cells, neutrophils, and macrophages) recognize bound antibody on a target cell and subsequently cause lysis of the target cell. The primary cells for mediating ADCC, NK cells, express FcyRIII only, whereas monocytes express FcyRI, FcyRII and FcyRIII. FcR expression on hematopoietic cells in summarized is Table 3 on page 464 of Ravetch and Kinet, Annu. Rev. Immunol 9:457-92 (1991). To assess ADCC activity of a molecule of interest, an *in vitro* ADCC assay, such as that described in U.S. Patent No. 5,500,362 or 5,821,337 may be performed. Useful effector cells for such assays include peripheral blood mononuclear cells (PBMC) and Natural Killer (NK) cells. Alternatively, or additionally, ADCC activity of the molecule of interest may be assessed *in vivo, e.g.,* in a animal model such as that disclosed in Clynes et al. PNAS (USA) 95:652-656 (1998).

"Human effector cells" are leukocytes which express one or more FcRs and perform effector functions. Preferably, the cells express at least FcγRIII and perform ADCC effector function. Examples of human leukocytes which mediate ADCC include peripheral blood mononuclear cells (PBMC), natural killer (NK) cells, monocytes, cytotoxic T cells and neutrophils; with PBMCs and NK cells being preferred. The effector cells may be isolated from a native source thereof, *e.g.,* from blood or PBMCs as described herein.

The terms "Fc receptor" or "FcR" are used to describe a receptor that binds to the Fc region of an antibody. The preferred FcR is a native sequence human FcR. Moreover, a preferred FcR is one which binds an IgG antibody (a gamma receptor) and includes receptors of the FcyRI, FcyRII, and FcyRIII subclasses, including allelic variants and alternatively spliced forms of these receptors. FcyRII receptors include FcyRIIA (an "activating receptor") and FcyRIIB (an "inhibiting receptor"), which have similar amino acid sequences that differ primarily in the cytoplasmic domains thereof. Activating receptor FcyRIIA contains an immunoreceptor tyrosine-based activation motif (ITAM) in its cytoplasmic domain. Inhibiting receptor FcyRIIB contains an immunoreceptor tyrosine-based inhibition motif (ITIM) in its cytoplasmic domain (see review M. in Daëron, Annu. Rev. Immunol. 15:203-234 (1997)). FcRs are reviewed in Ravetch and Kinet, Annu. Rev. Immunol 9:457-92 (1991); Capel et al., Immunomethods 4:25-34 (1994); and de Haas et al., J. Lab. Clin. Med. 126:330-41 (1995). Other FcRs, including those to be identified in the future, are encompassed by the term "FcR" herein. The term also includes the neonatal receptor, FcRn, which is responsible for the transfer of maternal IgGs to the fetus (Guyer et al., J. Immunol. 117:587 (1976) and Kim et al., J. Immunol. 24:249 (1994)), and regulates homeostasis of immunoglobulins. Antibodies with improved binding to the neonatal Fc receptor (FcRn), and increased half-lives, are described in WO00/42072 (Presta, L.) and US2005/0014934A1 (Hinton et al.). These antibodies comprise an Fc region with one or more substitutions therein which improve binding of the Fc region to FcRn. For example, the Fc region may have substitutions at one or more of positions 238, 250, 256, 265, 272, 286, 303, 305, 307, 311, 312, 314, 317, 340, 356, 360, 362, 376, 378, 380, 382, 413, 424, 428 or 434 (EU numbering of residues). The preferred Fc region-comprising antibody variant with improved FcRn binding comprises amino acid substitutions at one, two or three of positions 307, 380 and 434 of the Fc region thereof (EU numbering of residues).

"Single-chain Fv" or "scFv" antibody fragments comprise the V_{H} and V_{L} domains of antibody, wherein these domains are present in a single polypeptide chain. Preferably, the Fv polypeptide further comprises a polypeptide linker between the V_{H} and V_{L} domains which enables the scFv to form the desired structure for antigen binding. For a review of scFv see Plückthun in The Pharmacology of Monoclonal Antibodies, vol. 113, Rosenburg and Moore eds., Springer-Verlag, New York, pp. 269-315 (1994). HER2 antibody scFv fragments are described in WO93/16185; U.S. Patent No. 5,571,894; and U.S. Patent No. 5,587,458.

The term "diabodies" refers to small antibody fragments with two antigen-binding sites, which fragments comprise a variable heavy domain (V_{H}) connected to a variable light domain (V_{L}) in the same polypeptide chain (V_{H} - V_{L}). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger et al., Proc. Natl. Acad. Sci. USA, 90:6444-6448 (1993).

An "affinity matured" antibody is one with one or more alterations in one or more hypervariable regions thereof which result an improvement in the affinity of the antibody for antigen, compared to a parent antibody which does not possess those alteration(s). Preferred affinity matured antibodies will have nanomolar or even picomolar affinities for the target antigen. Affinity matured antibodies are produced by procedures known in the art. Marks et al. Bio/Technology 10:779-783 (1992) describes affinity maturation by VH and VL domain shuffling. Random mutagenesis of CDR and/or framework residues is described by: Barbas et al. Proc Nat. Acad. Sci, USA 91:3809-3813 (1994); Schier et al. Gene 169:147-155 (1995); Yelton et al. J. Immunol. 155:1994-2004 (1995); Jackson et al., J. Immunol. 154(7):3310-9 (1995); and Hawkins et al, J. Mol. Biol. 226:889-896 (1992).

An "amino acid sequence variant" antibody herein is an antibody with an amino acid sequence which differs from a main species antibody. Ordinarily, amino acid sequence variants will possess at least about 70% homology with the main species antibody, and preferably, they will be at least about 80%, more preferably at least about 90% homologous with the main species antibody. The amino acid sequence variants possess substitutions, deletions, and/or additions at certain positions within or adjacent to the amino acid sequence of the main species antibody. Examples of amino acid sequence variants herein include an acidic variant (e.g., deamidated antibody variant), a basic variant, an antibody with an amino-terminal leader extension (e.g. VHS-) on one or two light chains thereof, an antibody with a C-terminal lysine residue on one or two heavy chains thereof, etc, and includes combinations of variations to the amino acid sequences of heavy and/or light chains. The antibody variant of particular interest herein is the antibody comprising an amino-terminal leader extension on one or two light chains thereof, optionally further comprising other amino acid sequence and/or glycosylation differences relative to the main species antibody.

A "glycosylation variant" antibody herein is an antibody with one or more carbohydrate moieties attached thereto which differ from one or more carbohydrate moieties attached to a main species antibody. Examples of glycosylation variants herein include antibody with a G1 or G2 oligosaccharide structure, instead a G0 oligosaccharide structure, attached to an Fc region thereof, antibody with one or two carbohydrate moieties attached to one or two light chains thereof, antibody with no carbohydrate attached to one or two heavy chains of the antibody, etc, and combinations of glycosylation alterations. Where the antibody has an Fc region, an oligosaccharide structure may be attached to one or two heavy chains of the antibody, e.g. at residue 299 (298, EU numbering of residues).

The term "cytotoxic agent" as used herein refers to a substance that inhibits or prevents the function of cells and/or causes destruction of cells. The term is intended to include radioactive isotopes (e.g. At²¹¹, I¹³¹, I¹²⁵, Y⁹⁰, Re¹⁸⁶, Re¹⁸⁸, Sm¹⁵³, Bi²¹², P³² and radioactive isotopes of Lu), chemotherapeutic agents, and toxins such as small molecule toxins or enzymatically active toxins of bacterial, fungal, plant or animal origin, including fragments and/or variants thereof.

The term "cytokine" is a generic term for proteins released by one cell population which act on another cell as intercellular mediators. Examples of such cytokines are lymphokines, monokines, and traditional polypeptide hormones. Included among the cytokines are growth hormone such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor; fibroblast growth factor; prolactin; placental lactogen; tumor necrosis factor-a and -β; mullerian-inhibiting substance; mouse gonadotropin-associated peptide; inhibin; activin; vascular endothelial growth factor; integrin; thrombopoietin (TPO); nerve growth factors such as NGF-β; platelet-growth factor; transforming growth factors (TGFs) such as TGF-α and TGF-β; insulin-like growth factor-I and -II; erythropoietin (EPO); osteoinductive factors; interferons such as interferon-a, -β, and -γ; colony stimulating factors (CSFs) such as macrophage-CSF (M-CSF); granulocyte-macrophage-CSF (GM-CSF); and granulocyte-CSF (G-CSF); interleukins (ILs) such as IL-1, IL-1α, IL-2, IL-3, IL-4, IL-5, IL-6, IL-7, IL-8, IL-9, IL-10, IL-11, IL-12; a tumor necrosis factor such as TNF-α or TNF-β; and other polypeptide factors including LIF and kit ligand (KL). As used herein, the term cytokine includes proteins from natural sources or from recombinant cell culture and biologically active equivalents of the native sequence cytokines.

The term "immunosuppressive agent" as used herein for adjunct therapy refers to substances that act to suppress or mask the immune system of the subject being treated herein. This would include substances that suppress cytokine production, down-regulate or suppress self-antigen expression, or mask the MHC antigens. Examples of such agents include 2-amino-6-aryl-5-substituted pyrimidines (see U.S. Patent No. 4,665,077); non-steroidal anti-inflammatory drugs (NSAIDs); ganciclovir; tacrolimus; glucocorticoids such as cortisol or aldosterone; anti-inflammatory agents such as a cyclooxygenase inhibitor; a 5-lipoxygenase inhibitor; or a leukotriene receptor antagonist; purine antagonists such as azathioprine or mycophenolate mofetil (MMF); alkylating agents such as cyclophosphamide; bromocryptine; danazol; dapsone; glutaraldehyde (which masks the MHC antigens, as described in U.S. Patent No. 4,120,649); anti-idiotypic antibodies for MHC antigens and MHC fragments; cyclosporine; 6 mercaptopurine; steroids such as corticosteroids or glucocorticosteroids or glucocorticoid analogs, e.g., prednisone, methylprednisolone, including SOLU-MEDROL.RTM. methylprednisolone sodium succinate, and dexamethasone; dihydrofolate reductase inhibitors such as methotrexate (oral or subcutaneous); anti-malarial agents such as chloroquine and hydroxychloroquine; sulfasalazine; leflunomide; cytokine or cytokine receptor antibodies or antagonists including anti-interferon-alpha, -beta, or -gamma antibodies, anti-tumor necrosis factor(TNF)-alpha antibodies (infliximab (REMICADE.RTM.) or adalimumab), anti-TNF-alpha immunoadhesin (etanercept), anti-TNF-beta antibodies, anti-interleukin-2 (IL-2) antibodies and anti-IL-2 receptor antibodies, and anti-interleukin-6 (IL-6) receptor antibodies and antagonists; anti-LFA-1 antibodies, including anti-CD 11a and anti-CD 18 antibodies; anti-L3T4 antibodies; heterologous anti-lymphocyte globulin; pan-T antibodies, preferably anti-CD3 or anti-CD4/CD4a antibodies; soluble peptide containing a LFA-3 binding domain (WO 90/08187 published Jul. 26, 1990); streptokinase; transforming growth factor-beta (TGF-beta); streptodomase; RNA or DNA from the host; FK506; RS-61443; chlorambucil; deoxyspergualin; rapamycin; T-cell receptor (Cohen et al., U.S. Patent No. 5,114,721); T-cell receptor fragments (Offner et al., Science, 251: 430-432 (1991); WO 90/11294; Ianeway, Nature, 341: 482 (1989); and WO 91/01133); BAFF antagonists such as BAFF or BR3 antibodies or immunoadhesins and zTNF4 antagonists (for review, see Mackay and Mackay, Trends Immunol., 23:113-5 (2002) and see also definition below); biologic agents that interfere with T cell helper signals, such as anti-CD40 receptor or anti-CD40 ligand (CD154), including blocking antibodies to CD40-CD40 ligand.(e.g., Durie et al., Science, 261: 1328-30 (1993); Mohan et al., J. Immunol., 154: 1470-80 (1995)) and CTLA4-Ig (Finck et al., Science, 265: 1225-7 (1994)); and T-cell receptor antibodies (EP 340,109) such as T10B9.

The term "ameliorates" or "amelioration" as used herein refers to a decrease, reduction or elimination of a condition, disease, disorder, or phenotype, including an abnormality or symptom.

A "symptom" of a disease or disorder (e.g., inflammatory bowel disease, e.g., ulcerative colitis or Crohn's disease) is any morbid phenomenon or departure from the normal in structure, function, or sensation, experienced by a subject and indicative of disease.

The expression "therapeutically effective amount" refers to an amount that is effective for preventing, ameliorating, or treating a disease or disorder (e.g., inflammatory bowel disease, e.g., ulcerative colitis or Crohn's disease). For example, a "therapeutically effective amount" of an antibody refers to an amount of the antibody that is effective for preventing, ameliorating, or treating the specified disease or disorder. Similarly, a "therapeutically effective amount" of a combination of an antibody and a second compound refers to an amount of the antibody and an amount of the second compound that, in combination, is effective for preventing, ameliorating, or treating the specified disease or disorder.

It is to be understood that the terminology "a combination of' two compounds does not mean that the compounds have to be administered in admixture with each other. Thus, treatment with or use of such a combination encompasses a mixture of the compounds or separate administration of the compounds, and includes administration on the same day or different days. Thus the terminology "combination" means two or more compounds are used for the treatment, either individually or in admixture with each other. When an antibody and a second compound, for example, are administered in combination to a subject, the antibody is present in the subject at a time when the second compound is also present in the subject, whether the antibody and second compound are administered individually or in admixture to the subject. In certain embodiments, a compound other than the antibody is administered prior to the antibody. In certain embodiments, a compound other than the antibody is administered after the antibody.

For the purposes herein, "tumor necrosis factor-alpha (TNF-alpha)" refers to a human TNF-alpha molecule comprising the amino acid sequence as described in Pennica et al., Nature, 312:721 (1984) or Aggarwal et al., JBC, 260:2345 (1985).

A "TNF-alpha inhibitor" herein is an agent that inhibits, to some extent, a biological function of TNF-alpha, generally through binding to TNF-alpha and neutralizing its activity. Examples of TNF inhibitors specifically contemplated herein are etanercept (ENBREL^{®}), infliximab (REMICADE^{®}), adalimumab (HUMIRA^{®}), golimumab (SIMPONITM), and certolizumab pegol (CIMZIA^{®}).

"Corticosteroid" refers to any one of several synthetic or naturally occurring substances with the general chemical structure of steroids that mimic or augment the effects of the naturally occurring corticosteroids. Examples of synthetic corticosteroids include prednisone, prednisolone (including methylprednisolone), dexamethasone triamcinolone, and betamethasone.

An "antagonist" refers to a molecule capable of neutralizing, blocking, inhibiting, abrogating, reducing or interfering with the activities of a particular or specified protein, including its binding to one or more receptors in the case of a ligand or binding to one or more ligands in case of a receptor. Antagonists include antibodies and antigen-binding fragments thereof, proteins, peptides, glycoproteins, glycopeptides, glycolipids, polysaccharides, oligosaccharides, nucleic acids, bioorganic molecules, peptidomimetics, pharmacological agents and their metabolites, transcriptional and translation control sequences, and the like. Antagonists also include small molecule inhibitors of the protein, and fusion proteins, receptor molecules and derivatives which bind specifically to the protein thereby sequestering its binding to its target, antagonist variants of the protein, antisense molecules directed to the protein, RNA aptamers, and ribozymes against the protein.

The term "detection" includes any means of detecting, including direct and indirect detection.

A variety of additional terms are defined or otherwise characterized herein.

### II. COMPOSITIONS AND METHODS

### A. Beta7 integrin Antagonists

The present invention is directed to methods of predicting the responsiveness of a patient to the treatment of beta7 integrin antagonists. Examples of potential antagonists include an oligonucleotide that binds to the fusions of immunoglobulin with beta7 integrin, and, in particular, antibodies including, without limitation, poly- and monoclonal antibodies and antibody fragments, single-chain antibodies, anti-idiotypic antibodies, and chimeric or humanized versions of such antibodies or fragments, as well as human antibodies and antibody fragments. Alternatively, a potential antagonist may be a closely related protein, for example, a mutated form of the beta7 integrin that recognizes the ligand but imparts no effect, thereby competitively inhibiting the action of the beta7 integrin.

Another potential beta7 integrin antagonist is an antisense RNA or DNA construct prepared using antisense technology, where, e.g., an antisense RNA or DNA molecule acts to block directly the translation of mRNA by hybridizing to targeted mRNA and preventing protein translation. Antisense technology can be used to control gene expression through triple-helix formation or antisense DNA or RNA, both of which methods are based on binding of a polynucleotide to DNA or RNA. For example, the 5' coding portion of the polynucleotide sequence, which encodes the beta7 integrin herein, is used to design an antisense RNA oligonucleotide of from about 10 to 40 base pairs in length. A DNA oligonucleotide is designed to be complementary to a region of the gene involved in transcription (triple helix--see Lee et al., Nucl. Acids Res., 6:3073 (1979); Cooney et al., Science, 241: 456 (1988); Dervan et al., Science, 251:1360 (1991)), thereby preventing transcription and the production of the beta7 integrin. The antisense RNA oligonucleotide hybridizes to the mRNA *in vivo* and blocks translation of the mRNA molecule into beta7 integrin protein (antisense--Okano, Neurochem., 56:560 (1991); Oligodeoxynucleotides as Antisense Inhibitors of Gene Expression (CRC Press: Boca Raton, Fla., 1988). The oligonucleotides described above can also be delivered to cells such that the antisense RNA or DNA may be expressed *in vivo* to inhibit production of the PRO polypeptide. When antisense DNA is used, oligodeoxyribonucleotides derived from the translation-initiation site, e.g., between about -10 and +10 positions of the target gene nucleotide sequence, are preferred.

Other potential antagonists include small molecules that bind to the active site, the ligand or binding molecule binding site, thereby blocking the normal biological activity of the beta7 integrin. Examples of small molecules include, but are not limited to, small peptides or peptide-like molecules, preferably soluble peptides, and synthetic non-peptidyl organic or inorganic compounds.

Ribozymes are enzymatic RNA molecules capable of catalyzing the specific cleavage of RNA. Ribozymes act by sequence-specific hybridization to the complementary target RNA, followed by endonucleolytic cleavage. Specific ribozyme cleavage sites within a potential RNA target can-be identified by known techniques. For further details see, *e.g.,* Rossi, Current Biology, 4:469-471 (1994), and PCT Publication No. WO 97/33551 (published Sep. 18, 1997).

Nucleic acid molecules in triple-helix formation used to inhibit transcription should be single-stranded and composed of deoxynucleotides. The base composition of these oligonucleotides is designed such that it promotes triple-helix formation via Hoogsteen base-pairing rules, which generally require sizeable stretches of purines or pyrimidines on one strand of a duplex. For further details see, *e.g.,* PCT Publication No. WO 97/33551. These small molecules can be identified by any one or more of the screening assays discussed hereinabove and/or by any other screening techniques well known for those skilled in the art.

Screening assays for antagonists are designed to identify compounds that bind or complex with the beta7 integrin encoded by the genes identified herein, or otherwise interfere with the interaction of the encoded polypeptides with other cellular proteins. Such screening assays will include assays amenable to high-throughput screening of chemical libraries, making them particularly suitable for identifying small molecule drug candidates.

The assays can be performed in a variety of formats, including protein-protein binding assays, biochemical screening assays, immunoassays, and cell-based assays, which are well characterized in the art.

### B. Anti-Beta7 integrin Antibodies

In one embodiment, the beta7 integrin antagonists are anti-beta7 antibodies. Exemplary antibodies include polyclonal, monoclonal, humanized, human, bispecific, and heteroconjugate antibodies, etc., as described below.

### 1. Polyclonal Antibodies

Polyclonal antibodies are preferably raised in animals by multiple subcutaneous (sc) or intraperitoneal (ip) injections of the relevant antigen and an adjuvant. It may be useful to conjugate the relevant antigen to a protein that is immunogenic in the species to be immunized, e.g., keyhole limpet hemocyanin, serum albumin, bovine thyroglobulin, or soybean trypsin inhibitor using a bifunctional or derivatizing agent, for example, maleimidobenzoyl sulfosuccinimide ester (conjugation through cysteine residues), N-hydroxysuccinimide (through lysine residues), glutaraldehyde, succinic anhydride, SOCh, or R¹N=C=NR, where R and R¹ are different alkyl groups.

Animals are immunized against the antigen, immunogenic conjugates, or derivatives by combining, e.g., 100 µg or 5 µg of the protein or conjugate (for rabbits or mice, respectively) with 3 volumes of Freund's complete adjuvant and injecting the solution intradermally at multiple sites. One month later the animals are boosted with 1/5 to 1/10 the original amount of peptide or conjugate in Freund's complete adjuvant by subcutaneous injection at multiple sites. Seven to 14 days later the animals are bled and the serum is assayed for antibody titer. Animals are boosted until the titer plateaus. Preferably, the animal is boosted with the conjugate of the same antigen, but conjugated to a different protein and/or through a different cross-linking reagent. Conjugates also can be made in recombinant cell culture as protein fusions. Also, aggregating agents such as alum are suitably used to enhance the immune response.

### 2. Monoclonal Antibodies

Monoclonal antibodies may be made using the hybridoma method first described by Kohler et al., Nature, 256:495 (1975), or may be made by recombinant DNA methods (U.S. Patent No. 4,816,567).

In the hybridoma method, a mouse or other appropriate host animal, such as a hamster, is immunized as described above to elicit lymphocytes that produce or are capable of producing antibodies that will specifically bind to the protein used for immunization. Alternatively, lymphocytes may be immunized *in vitro.* After immunization, lymphocytes are isolated and then fused with a myeloma cell line using a suitable fusing agent, such as polyethylene glycol, to form a hybridoma cell (Goding, Monoclonal Antibodies: Principles and Practice, pp. 59-103 (Academic Press, 1986)).

The hybridoma cells thus prepared are seeded and grown in a suitable culture medium which medium preferably contains one or more substances that inhibit the growth or survival of the unfused, parental myeloma cells (also referred to as fusion partner). For example, if the parental myeloma cells lack the enzyme hypoxanthine guanine phosphoribosyl transferase (HGPRT or HPRT), the selective culture medium for the hybridomas typically will include hypoxanthine, aminopterin, and thymidine (HAT medium), which substances prevent the growth of HGPRT-deficient cells.

Preferred fusion partner myeloma cells are those that fuse efficiently, support stable high-level production of antibody by the selected antibody-producing cells, and are sensitive to a selective medium that selects against the unfused parental cells. Preferred myeloma cell lines are murine myeloma lines, such as those derived from MOPC-21 and MPC-11 mouse tumors available from the Salk Institute Cell Distribution Center, San Diego, Calif. USA, and SP-2 and derivatives e.g., X63-Ag8-653 cells available from the American Type Culture Collection, Manassas, Va., USA. Human myeloma and mouse-human heteromyeloma cell lines also have been described for the production of human monoclonal antibodies (Kozbor, J. Immunol., 133:3001 (1984); and Brodeur et al., Monoclonal Antibody Production Techniques and Applications, pp. 51-63 (Marcel Dekker, Inc., New York, 1987)).

Culture medium in which hybridoma cells are growing is assayed for production of monoclonal antibodies directed against the antigen. Preferably, the binding specificity of monoclonal antibodies produced by hybridoma cells is determined by immunoprecipitation or by an *in vitro* binding assay, such as radioimmunoassay (RIA) or enzyme-linked immunosorbent assay (ELISA).

The binding affinity of the monoclonal antibody can, for example, be determined by the Scatchard analysis described in Munson et al., Anal. Biochem., 107:220 (1980). Once hybridoma cells that produce antibodies of the desired specificity, affinity, and/or activity are identified, the clones may be subcloned by limiting dilution procedures and grown by standard methods (Goding, Monoclonal Antibodies: Principles and Practice, pp. 59-103 (Academic Press, 1986)). Suitable culture media for this purpose include, for example, D-MEM or RPMI-1640 medium. In addition, the hybridoma cells may be grown *in vivo* as ascites tumors in an animal e.g., by i.p. injection of the cells into mice. The monoclonal antibodies secreted by the subclones are suitably separated from the culture medium, ascites fluid, or serum by conventional antibody purification procedures such as, for example, affinity chromatography (e.g., using protein A or protein G-Sepharose) or ion-exchange chromatography, hydroxylapatite chromatography, gel electrophoresis, dialysis, etc.

DNA encoding the monoclonal antibodies is readily isolated and sequenced using conventional procedures (e.g., by using oligonucleotide probes that are capable of binding specifically to genes encoding the heavy and light chains of murine antibodies). The hybridoma cells serve as a preferred source of such DNA. Once isolated, the DNA may be placed into expression vectors, which are then transfected into host cells such as *E. coli* cells, simian COS cells, Chinese Hamster Ovary (CHO) cells, or myeloma cells that do not otherwise produce antibody protein, to obtain the synthesis of monoclonal antibodies in the recombinant host cells. Review articles on recombinant expression in bacteria of DNA encoding the antibody include Skerra et al., Curr. Opinion in Immunol., 5:256-262 (1993) and Pluckthun, Immunol. Revs. 130:151-188 (1992).

In a further embodiment, monoclonal antibodies or antibody fragments can be isolated from antibody phage libraries generated using the techniques described in McCafferty et al., Nature, 348:552-554 (1990). Clackson et al., Nature, 352:624-628 (1991) and Marks et al., J. Mol. Biol., 222:581-597 (1991) describe the isolation of murine and human antibodies, respectively, using phage libraries. Subsequent publications describe the production of high affinity (nM range) human antibodies by chain shuffling (Marks et al., Bio/Technology, 10:779-783 (1992)), as well as combinatorial infection and *in vivo* recombination as a strategy for constructing very large phage libraries (Waterhouse et al., Nuc. Acids. Res. 21:2265-2266 (1993)). Thus, these techniques are viable alternatives to traditional monoclonal antibody hybridoma techniques for isolation of monoclonal antibodies.

The DNA that encodes the antibody may be modified to produce chimeric or fusion antibody polypeptides, for example, by substituting human heavy chain and light chain constant domain (CH and CL) sequences for the homologous murine sequences (U.S. Patent No. 4,816,567; and Morrison, et al., Proc. Natl. Acad. Sci. USA, 81:6851 (1984)), or by fusing the immunoglobulin coding sequence with all or part of the coding sequence for a non-immunoglobulin polypeptide (heterologous polypeptide). The non-immunoglobulin polypeptide sequences can substitute for the constant domains of an antibody, or they are substituted for the variable domains of one antigen-combining site of an antibody to create a chimeric bivalent antibody comprising one antigen-combining site having specificity for an antigen and another antigen-combining site having specificity for a different antigen.

Exemplary anti-beta7 antibodies are Fib504, Fib 21, 22, 27, 30 (Tidswell, M. J Immunol. 1997 Aug 1;159(3): 1497-505) or humanized derivatives thereof. Humanized antibodies of Fib504 was disclosed in detail in U.S. Patent Publication No. 20060093601 (issued as U.S. Patent No. 7,528,236), the content of which is incorporated by reference in its entirety (also see discussion below).

### 3. Human and Humanized Antibodies

The anti-beta7 integrin antibodies of the invention may further comprise humanized antibodies or human antibodies. Humanized forms of non-human (e.g., murine) antibodies are chimeric immunoglobulins, immunoglobulin chains or fragments thereof (such as Fv, Fab, Fab', F(ab')₂ or other antigen-binding subsequences of antibodies) which contain minimal sequence derived from non-human immunoglobulin. Humanized antibodies include human immunoglobulins (recipient antibody) in which residues from a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Humanized antibodies may also comprise residues which are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin consensus sequence. The humanized antibody optimally also will comprise at least a portion of an immunoglobulin constant region (Fc), typically that of a human immunoglobulin [Jones et al., Nature, 321:522-525 (1986); Riechmann et al., Nature 332:323-329 (1988); and Presta, Curr. Op. Struct. Biol., 2:593-596 (1992)].

Methods for humanizing non-human antibodies are well known in the art. Generally, a humanized antibody has one or more amino acid residues introduced into it from a source which is non-human. These non-human amino acid residues are often referred to as "import" residues, which are typically taken from an "import" variable domain. Humanization can be essentially performed following the method of Winter and co-workers [Jones et al., Nature, 321:522-525 (1986); Riechmann et al., Nature, 332:323-327 (1988); Verhoeyen et al., Science, 239:1534-1536 (1988)], by substituting rodent CDRs or CDR sequences for the corresponding sequences of a human antibody. Accordingly, such "humanized" antibodies are chimeric antibodies (U.S. Patent No. 4,816,567), wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies. The choice of human variable domains, both light and heavy, to be used in making the humanized antibodies is very important to reduce antigenicity and HAMA response (human anti-mouse antibody) when the antibody is intended for human therapeutic use. According to the so-called "best-fit" method, the sequence of the variable domain of a rodent antibody is screened against the entire library of known human variable domain sequences. The human V domain sequence which is closest to that of the rodent is identified and the human framework region (FR) within it accepted for the humanized antibody (Sims et al., J. Immunol. 151:2296 (1993); Chothia et al., J. Mol. Biol., 196:901 (1987)). Another method uses a particular framework region derived from the consensus sequence of all human antibodies of a particular subgroup of light or heavy chains. The same framework may be used for several different humanized antibodies (Carter et al., Proc. Natl. Acad. Sci. USA, 89:4285 (1992); Presta et al., J. Immunol. 151:2623 (1993)). It is further important that antibodies be humanized with retention of high binding affinity for the antigen and other favorable biological properties. To achieve this goal, according to a preferred method, humanized antibodies are prepared by a process of analysis of the parental sequences and various conceptual humanized products using three-dimensional models of the parental and humanized sequences. Three-dimensional immunoglobulin models are commonly available and are familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, *i.e.,* the analysis of residues that influence the ability of the candidate immunoglobulin to bind its antigen. In this way, FR residues can be selected and combined from the recipient and import sequences so that the desired antibody characteristic, such as increased affinity for the target antigen(s), is achieved. In general, the hypervariable region residues are directly and most substantially involved in influencing antigen binding.

Various forms of a humanized Anti-beta7 integrin antibody are contemplated. For example, the humanized antibody may be an antibody fragment, such as a Fab, which is optionally conjugated with one or more cytotoxic agent(s) in order to generate an immunoconjugate. Alternatively, the humanized antibody may be an intact antibody, such as an intact IgG1 antibody.

Exemplary humanized anti-beta7 antibodies include, but are not limited to rhuMAb Beta7, which is a humanized monoclonal antibody against the integrin subunit β7 and was derived from the rat anti-mouse/human monoclonal antibody FIB504 (Andrew et al., 1994 J Immunol 1994;153:3847-61). It has been engineered to include human immunoglobulin IgG1 heavy chain and κ1 light chain frameworks and is produced by Chinese hamster ovary cells. This antibody binds to two integrins, α4β7 (Holzmann et al. 1989 Cell, 1989;56:37-46; Hu et al., 1992, Proc Natl Acad Sci USA 1992;89:8254-8) and αEβ7 (Cepek et al., 1993 J Immunol 1993;150:3459-70), which regulate trafficking and retention of lymphocyte subsets in the gastrointestinal tract and are involved in inflammatory bowel diseases (IBD) such as ulcerative colitis (UC) and Crohn's disease (CD). rhuMAb Beta7 is a potent *in vitro* blocker of the cellular interaction between α4β7 and its ligands (mucosal addressin cell adhesion molecule-1 [MAdCAM]-1, vascular cell adhesion molecule [VCAM]-1, and fibronectin) as well as the interaction between αEβ7 and its ligand (E-cadherin). rhuMAb Beta7 binds reversibly, with similar high affinity, to β7 on lymphocytes from rabbits, cynomolgus monkeys, and humans. It also binds to mouse β7 with high affinity. The amino acid sequence and the make and use of rhuMAb Beta7 and its variants are disclosed in detail in U.S. Patent Application Publication No. 20060093601 (issued as U.S. Patent No. 7,528,236), the content of which is incorporated in its entirety.

FIGS. 1A and 1B depict alignment of sequences of the variable light and heavy chains for the following: light chain human subgroup kappa I consensus sequence (FIG. 1A, SEQ ID NO:12), heavy chain human subgroup III consensus sequence (FIG. 1B, SEQ ID NO:13), rat anti-mouse beta7 antibody (Fib504) variable light chain (FIG. 1A, SEQ ID NO:10), rat anti-mouse beta7 antibody (Fib504) variable heavy chain (FIG. 1B, SEQ ID NO:11), and humanized antibody variants: Humanized hu504Kgraft variable light chain (FIG. 1A, SEQ ID NO:14), humanized hu504K graft variable heavy chain (FIG. 1B, SEQ ID NO:15), variants hu504-5, hu504-16, and hu504-32 (amino acid variations from humanized hu504K graft are indicated in FIG. 1A (light chain) (SEQ ID NOS:22-24, respectively, in order of appearance) and FIG. 1B (heavy chain) for variants hu504-5, hu504-16, and 504-32 (SEQ ID NO:25).

### 4. Human Antibodies

As an alternative to humanization, human antibodies can be generated. For example, it is now possible to produce transgenic animals (e.g., mice) that are capable, upon immunization, of producing a full repertoire of human antibodies in the absence of endogenous immunoglobulin production. For example, it has been described that the homozygous deletion of the antibody heavy-chain joining region (J.sub.H) gene in chimeric and germ-line mutant mice results in complete inhibition of endogenous antibody production. Transfer of the human germ-line immunoglobulin gene array into such germ-line mutant mice will result in the production of human antibodies upon antigen challenge. See, *e.g.,* Jakobovits et al., Proc. Natl. Acad. Sci. USA, 90:2551 (1993); Jakobovits et al., Nature, 362:255-258 (1993); Bruggemann et al., Year in Immuno. 7:33 (1993); U.S. Patent Nos. 5,545,806, 5,569,825, 5,591,669 (all of GenPharm); U.S. Patent No. 5,545,807; and WO 97/17852.

Alternatively, phage display technology (McCafferty et al., Nature 348:552-553 [1990]) can be used to produce human antibodies and antibody fragments *in vitro,* from immunoglobulin variable (V) domain gene repertoires from unimmunized donors. According to this technique, antibody V domain genes are cloned in-frame into either a major or minor coat protein gene of a filamentous bacteriophage, such as M13 or fd, and displayed as functional antibody fragments on the surface of the phage particle. Because the filamentous particle contains a single-stranded DNA copy of the phage genome, selections based on the functional properties of the antibody also result in selection of the gene encoding the antibody exhibiting those properties. Thus, the phage mimics some of the properties of the B-cell. Phage display can be performed in a variety of formats, reviewed in, *e.g.,* Johnson, Kevin S. and Chiswell, David J., Current Opinion in Structural Biology 3:564-571 (1993). Several sources of V-gene segments can be used for phage display. Clackson et al., Nature, 352:624-628(1991) isolated a diverse array of anti-oxazolone antibodies from a small random combinatorial library of V genes derived from the spleens of immunized mice. A repertoire of V genes from unimmunized human donors can be constructed and antibodies to a diverse array of antigens (including self-antigens) can be isolated essentially following the techniques described by Marks et al., J. Mol. Biol. 222:581-597 (1991), or Griffith et al., EMBO J. 12:725-734 (1993). See, also, U.S. Patent Nos. 5,565,332 and 5,573,905.

As discussed above, human antibodies may also be generated by *in vitro* activated B cells (see U.S. Patent Nos. 5,567,610 and 5,229,275).

### 5. Antibody Fragments

In certain circumstances there are advantages of using antibody fragments, rather than whole antibodies. The smaller size of the fragments allows for rapid clearance, and may lead to improved access to solid tumors.

Various techniques have been developed for the production of antibody fragments. Traditionally, these fragments were derived via proteolytic digestion of intact antibodies (see, *e.g.,* Morimoto et al., Journal of Biochemical and Biophysical Methods 24:107-117 (1992) and Brennan et al., Science, 229:81 (1985)). However, these fragments can now be produced directly by recombinant host cells. For example, the antibody fragments can be isolated from the antibody phage libraries discussed above. Alternatively, Fab'-SH fragments can be directly recovered from *E. coli* and chemically coupled to form F(ab')₂ fragments (Carter et al., Bio/Technology 10:163-167 (1992)). According to another approach, F(ab')₂ fragments can be isolated directly from recombinant host cell culture. Other techniques for the production of antibody fragments will be apparent to the skilled practitioner. In other embodiments, the antibody of choice is a single chain Fv fragment (scFv). See WO 93/16185; US Patent No. 5,571,894; and US Patent No. 5,587,458. The antibody fragment may also be a "linear antibody," e.g., as described in US Patent No. 5,641,870 for example. Such linear antibody fragments may be monospecific or bispecific.

### 6. Bispecific Antibodies

Bispecific antibodies are antibodies that have binding specificities for at least two different epitopes. Exemplary bispecific antibodies may bind to two different epitopes of beta7 integrin as described herein. Other such antibodies may combine a TAT binding site with a binding site for another protein. Alternatively, an anti-Beta7 integrin arm may be combined with an arm which binds to a triggering molecule on a leukocyte such as a T-cell receptor molecule (*e.g.,* CD3), or Fc receptors for IgG (Fc.γR), such as Fc.γRI (CD64), Fc.γRII (CD32) and Fc. γ.RIII (CD16), so as to focus and localize cellular defense mechanisms to the TAT-expressing cell. Bispecific antibodies may also be used to localize cytotoxic agents to cells which express TAT. These antibodies possess a TAT-binding arm and an arm which binds the cytotoxic agent (e.g., saporin, anti-interferon-.alpha., vinca alkaloid, ricin A chain, methotrexate or radioactive isotope hapten). Bispecific antibodies can be prepared as full length antibodies or antibody fragments (e.g., F(ab')₂ bispecific antibodies).

Methods for making bispecific antibodies are known in the art. Traditional production of full length bispecific antibodies is based on the co-expression of two immunoglobulin heavy chain-light chain pairs, where the two chains have different specificities (Millstein et al., Nature 305:537-539 (1983)). Because of the random assortment of immunoglobulin heavy and light chains, these hybridomas (quadromas) produce a potential mixture of 10 different antibody molecules, of which only one has the correct bispecific structure. Purification of the correct molecule, which is usually done by affinity chromatography steps, is rather cumbersome, and the product yields are low. Similar procedures are disclosed in WO 93/08829, and in Traunecker et al., EMBO J. 10:3655-3659 (1991).

According to a different approach, antibody variable domains with the desired binding specificities (antibody-antigen combining sites) are fused to immunoglobulin constant domain sequences. Preferably, the fusion is with an Ig heavy chain constant domain, comprising at least part of the hinge, C_{H2}, and C_{H3} regions. It is preferred to have the first heavy-chain constant region (C_{H1}) containing the site necessary for light chain bonding, present in at least one of the fusions. DNAs encoding the immunoglobulin heavy chain fusions and, if desired, the immunoglobulin light chain, are inserted into separate expression vectors, and are co-transfected into a suitable host cell. This provides for greater flexibility in adjusting the mutual proportions of the three polypeptide fragments in embodiments when unequal ratios of the three polypeptide chains used in the construction provide the optimum yield of the desired bispecific antibody. It is, however, possible to insert the coding sequences for two or all three polypeptide chains into a single expression vector when the expression of at least two polypeptide chains in equal ratios results in high yields or when the ratios have no significant affect on the yield of the desired chain combination.

In a preferred embodiment of this approach, the bispecific antibodies are composed of a hybrid immunoglobulin heavy chain with a first binding specificity in one arm, and a hybrid immunoglobulin heavy chain-light chain pair (providing a second binding specificity) in the other arm. It was found that this asymmetric structure facilitates the separation of the desired bispecific compound from unwanted immunoglobulin chain combinations, as the presence of an immunoglobulin light chain in only one half of the bispecific molecule provides for a facile way of separation. This approach is disclosed in WO 94/04690. For further details of generating bispecific antibodies see, for example, Suresh et al., Methods in Enzymology 121:210 (1986).

According to another approach described in U.S. Patent No. 5,731,168, the interface between a pair of antibody molecules can be engineered to maximize the percentage of heterodimers which are recovered from recombinant cell culture. The preferred interface comprises at least a part of the C.sub.H3 domain. In this method, one or more small amino acid side chains from the interface of the first antibody molecule are replaced with larger side chains (*e.g.,* tyrosine or tryptophan). Compensatory "cavities" of identical or similar size to the large side chain(s) are created on the interface of the second antibody molecule by replacing large amino acid side chains with smaller ones (*e.g.**,*** alanine or threonine). This provides a mechanism for increasing the yield of the heterodimer over other unwanted end-products such as homodimers.

Bispecific antibodies include cross-linked or "heteroconjugate" antibodies. For example, one of the antibodies in the heteroconjugate can be coupled to avidin, the other to biotin. Such antibodies have, for example, been proposed to target immune system cells to unwanted cells (U.S. Patent No. 4,676,980), and for treatment of HIV infection (WO 91/00360, WO 92/200373, and EP 03089). Heteroconjugate antibodies may be made using any convenient cross-linking methods. Suitable cross-linking agents are well known in the art, and are disclosed in U.S. Patent No. 4,676,980, along with a number of cross-linking techniques.

Techniques for generating bispecific antibodies from antibody fragments have also been described in the literature. For example, bispecific antibodies can be prepared using chemical linkage. Brennan et al., Science 229:81 (1985) describe a procedure wherein intact antibodies are proteolytically cleaved to generate F(ab').sub.2 fragments. These fragments are reduced in the presence of the dithiol complexing agent, sodium arsenite, to stabilize vicinal dithiols and prevent intermolecular disulfide formation. The Fab' fragments generated are then converted to thionitrobenzoate (TNB) derivatives: One of the Fab'-TNB derivatives is then reconverted to the Fab'-thiol by reduction with mercaptoethylamine and is mixed with an equimolar amount of the other Fab'-TNB derivative to form the bispecific antibody. The bispecific antibodies produced can be used as agents for the selective immobilization of enzymes.

Recent progress has facilitated the direct recovery of Fab'-SH fragments from E. *coli,* which can be chemically coupled to form bispecific antibodies. Shalaby et al., J. Exp. Med. 175: 217-225 (1992) describe the production of a fully humanized bispecific antibody F(ab')₂ molecule. Each Fab' fragment was separately secreted from *E. coli* and subjected to directed chemical coupling *in vitro* to form the bispecific antibody. The bispecific antibody thus formed was able to bind to cells overexpressing the ErbB2 receptor and normal human T cells, as well as trigger the lytic activity of human cytotoxic lymphocytes against human breast tumor targets. Various techniques for making and isolating bispecific antibody fragments directly from recombinant cell culture have also been described. For example, bispecific antibodies have been produced using leucine zippers. Kostelny et al., J. Immunol. 148(5):1547-1553 (1992). The leucine zipper peptides from the Fos and Jun proteins were linked to the Fab' portions of two different antibodies by gene fusion. The antibody homodimers were reduced at the hinge region to form monomers and then re-oxidized to form the antibody heterodimers. This method can also be utilized for the production of antibody homodimers. The "diabody" technology described by Hollinger et al., Proc. Natl. Acad. Sci. USA 90:6444-6448 (1993) has provided an alternative mechanism for making bispecific antibody fragments. The fragments comprise a V.sub.H connected to a V.sub.L by a linker which is too short to allow pairing between the two domains on the same chain. Accordingly, the V.sub.H and V.sub.L domains of one fragment are forced to pair with the complementary V.sub.L and V.sub.H domains of another fragment, thereby forming two antigen-binding sites. Another strategy for making bispecific antibody fragments by the use of single-chain Fv (sFv) dimers has also been reported. See Gruber et al., J. Immunol., 152:5368 (1994).

Antibodies with more than two valencies are contemplated. For example, trispecific antibodies can be prepared. Tutt et al., J. Immunol. 147:60 (1991).

### 7. Heteroconjugate Antibodies

Heteroconjugate antibodies are also within the scope of the present invention. Heteroconjugate antibodies are composed of two covalently joined antibodies. Such antibodies have, for example, been proposed to target immune system cells to unwanted cells [U.S. Patent No. 4,676,980], and for treatment of HIV infection [WO 91/00360; WO 92/200373; EP 03089]. It is contemplated that the antibodies may be prepared *in vitro* using known methods in synthetic protein chemistry, including those involving crosslinking agents. For example, immunotoxins may be constructed using a disulfide exchange reaction or by forming a thioether bond. Examples of suitable reagents for this purpose include iminothiolate and methyl-4-mercaptobutyrimidate and those disclosed, for example, in U.S. Patent No. 4,676,980.

### 8. Multivalent Antibodies

A multivalent antibody may be internalized (and/or catabolized) faster than a bivalent antibody by a cell expressing an antigen to which the antibodies bind. The antibodies of the present invention can be multivalent antibodies (which are other than of the IgM class) with three or more antigen binding sites (e.g., tetravalent antibodies), which can be readily produced by recombinant expression of nucleic acid encoding the polypeptide chains of the antibody. The multivalent antibody can comprise a dimerization domain and three or more antigen binding sites. The preferred dimerization domain comprises (or consists of) an Fc region or a hinge region. In this scenario, the antibody will comprise an Fc region and three or more antigen binding sites amino-terminal to the Fc region. The preferred multivalent antibody herein comprises (or consists of) three to about eight, but preferably four, antigen binding sites. The multivalent antibody comprises at least one polypeptide chain (and preferably two polypeptide chains), wherein the polypeptide chain(s) comprise two or more variable domains. For instance, the polypeptide chain(s) may comprise VD1-(X1).sub.n-VD2-(X2).sub.n-Fc, wherein VD1 is a first variable domain, VD2 is a second variable domain, Fc is one polypeptide chain of an Fc region, X1 and X2 represent an amino acid or polypeptide, and n is 0 or 1. For instance, the polypeptide chain(s) may comprise: VH-CH1-flexible linker-VH-CH1-Fc region chain; or VH-CH1-VH-CH1-Fc region chain. The multivalent antibody herein preferably further comprises at least two (and preferably four) light chain variable domain polypeptides. The multivalent antibody herein may, for instance, comprise from about two to about eight light chain variable domain polypeptides. The light chain variable domain polypeptides contemplated here comprise a light chain variable domain and, optionally, further comprise a CL domain.

### 9. Effector Function Engineering

It may be desirable to modify the antibody of the invention with respect to effector function, *e.g.,* so as to enhance antigen-dependent cell-mediated cytotoxicity (ADCC) and/or complement dependent cytotoxicity (CDC) of the antibody. This may be achieved by introducing one or more amino acid substitutions in an Fc region of the antibody. Alternatively or additionally, cysteine residue(s) may be introduced in the Fc region, thereby allowing interchain disulfide bond formation in this region. The homodimeric antibody thus generated may have improved internalization capability and/or increased complement-mediated cell killing and antibody-dependent cellular cytotoxicity (ADCC). See Caron et al., J. Exp Med. 176:1191-1195 (1992) and Shopes, B. J. Immunol. 148:2918-2922 (1992). Homodimeric antibodies with enhanced anti-tumor activity may also be prepared using heterobifunctional cross-linkers as described in Wolff et al., Cancer Research 53:2560-2565 (1993). Alternatively, an antibody can be engineered which has dual Fc regions and may thereby have enhanced complement lysis and ADCC capabilities. See Stevenson et al., AntiCancer Drug Design 3:219-230 (1989). To increase the serum half life of the antibody, one may incorporate a salvage receptor binding epitope into the antibody (especially an antibody fragment) as described in U.S. Patent No. 5,739,277, for example. As used herein, the term "salvage receptor binding epitope" refers to an epitope of the Fc region of an IgG molecule (*e.g.,* IgG₁, IgG₂, IgG₃, or IgG₄) that is responsible for increasing the *in vivo* serum half-life of the IgG molecule.

### 10. Immunoconjugates

The antagonist or antibody used in the methods herein is optionally conjugated to another agent, such as a cytotoxic agent, or cytokine.

Conjugation will ordinarily be achieved through a covalent linkage, the precise nature of which will be determined by the targeting molecule and the linking site on the integrin beta7 antagonist or antibody polypeptide. Typically, a non-peptidic agent is modified by the addition of a linker that allows conjugation to anti-beta7 integrin antibody through its amino acid side chains, carbohydrate chains, or reactive groups introduced on antibody by chemical modification. For example, a drug may be attached through the .epsilon.-amino group of a lysine residue, through a free .alpha.-amino group, by disulfide exchange to a cysteine residue, or by oxidation of the 1,2- diols in a carbohydrate chain with periodic acid to allow attachment of drugs containing various nucleophiles through a Schiff-base linkage. See, for example, U.S. Patent No. 4,256,833. Protein modifying agents include amine-reactive reagents (*e.g.,* reactive esters, isothiocyanates, aldehydes, and sulfonyl halides), thiol-reactive reagents (*e.g.,* haloacetyl derivatives and maleimides), and carboxylic acid- and aldehyde-reactive reagents. Integrin beta7 antagonist or antibody polypeptides can be covalently joined to peptidic agents through the use of bifunctional cross-linking reagents. Heterobifunctional reagents are more commonly used and permit the controlled coupling of two different proteins through the use of two different reactive moieties (*e.g.,* amine-reactive plus thiol, iodoacetamide, or maleimide). The use of such linking agents is well known in the art. See, for example, Brinkley, *supra,* and U.S. Patent No. 4,671,958. Peptidic linkers can also be employed. In the alternative, an anti-beta7 integrin antibody polypeptide can be linked to a peptidic moiety through preparation of a fusion polypeptide.

Examples of further bifunctional protein coupling agents include N-succinimidyl-3-(2-pyridyldithiol) propionate (SPDP), succinimidyl-4-(N-maleimidomethyl) cyclohexane-1-carboxylate, iminothiolane (IT), bifunctional derivatives of imidoesters (such as dimethyl adipimidate HCL), active esters (such as disuccinimidyl suberate), aldehydes (such as glutareldehyde), bis-azido compounds (such as bis (p-azidobenzoyl) hexanediamine), bis-diazonium derivatives (such as bis-(p-diazoniumbenzoyl)-ethylenediamine), diisocyanates (such as tolyene 2,6-diisocyanate), and bis-active fluorine compounds (such as 1,5-difluoro-2,4-dinitrobenzene).

### 11. Immunoliposomes

The anti-beta7 integrin antibodies disclosed herein may also be formulated as immunoliposomes. A "liposome" is a small vesicle composed of various types of lipids, phospholipids and/or surfactant which is useful for delivery of a drug to a mammal. The components of the liposome are commonly arranged in a bilayer formation, similar to the lipid arrangement of biological membranes. Liposomes containing the antibody are prepared by methods known in the art, such as described in Epstein et al., Proc. Natl. Acad. Sci. USA 82:3688 (1985); Hwang et al., Proc. Natl Acad. Sci. USA 77:4030 (1980); U.S. Patent Nos. 4,485,045 and 4,544,545; and WO97/38731 published Oct. 23, 1997. Liposomes with enhanced circulation time are disclosed in U.S. Patent No. 5,013,556.

Particularly useful liposomes can be generated by the reverse phase evaporation method with a lipid composition comprising phosphatidylcholine, cholesterol and PEG-derivatized phosphatidylethanolamine (PEG-PE). Liposomes are extruded through filters of defined pore size to yield liposomes with the desired diameter.

Fab' fragments of the antibody of the present invention can be conjugated to the liposomes as described in Martin et al., J. Biol. Chem. 257:286-288 (1982) via a disulfide interchange reaction. A chemotherapeutic agent is optionally contained within the liposome. See Gabizon et al., J. National Cancer Inst. 81(19):1484 (1989).

### 12. Vectors, Host Cells and Recombinant Methods For Antibody Production

Also provided are isolated nucleic acids encoding the anti-beta7 antibodies or polypeptide agents described herein, vectors and host cells comprising the nucleic acids and recombinant techniques for the production of the antibodies.

For recombinant production of the antibody, the nucleic acid encoding it may be isolated and inserted into a replicable vector for further cloning (amplification of the DNA) or for expression. In another embodiment, the antibody may be produced by homologous recombination, *e.g.,* as described in U.S. Patent No. 5,204,244, specifically incorporated herein by reference. DNA encoding the monoclonal antibody is readily isolated and sequenced using conventional procedures (*e.g.,* by using oligonucleotide probes that are capable of binding specifically to genes encoding the heavy and light chains of the antibody). Many vectors are available. The vector components generally include, but are not limited to, one or more of the following: a signal sequence, an origin of replication, one or more marker genes, an enhancer element, a promoter, and a transcription termination sequence, *e.g.,* as described in U.S. Patent No. 5,534,615 issued Jul. 9, 1996 and specifically incorporated herein by reference.

Suitable host cells for cloning or expressing the DNA in the vectors herein are the prokaryote, yeast, or higher eukaryote cells described above. Suitable prokaryotes for this purpose include eubacteria, such as Gram-negative or Gram-positive organisms, for example, Enterobacteriaceae such as Escherichia, *e.g., E. coli,* Enterobacter, Erwinia, Klebsiella, Proteus, Salmonella, *e.g.,* Salmonella typhimurium, Serratia, *e.g.,* Serratia marcescans, and Shigella, as well as Bacilli such as B. subtilis and B. licheniformis (*e.g.*, B. licheniformis 41P disclosed in DD 266,710 published 12 Apr. 1989), Pseudomonas such as P. aeruginosa, and Streptomyces. One preferred *E. coli* cloning host is *E. coli* 294 (ATCC 31,446), although other strains such as *E. coli* B, *E. coli* X1776 (ATCC 31,537), and *E. coli* W3110 (ATCC 27,325) are suitable. These examples are illustrative rather than limiting.

In addition to prokaryotes, eukaryotic microbes such as filamentous fungi or yeast are suitable cloning or expression hosts for anti-beta7 integrin antibody-encoding vectors. Saccharomyces cerevisiae, or common baker's yeast, is the most commonly used among lower eukaryotic host microorganisms. However, a number of other genera, species, and strains are commonly available and useful herein, such as Schizosaccharomyces pombe; Kluyveromyces hosts such as, *e.g.,* K. lactis, K. fragilis (ATCC 12,424), K. bulgaricus (ATCC 16,045), K. wickeramii (ATCC 24,178), K. waltii (ATCC 56,500), K. drosophilarum (ATCC 36,906), K. thermotolerans, and K. marxianus; yarrowia (EP 402,226); Pichia pastoris (EP 183,070); Candida; Trichoderma reesia (EP 244,234); Neurospora crassa; Schwanniomyces such as Schwanniomyces occidentalis; and filamentous fungi such as, *e.g.,* Neurospora, Penicillium, Tolypocladium, and Aspergillus hosts such as A. nidulans and A. niger.

Suitable host cells for the expression of glycosylated anti-Beta7 antibody are derived from multicellular organisms. Examples of invertebrate cells include plant and insect cells. Numerous baculoviral strains and variants and corresponding permissive insect host cells from hosts such as Spodoptera frugiperda (caterpillar), Aedes aegypti (mosquito), Aedes albopictus (mosquito), Drosophila melanogaster (fruitfly), and Bombyx mori have been identified. A variety of viral strains for transfection are publicly available, e.g., the L-1 variant of Autographa californica NPV and the Bm-5 strain of Bombyx mori NPV, and such viruses may be used as the virus herein according to the present invention, particularly for transfection of Spodoptera frugiperda cells. Plant cell cultures of cotton, corn, potato, soybean, petunia, tomato, and tobacco can also be utilized as hosts.

However, interest has been greatest in vertebrate cells, and propagation of vertebrate cells in culture (tissue culture) has become a routine procedure. Examples of useful mammalian host cell lines are monkey kidney CV1 line transformed by SV40 (COS-7, ATCC CRL 1651); human embryonic kidney line (293 or 293 cells subcloned for growth in suspension culture, Graham et al., J. Gen Virol. 36:59 (1977)); baby hamster kidney cells (BHK, ATCC CCL 10); Chinese hamster ovary cells/-DHFR(CHO, Urlaub et al., Proc. Natl. Acad. Sci. USA 77:4216 (1980)); mouse sertoli cells (TM4, Mather, Biol. Reprod. 23:243-251 (1980)); monkey kidney cells (CV1 ATCC CCL 70); African green monkey kidney cells (VERO-76, ATCC CRL-1587); human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); TRI cells (Mather et al., Annals N.Y. Acad. Sci. 383:44-68 (1982)); MRC 5 cells; FS4 cells; and a human hepatoma line (Hep G2).

Host cells are transformed with the above-described expression or cloning vectors for anti-beta7 integrin antibody production and cultured in conventional nutrient media modified as appropriate for inducing promoters, selecting transformants, or amplifying the genes encoding the desired sequences.

The host cells used to produce the anti-beta7 integrin antibody of this invention may be cultured in a variety of media. Commercially available media such as Ham's F10 (Sigma), Minimal Essential Medium ((MEM), (Sigma), RPMI-1640 (Sigma), and Dulbecco's Modified Eagle's Medium ((DMEM), Sigma) are suitable for culturing the host cells. In addition, any of the media described in Ham et al., Meth. Enz. 58:44 (1979), Barnes et al., Anal. Biochem. 1 02:255 (1980), U.S. Patent Nos. 4,767,704; 4,657,866; 4,927,762; 4,560,655; or 5,122,469; WO 90/03430; WO 87/00195; or U.S. Patent Re. 30,985 may be used as culture media for the host cells. Any of these media may be supplemented as necessary with hormones and/or other growth factors (such as insulin, transferrin, or epidermal growth factor), salts (such as sodium chloride, calcium, magnesium, and phosphate), buffers (such as HEPES), nucleotides (such as adenosine and thymidine), antibiotics (such as GENTAMYCIN.TM.drug), trace elements (defined as inorganic compounds usually present at final concentrations in the micromolar range), and glucose or an equivalent energy source. Any other necessary supplements may also be included at appropriate concentrations that would be known to those skilled in the art. The culture conditions, such as temperature, pH, and the like, are those previously used with the host cell selected for expression, and will be apparent to the ordinarily skilled artisan.

When using recombinant techniques, the antibody can be produced intracellularly, in the periplasmic space, or directly secreted into the medium. If the antibody is produced intracellularly, as a first step, the particulate debris, either host cells or lysed fragments, is removed, for example, by centrifugation or ultrafiltration. Carter et al., Bio/Technology 10:163-167 (1992) describe a procedure for isolating antibodies which are secreted to the periplasmic space of *E. coli.* Briefly, cell paste is thawed in the presence of sodium acetate (pH 3.5), EDTA, and phenylmethylsulfonylfluoride (PMSF) over about 30 min. Cell debris can be removed by centrifugation. Where the antibody is secreted into the medium, supernatants from such expression systems are generally first concentrated using a commercially available protein concentration filter, for example, an Amicon or Millipore Pellicon ultrafiltration unit. A protease inhibitor such as PMSF may be included in any of the foregoing steps to inhibit proteolysis and antibiotics may be included to prevent the growth of adventitious contaminants.

The antibody composition prepared from the cells can be purified using, for example, hydroxylapatite chromatography, gel electrophoresis, dialysis, and affinity chromatography, with affinity chromatography being the preferred purification technique. The suitability of protein A as an affinity ligand depends on the species and isotype of any immunoglobulin Fc domain that is present in the antibody. Protein A can be used to purify antibodies that are based on human .gamma.1, .gamma.2, or .gamma.4 heavy chains (Lindmark et al., J. Immunol. Meth. 62:1-13 (1983)). Protein G is recommended for all mouse isotypes and for human .gamma.3 (Guss et al., EMBO J. 5:15671575 (1986)). The matrix to which the affinity ligand is attached is most often agarose, but other matrices are available. Mechanically stable matrices such as controlled pore glass or poly(styrenedivinyl)benzene allow for faster flow rates and shorter processing times than can be achieved with agarose. Where the antibody comprises a C.sub.H3 domain, the Bakerbond ABX.TM.resin (J. T. Baker, Phillipsburg, N.J.) is useful for purification. Other techniques for protein purification such as fractionation on an ion-exchange column, ethanol precipitation, Reverse Phase HPLC, chromatography on silica, chromatography on heparin SEPHAROSE.TM. chromatography on an anion or cation exchange resin (such as a polyaspartic acid column), chromatofocusing, SDS-PAGE, and ammonium sulfate precipitation are also available depending on the antibody to be recovered. Following any preliminary purification step(s), the mixture comprising the antibody of interest and contaminants may be subjected to low pH hydrophobic interaction chromatography using an elution buffer at a pH between about 2.5-4.5, preferably performed at low salt concentrations (*e.g.,* from about 0-0.25 M salt).

### C. Pharmaceutical Formulations

Therapeutic formulations comprising the therapeutic agents, antagonists or antibodies of the invention are prepared for storage by mixing the antibody having the desired degree of purity with optional physiologically acceptable carriers, excipients or stabilizers (Remington's Pharmaceutical Sciences 16th edition, Osol, A. Ed. (1980)), in the form of aqueous solutions, lyophilized or other dried formulations. Acceptable carriers, excipients, or stabilizers are nontoxic to recipients at the dosages and concentrations employed, and include buffers such as phosphate, citrate, histidine and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g., Zn-protein complexes); and/or non-ionic surfactants such as TWEEN.TM., PLURONICS.TM. or polyethylene glycol (PEG).

The formulation herein may also contain more than one active compound as necessary for the particular indication being treated, preferably those with complementary activities that do not adversely affect each other. Such molecules are suitably present in combination in amounts that are effective for the purpose intended.

The active ingredients may also be entrapped in microcapsule prepared, for example, by coacervation techniques or by interfacial polymerization, for example, hydroxymethylcellulose or gelatin-microcapsule and poly-(methylmethacylate) microcapsule, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

The formulations to be used for *in vivo* administration must be sterile. This is readily accomplished by filtration through sterile filtration membranes.

Sustained-release preparations may be prepared. Suitable examples of sustained-release preparations include semipermeable matrices of solid hydrophobic polymers containing the immunoglobulin of the invention, which matrices are in the form of shaped articles, e.g., films, or microcapsule. Examples of sustained-release matrices include polyesters, hydrogels (for example, poly(2-hydroxyethyl-methacrylate), or poly(vinylalcohol)), polylactides (U.S. Patent No. 3,773,919), copolymers of L-glutamic acid and .gamma. ethyl-L-glutamate, non-degradable ethylene-vinyl acetate, degradable lactic acid-glycolic acid copolymers such as the LUPRON DEPOT.TM. (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods. When encapsulated immunoglobulins remain in the body for a long time, they may denature or aggregate as a result of exposure to moisture at 37°C., resulting in a loss of biological activity and possible changes in immunogenicity. Rational strategies can be devised for stabilization depending on the mechanism involved. For example, if the aggregation mechanism is discovered to be intermolecular S--S bond formation through thio-disulfide interchange, stabilization may be achieved by modifying sulfhydryl residues, lyophilizing from acidic solutions, controlling moisture content, using appropriate additives, and developing specific polymer matrix compositions.

### D. Treatment Methods

The integrin beta7 antagonists, such as anti-beta7 antibodies of the invention (and adjunct therapeutic agents) are administered by any suitable means, including parenteral, subcutaneous, intraperitoneal, intrapulmonary, and intranasal, and, if desired for local treatment, intralesional administration. Parenteral infusions include intramuscular, intravenous, intraarterial, intraperitoneal, or subcutaneous administration. In addition, the antibody is suitably administered by pulse infusion, particularly with declining doses of the antibody. Dosing can be by any suitable route, for example by injections, such as intravenous or subcutaneous injections, depending in part on whether the administration is brief or chronic.

The therapeutic agents of the invention will be formulated and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The therapeutic agent need not be, but is optionally formulated with one or more agents currently used to prevent or treat the disorder in question.

The standard of care for subjects with active moderate-severe active UC involves therapy with standard doses of: an aminosalicylate, an oral corticosteroid, 6-mercaptopurine (6-MP) and/or azathioprine. Therapy with an integrin beta7 antagonist, such as an anti-beta7 integrin antibody as disclosed herein will result in an improvement in disease remission (rapid control of disease and/or prolonged remission), and/or clinical response, superior to that achieved with the standard of care for such subjects.

In one embodiment, the treatment of the present invention for inflammatory bowel disease (IBD) in a human subject with IBD comprises administering to the subject an effective amount of an therapeutic agent, such as an anti-beta7 integrin antibody, and further comprising administering to the subject an effective amount of a second medicament, that is an immunosuppressant, a pain-control agent, an antidiarrheal agent, an antibiotic, or a combination thereof.

In an exemplary embodiment, said secondary medicine is selected from the group consisting of an aminosalicylate, an oral corticosteroid, 6-mercaptopurine (6-MP) and azathioprine. In another exemplary embodiment, said secondary medicine is another integrin beta7 antagonist, such as another anti-beta7 integrin antibody or an antibody against a cytokine.

All these second medicaments may be used in combination with each other or by themselves with the first medicament, so that the expression "second medicament" as used herein does not mean it is the only medicament besides the first medicament, respectively. Thus, the second medicament need not be one medicament, but may constitute or comprise more than one such drug.

These second medicaments as set forth herein are generally used in the same dosages and with administration routes as used hereinbefore or about from 1 to 99% of the heretofore-employed dosages. If such second medicaments are used at all, optionally, they are used in lower amounts than if the first medicament were not present, especially in subsequent dosings beyond the initial dosing with the first medicament, so as to eliminate or reduce side effects caused thereby. For instance, therapy with a anti-beta7 integrin antibody herein permits tapering or discontinued administration of steroid.

Combined administration herein includes co-administration, using separate formulations or a single pharmaceutical formulation, and consecutive administration in either order, wherein preferably there is a time period while both (or all) active agents simultaneously exert their biological activities.

The combined administration of a second medicament includes co-administration (concurrent administration), using separate formulations or a single pharmaceutical formulation, and consecutive administration in either order, wherein preferably there is a time period while both (or all) active agents (medicaments) simultaneously exert their biological activities.

### E. FACS Analysis

Various lymphocyte populations are identified by the expression levels of a combination of biomarkers, for example, but not limited to, CD4, CD45RA and Beta7, using the techniques available in the art, for example, flow cytometry analysis (FACS), etc. The subset population of lymphocytes with different expression patterns of these markers are identified with the standard techniques used in the art, such as flow cytometry (FACS).

Fluorescence-activated cell sorting (FACS) provides a method for sorting a heterogeneous mixture of biological cells into two or more containers, one cell at a time, based upon the specific light scattering and florescent characteristics of each cell. It is a useful scientific instrument as it provides fast, objective and quantitative recording of fluorescent signals from individual cells as well as physical separation of cells of particular interest. The cell suspension is entrained in the center of a narrow, rapidly flowing stream of liquid. The flow is arranged so that there is a large separation between cells relative to their diameter. A vibrating mechanism causes the stream of cells to break into individual droplets. The system is adjusted so that there is a low probability of more than one cell being in a droplet. Just before the stream breaks into droplets the flow passes through a fluorescence measuring station where the fluorescent character of interest of each cell is measured. An electrical charging ring is placed just at the point where the stream breaks into droplets. A charge is placed on the ring based on the immediately prior fluorescence intensity measurement and the opposite charge is trapped on the droplet as it breaks from the stream. The charged droplets then fall through an electrostatic deflection system that diverts droplets into containers based upon their charge. In some systems the charge is applied directly to the stream and the droplet breaking off retains charge of the same sign as the stream. The stream is then returned to neutral after the droplet breaks off.

The data generated by flow-cytometers can be plotted in a single dimension to produce a histogram, or in two dimensional dot plots or even in three dimensions. The regions on these plots can be sequentially separated, based on fluorescence intensity, by creating a series of subset extractions, termed "gates." Specific gating protocols exist for diagnostic and clinical purposes. The plots are often made on logarithmic scales. Because different fluorescent dyes' emission spectra overlap signals at the detectors have to be compensated electronically as well as computationally. Often, data accumulated using the flow cytometer can be re-analyzed elsewhere freeing up the machine for other people to use (Loken MR. "Immunofluorescence Techniques in Flow Cytometry and Sorting": 341-53. Wiley. (1990).

The amount of various lymphocytes can be quantified in various ways in the art. Absolute counts as percentage of baseline levels at predose can be calculated for lymphocyte subsets at each time point in the samples collected from a patient. Also can be calculated are the absolute counts for each respective subset of lymphocytes, which equal to the absolute lymphocyte counts (a value obtained from hematology measurements expressed as lymphocytes per microliter of peripheral blood) times the percentage of gated lymphocytes for each subset (obtained from flow cytometry analysis).

### F. Design Treatment Regimens

Drug development is a complex and expensive process. The cost of bringing a new drug to market is estimated to be $1 billion or more. Less than 10% of drugs in phase I clinical trials make it to the approval phase. Two key reasons why drugs fail at late stages are a lack of understanding of the relationship between dose-concentration response and unanticipated safety events. Given this scenario, it is critical to have enabling tools that help predict how a drug will perform *in vivo* and assist in the success of a clinical therapeutic candidate (Lakshmi Kamath, Drug Discovery and Development; Modeling Success in PK/PD Testing Drug Discovery & Development (2006)).

Pharmacokinetics (PK) characterizes the absorption, distribution, metabolism, and elimination properties of a drug. Pharmacodynamics (PD) defines the physiological and biological response to the administered drug. PK/PD modeling establishes a mathematical and theoretical link between these two processes and helps better predict drug action. Integrated PK/PD modeling and computer-assisted trial design via simulation are being incorporated into many drug development programs and are having a growing impact (Lakshmi Kamath, Drug Discovery and Development; Modeling Success in PK/PD Testing Drug Discovery & Development (2006)).

PK/PD testing is typically performed at every stage of the drug development process. Because development is becoming increasingly complex, time consuming, and cost intensive, companies are looking to make better use of PK/PD data to eliminate flawed candidates at the beginning and identify those with the best chance of clinical success. (Lakshmi Kamath, *supra*)*.*

PK/PD modeling approaches are proving useful in determining relationships between biomarker response, drug levels, and dosing regimens. The PK/PD profile of a drug candidate and the ability to predict a patient's response to it are critical to the success of clinical trials. Recent advances in molecular biology techniques and a better understanding of targets for various diseases have validated biomarkers as a good clinical indicator of a drug's therapeutic efficacy. Biomarker assays help identify a biological response to a drug candidate. Once a biomarker is clinically validated, trial simulations can be effectively modeled. Biomarkers have the potential to achieve surrogate status that may someday substitute for clinical outcomes in drug development. (Lakshmi Kamath, *supra*)*.*

Further details of the invention are illustrated by the following non-limiting Examples.

### EXAMPLES

### Example 1

### PHASE II RANDOMIZED DOUBLE-BLIND PLACEBO-CONTROLLED STUDY TO EVALUATE THE EFFICACY AND SAFETY OF rhuMAb BETA7 (ETROLIZUMAB) IN PATIENTS WITH MODERATE TO SEVERE ULCERATIVE COLITIS

### Description of the Clinical Study

### Description of rhuMAb Beta7 (etrolizumab)

RhuMAb Beta7 (etrolizumab) is a humanized monoclonal antibody based on the human IgG1 subgroup III V_{H}, κ subgroup-I V_{L} consensus sequences and is directed specifically against the β7 subunit of the integrin heterodimer. See Figs. 1A and B. It has been shown to bind with high affinity to α4β7 (K_{d} of about 116 pM) and αEβ7 (K_{d} of about 1800 pM).

This recombinant antibody has two heavy chains (446 residues) and two light chains (214 residues) that are covalently linked by interchain and intrachain disulfide bonds typical of IgG1 antibodies. For the work described herein, it was produced in Chinese hamster ovary (CHO) cells. The molecular mass of the intact, nonglycosylated rhuMAb Beta7 molecule was approximately 144 kDa. Each heavy chain of rhuMAb Beta7 has one conserved N linked glycosylation site at Asn297. The oligosaccharides present at this site were typical of those observed in recombinant antibodies expressed in CHO cells, with the predominant glycoforms being the asialo, biantennary G0, and G1 glycans. The mass of the most prevalent rhuMAb Beta7 form containing two GO glycans and no C terminal lysine residues was approximately 147 kDa.

RhuMAb Beta7 drug product and placebo were prepared by Genentech. They were clear to slightly opalescent, colorless to slightly yellow aqueous solutions. Both solutions were sterile and preservative-free liquid intended for IV and SC administration.

### Study Design

### Description of the Study

This Phase II study was a randomized, double-blind, placebo-controlled multicenter study to evaluate the efficacy and safety across two rhuMAb Beta7 dose levels compared with placebo in patients with moderate to severe UC. The primary efficacy endpoint was evaluated at Week 10 (2 weeks after the final dose of study drug was administered) with a secondary efficacy endpoint at Week 6.

Patients were randomized in a 1:1:1 ratio across a dose range of rhuMAb Beta7 100 mg SC (flat dose) at Weeks 0, 4, and 8 (100 mg dose) and 420 mg SC (flat loading dose) at Week 0 followed by 300 mg SC at Weeks 2, 4, and 8 (referred to herein as "300 mg + LD," LD = loading dose) or matching placebo SC. The study schema is shown in Figure 2. The study was divided into a screening period of 0-35 days, a double-blind treatment period of 10 weeks, a safety follow-up period of 18 weeks, and a progressive multifocal leukoencephalopathy (PML) follow up period of 17 months (2 years after randomization).

The dose values provided in the preceding paragraph are nominal doses. The phase II dose administration used a vial and a syringe with a vial concentration of 150 mg/ml. In order to enable consistent accurate dose administration, 0.7 ml was the selected volume per subcutaneous (SC) injection. The actual drug amount therefore, for the nominal 100 mg dose arm was 105 mg (1 x 0.7 ml SC injection) and for the nominal 300 mg dose was 315 mg (3 x 0.7 ml SC injections). The actual loading dose of 420 mg was 420 mg (4 x 0.7 ml SC injections). All SC injections were administered into the abdomen. Accordingly, a dose of "100 mg" and a dose of "105 mg" are used interchangeably herein. In addition, a dose of "300 mg" and a dose of "315 mg" are used interchangeably herein.

To be eligible, patients must have had a minimum of a 12-week duration of UC diagnosed according to the American College of Gastroenterology (ACG) practice guidelines; that is, clinical and endoscopic evidence corroborated by a histopathology report, with evidence of moderate to severe disease as evidenced by, in certain instances an MCS of ≥ 5, or in certain instances, an MCS of ≥ 6, including an endoscopy subscore of ≥ 2; a rectal bleeding subscore of ≥ 1 (see Table 1); and endoscopic evidence of disease activity a minimum of 25 cm from the anal verge. Additional inclusion and exclusion criteria for this study are provided in Intn'l Patent Pub. No. WO/2012/135589.

**Table 1. Mayo Clinic Scoring System for Assessment of Ulcerative Colitis Activity.**

| | **Assessment Category** | | | |
|---|---|---|---|---|
| **Score** | **Stool frequency^{a}** | **Rectal bleeding^{b}** | **Findings on Endoscopy** | **Physician's global assessment^{c}** |
| 0 | normal no. of stools for this patient | no blood seen | normal or inactive disease | normal |
| 1 | 1 to 2 stools more than normal | streaks of blood with stool less than half the time | mild disease (erythema, decreased vascular pattern, mild friability) | mild disease |
| 2 | 3 to 4 stools more than normal | obvious blood with stool most of the time | moderate disease (marked erythema, lack of vascular pattern, friability, erosions) | moderate disease |
| 3 | 5 or more stools more than normal | blood alone passes | severe disease (spontaneous bleeding, ulceration) | severe disease |
| | subscore: 0 to 3 | subscore: 0 to 3 | subscore: 0 to 3 | subscore: 0 to 3 |

| | | | | |
|---|---|---|---|---|
| ^{a} Each patient serves as his or her own control to establish the degree of abnormality of the stool frequency. ^{b} The daily bleeding score represents the most severe bleeding of the day. ^{c} The physician's global assessment acknowledges the three other criteria, the patient's daily recollection of abdominal discomfort and general sense of well-being, and other observations, such as physical findings and the patient's performance status. | | | | |

Prior to randomization, patients must have been on stable doses of concomitant medications for UC. Oral 5-aminosalicylic acid (5-ASA) and immunosuppressant (azathioprine [AZA], 6-mercaptopurine [6-MP], or methotrexate) doses must have been kept stable for at least 4 weeks prior to randomization on Day 1. Patients who were receiving topical 5-ASA or corticosteroids must have discontinued 2 weeks before randomization on Day 1. Oral corticosteroid doses must have been kept stable for 2 weeks prior to randomization on Day 1. Patients receiving high-dose steroids must have had the dose reduced to ≤ 20 mg/day for 2 weeks prior to randomization on Day 1. For patients receiving oral corticosteroids during the study treatment period, tapering of steroids must have been commenced at Week 10 at a rate of a 5-mg prednisone or prednisone equivalent per week for 2 weeks and then at a rate of 2.5 mg prednisone or prednisone equivalent per week to discontinuation. For patients receiving oral immunosuppressants (other than oral corticosteroids), tapering of immunosuppressants must have been commenced at Week 8, and patients must have completely discontinued immunosuppressants by Week 10. Patients who have previously received anti-TNF therapy must have discontinued therapy for a minimum of 8 weeks prior to randomization to receive study drug on Day 1. If patients experienced persisting or increasing disease activity at any time during the study, rescue therapy in the form of an increase in steroids and or immunosuppressant dose may be increased or initiated according to the investigator's clinical judgment. Patients who required rescue therapy were permitted to remain in the study but discontinued study treatment and, during data analysis, were classified as having experienced treatment failure.

Patients were assessed to determine whether they failed to respond to conventional therapy, including at least one anti-TNF agent. As used herein, loss of response and/or intolerance to anti-TNF agents and immunosuppressants means the following. With respect to anti-TNF agents, loss of response and/or intolerance means that symptoms of active disease persist despite previous treatment with one or more of (a) infliximab: 5 mg/kg IV, 3 doses over 6 weeks with assessment at 8 weeks; (b) adalimumab: one 160-mg SC does at week 0, followed by one 80-mg dose at week 2 and then 40 mg at 4 and 6 weeks, with assessment at 8 weeks; or recurrent active symptoms during regularly scheduled maintenance dosing following a previous response (elective discontinuation of treatment by patient who has responded and did not lose response does not qualify); or history of intolerance to at least one ant-TNF antibody (including but not exclusive of or limited to infusion-related reaction or injection-site reaction, infection, congestive heart failure, demyelination). With respect to immunosuppressant agents, loss of response and/or intolerance means that symptoms of active disease persist despite previous treatment with one or more of azathioprine (≥ 1.5 mg/kg) or equivalent dose of 6-mercaptopurine mg/kg (≥ 0.75 mg/kg) or methotrexate, 25 mg SC/intramuscular (or as indicated) per week for at least 8 weeks; or history of intolerance of at least one immunosuppressive (including, but not exclusive of pancreatitis, drug fever, rash, nausea/vomiting, liver function test elevation, thiopurine S-methyltransferase genetic mutation, infection).

Randomization to study treatment were stratified by concomitant treatment with corticosteroids (yes/no), concomitant treatment with immunosuppressants (yes/no), previous anti-TNF exposure (yes/no) (except for patients randomized in the United States), and study site.

UC disease activity was assessed using the MCS at Screening (and this was considered the baseline MCS), Week 6 (2 weeks after dosing at Week 4), and Week 10 (2 weeks after the final dose of study drug). Biopsies of the colon were obtained during the flexible sigmoidoscopy conducted at these same time points. Partial MCS was also collected throughout the study. Patient Reported Outcomes (PROs) were also assessed by using a Short Inflammatory Bowel Disease Questionnaire (SIBDQ) and MCS, which were to be completed by patients at Day 1 and at Weeks 6 and 10. In addition, disease activity, daily symptoms, and impact of UC were collected in a patient diary, to be completed daily by patients from screening (approximately 7 days prior to and up to Day 1) and at least 7 days prior to and up to the study visits at Weeks 6 and 10. Serum and fecal samples were also obtained for biomarker analysis. Stool was obtained at screening and Weeks 6, 10, and 28 for measurement of biomarkers. Exemplary biomarkers that were considered for measurement include, but are not limited to, lipocalin, calprotectin, and lactorferrin. Serum and plasma were obtained at screening, at Day 1, and at Weeks 4, 6, 10, 16, and 28 for measurement of exploratory biomarkers.

The primary efficacy endpoint for this study was the proportion of patients who achieved clinical remission, defined as an absolute reduction in MCS to ≤ 2 with no individual subscore exceeding 1 point, by Week 10. Additional secondary endpoints are listed in the study outcome measures as described below.

### Outcome Measures

### Primary Efficacy Outcome Measure

The primary efficacy outcome measure was clinical remission at Week 10. Clinical remission is defined by an MCS ≤ 2 with no individual subscore exceeding 1 point (see Table 1).

### Secondary Efficacy Outcome Measures

The secondary efficacy outcome measures for this study were (1) Clinical response at Week 6 and Week 10 where clinical response was defined by at least a 3-point decrease and 30% reduction from baseline in MCS and a ≥ 1-point decrease in rectal bleeding subscore or absolute rectal bleeding score of 0 or 1; (2) Clinical remission (defined above) at Week 6; and (3) An indicator for endoscopic score and rectal bleeding score of 0 at Week 6 and Week 10.

### Exploratory Outcome Measures

The exploratory outcome measures for this study were the time to flare of UC in patients who achieved response or remission. For this outcome measure, a flare is defined as a 2 point increase in partial MCS accompanied by 3 days of continuous rectal bleeding and an endoscopy score of 2 on flexible sigmoidoscopy.

### Safety Outcome Measures

The safety and tolerability of rhuMAb Beta7 were assessed using the following measures: (1) Incidence of adverse events and serious adverse events graded according to National Cancer Institute Common Terminology Criteria for Adverse Events (NCI CTCAE) Version 4.0; (2) Clinically significant changes in vital signs and safety laboratory measures; (3) Discontinuation due to adverse event(s); (4) Incidence and nature of injection-site reactions and hypersensitivity; (5) Incidence of infectious complications; and (6) Immunogenicity as measured by the incidence of ATAs.

### Pharmacokinetic Assays

Serum samples were obtained from all patients for determination and characterization of the PK of rhuMAb Beta7. Serum PK samples were analyzed using a validated bridging ELISA, with a minimum quantifiable concentration of 12.5 ng/mL. The assay method has been described previously (see, e.g., Intn'l Patent Pub. No. WO 2012/135589). Briefly, a sandwich ELISA with a colorimetric detection system was validated to quantify rhuMAb Beta7 (PRO145223) in human serum. Microtiter plates were coated with an anti-rhuMAb Beta7 antibody at 1.0 µg/mL to capture rhuMAb Beta7. Diluted samples, standards, and controls were added to the plate and incubated. Subsequently, biotinylated anti-rhuMAb Beta7 and streptavidin conjugated to horseradish peroxidase (HRP) were added for detection and incubated. A peroxidase substrate (tetramethyl benzidine) was added to develop color, and the reaction was stopped by adding 1 M phosphoric acid. The plates were read at 450 nm for detection absorbance and at 620 or 630 nm for reference absorbance.

### Pharmacodynamic Assays

To evaluate etrolizumab pharmacodynamic (PD) effects, two different assays, each using an anti-beta7 antibody, were employed. The first assay, referred to herein as an "occupancy assay" and shown schematically in Fig. 2A was performed essentially as described previously (see, e.g., Intn'l Patent Pub. No. WO 2012/135589), with the exception that a different competing anti-beta7 antibody, FIB504, (which binds to the same epitope as rhuMAb Beta7) was used instead of rhuMabBeta7. Briefly, peripheral blood samples from each of the patients were collected at certain time points throughout the study. Samples were collected in sodium heparin vacutainer tubes and shipped at room temperature overnight to contract testing facility for assessment. Samples were aliquotted, lysed, washed and stained with the fluorescently labeled anti-Beta7 antibody, FIB504 (BD Biosciences, San Jose, CA), which does not bind to β7 integrin in the presence of etrolizumab. Antibodies to CD3, CD4, CD45RA, CD19, CD38 and IgD were also added to identify the specific subsets of T and B lymphocytes. Samples were then acquired by BD FACS Canto and analyzed by gating on subsets of lymphocytes. The subsets are described further in Table 2 below.

**Table 2. Subsets of lymphocytes.**

| **Lymphocyte Subset** | **Cell Surface Markers** |
|---|---|
| T Helper lymphocytes | CD3+, CD4+ |
| Cytotoxic T lymphocytes | CD3+, CD4- |
| Naive T lymphocytes | CD45RA+, CD3+ |
| Systemic (peripheral) homing T lymphocytes | CD45RA-, CD3+, beta7low |
| Mucosal (gut) homing T lymphocytes | CD45RA-, CD3+, beta7high |
| Naive B lymphocytes | IgD+, CD19+ |
| Systemic (peripheral) B homing lymphocytes | IgD-, CD19+, beta7low |
| Mucosal (gut) B homing lymphocytes | IgD-, CD19+, beta7high |

T lymphocytes as described in Table 2 above were in certain experiments further analyzed as CD4+ or CD4-.

Binding of fluorescently labeled FIB504 was evaluated at 2 separate pre-dose time points (screen, and day 1 pre-dose) and at certain post dose time points throughout the study. The absolute number of T and B cell subsets with unoccupied β7 integrin was assessed at each study time point and expressed as a percentage of the respective predose baseline (%BL) or as change from baseline. Baseline was defined as the average of the predose (screening and Day 1 predose) values. For each cohort, the mean absolute counts of patients treated with etrolizumab or placebo was calculated.

The second assay, shown schematically in Fig. 2B and referred to herein as a "cell surface beta7 integrin detection assay" or "expression assay" was designed to assess the level of integrin beta7 present on the surface of lymphocytes before, during and/or after treatment with etrolizumab or placebo. The expression assay is also a method for assessing etrolizumab mechanism of action (MOA). This is because in the periphery, binding of etrolizumab to integrin alpha4beta7 blocks interaction with the ligand MAdCAM-1 and is thus hypothesized to block trafficking of gut homing lymphocytes to the gut resulting in a concomitant rise in gut homing lymphocytes in the periphery. Indeed, this has been observed and previously described in both pre-clinical and clinical studies. See, e.g, Intn'l Patent Pub. No. 2009/140684 and Stefanich et al., Br. J. Pharmacol. 162:1855-1870 (2011). Accordingly, the assay is also referred to as an "MOA assay." In addition, binding of etrolizumab to integrin alphaEbeta7 blocks interaction with the ligand E-cadherin and in the gut is hypothesized to inhibit retention of lymphocytes in the gut. Etrolizumab-mediated disruption of homing and retention of proinflammatory leukocytes within lymphoid tissue is hypothesized to lead to decreased inflammation in the gastrointestinal tract, thereby down-modulating disease symptoms characteristic of IBD, including those associated with UC and CD.

The beta7 expression assay (MOA assay) has been described previously. See, e.g., Intn'l Patent Pub. No. WO 2009/140684. Briefly, the number of β7 expressing T cells was evaluated by flow cytometry. The assay was conducted similarly to the occupancy assay as described above, with the exception of substitution of fluorescently conjugated anti-beta7 antibody known as 9D8, a non-competing anti-Beta7 antibody (i.e. capable of binding to β7 integrin in the presence of etrolizumab) for fluorescently labeled FIB504. The absolute number of T and B cell subsets with β7 integrin expressed on cell surface was assessed at each study time point and expressed as a percentage of the cell subset pre-dose baseline (%BL) or change from baseline.

To evaluate the levels of β7 integrin expression on the surface of T and B lymphocyte subsets, median fluorescence intensity (MFI) of cell surface β7 expression was assessed by flow cytometry and normalized to fluorescently labeled microbead standards of known fluorophore concentrations. Molecules of Equivalent Soluble Fluorochrome (MESF) of fluorescent 9D8 on cell surface of T and B cell subsets was evaluated at pre-dose and at subsequent post-dose time points. The MESF of β7 integrin expressed on the surface of T and B cell subsets was evaluated at each study time point and expressed as a percentage of that subset's pre-dose baseline (%BL) or change from baseline.

### Colonic Tissue Analysis

Etrolizumab binds the β7 subunits of the heterodimeric integrins α4β7 and αEβ7. While α4β7 is expressed on leukocyte subsets in both blood and mucosal tissue, αEβ7 is primarily confined to intraepithelial lymphocytes and dendritic cells in mucosal tissue. Therefore, to evaluate the pharmacology and mechanism of action of etrolizumab at the site of action as well as in circulation, colonic tissue was assessed in addition to peripheral blood. Colonic tissue biopsies were collected using standard surgical procedures from 23 consented, study-enrolled patients at pre-dose (screen) and at certain post dose time points (day 43 and day 71). Sample tissues collected were processed on-site by washing whole biopsies in sterile HBSS, reducing with HBSS+ 5mM DTT, then washing in culture medium and digesting with collagenase VIII (1.5 mg/ml). Samples were then filtered through a 40 µM nylon filter to remove tissue debris. Cell suspensions were washed with culture medium prior to staining with fluorescently labeled antibodies (noted below) for evaluation by flow cytometry.

Similar to the flow cytometry assay conducted on peripheral blood cells, occupancy of β7 integrin receptors on colonic tissue T lymphocyte subsets was assessed by staining with fluorescently labeled (PE label) FIB504, while expression of β7 integrin receptors was evaluated using fluorescently labeled 9D8 (PE label). Antibodies to CD3 (APC label), CD4 (PerCP label), CD45RA (FITC label), αE integrin (CD103) and α4 integrin (CD49d) were also added to identify specific subsets of colonic tissue T lymphocytes. Samples were then acquired on a BD FACS Cantoll^{™} or a BD FACSCalibur^{™} (BD Biosciences, San Jose, CA USA) and analyzed by gating on subsets of αEβ7-expressing and α4β7-expressing CD3+CD45+, αEβ7-expressing and α4β7-expressing CD3+CD4+CD45+, and αEβ7-expressing and α4β7-expressing CD3+CD4-CD45+ T cell subsets. Percentages of αEβ7+ and α4β7+ T cell subsets, as determined by FIB504 (to detect un-occupied β7) or 9D8 (to detect total cell surface β7), were assessed at each sample collection time point and expressed as a percentage of the cell subset pre-dose baseline (%BL) or as change from baseline.

Changes in the levels of αE and β7 integrin expression on surface of T lymphocyte subsets were also evaluated similar to blood (noted above). In brief, median fluorescence intensity (MFI) of cell surface αE and β7 expression was assessed by flow cytometry and normalized to fluorescently labeled microbead standards of known fluorophore concentrations. Molecules of Equivalent Fluorescence (MOEF) of fluorescent 9D8 and anti-αE on the cell surface of T cell subsets were evaluated at pre-dose and at subsequent post-dose time points. The MOEF of β7 integrin expressed on surface of T cell subsets was evaluated at each study time point and expressed as a percentage of the respective cell subset pre-dose baseline (%BL).

### Gene Expression Analysis by Quantitative Polymerase chain Reaction

RNA*later*^{®} biopsies were thawed and homogenized and ribonucleic acid (RNA) was isolated using the RNeasy^{®} Mini Kit (Qiagen, Hilden, Germany) according to the manufacturer's instructions. RNA integrity was assessed with the Agilent 2100 Bioanalyzer using the Agilent RNA 6000 Pico Kit (Agilent Technologies, Inc., Santa Clara, CA, USA). Samples with low RNA quality (RNA integrity number ≤5) were excluded from the analysis (n=9). RNA was reverse transcribed into complementary deoxyribonucleic acid using the High-Capacity cDNA Reverse Transcription Kit (Life Technologies Corporation, Carlsbad, CA, USA). Gene expression levels were assessed by real-time polymerase chain reaction, also referred to as quantitative polymerase chain reaction. Real-time polymerase chain reactions were run on the BioMark^{™} HD System (Fluidigm Corporation, South San Francisco, CA, USA) with TaqMan PreAmp Master Mix (Life Technologies Corporation, Carlsbad, CA, USA) and reagents (Fluidigm) using human integrin αE (*ITGAE*), integrin α4 (*ITGA4*), integrin β1 (*ITGB1*), β7 integrin (*ITGB7*), interleukin (*IL*) *17A, IL23A,* interferon γ (*IFNG*), *IL17F, IL1B, IL12B, IL6,* tumor necrosis factor α (*TNFA*)*,* mucosal addressin cell adhesion molecule-1 (*MADCAM1*), E-cadherin (*CDH1*), and glyceraldehyde 3-phosphate dehydrogenase (*GAPDH*) primer sets (all from Life Technologies Corporation, Carlsbad, CA, USA) according to manufacturer's instructions. *ITGAE, ITGA4, ITGB1, ITGB7, IL17A, IL23A, IFNG, IL17F, IL1B, IL12B, IL6, TNFA, MADCAM1,* and *CDH1* expression were normalized to GAPDH expression using the ΔCt method.

### Immunohistochemistry and Image Quantification

Formalin-fixed tissue samples were embedded in paraffin blocks and cut into 4 µM sections for staining. Staining was performed on a Benchmark XT (Ventana Medical Systems, Inc., Tucson, AZ, USA) autostainer with anti-integrin αE antibody (EPR4166; Abcam plc, Cambridge, MA, USA), developed with 3,3"-diaminobenzidine and counterstained with hematoxylin.

Whole slide images were acquired by the Olympus Nanozoomer automated slide scanning platform (Hamamatsu Photonics K.K., Bridgewater, NJ, USA) at 200x final magnification. Scanned slides were analyzed in the MATLAB^{®} software package (version R2012a; The MathWorks, Inc., Natick, MA, USA) as 24-bit RGB images. Total cells, αE⁺ cells, and αE⁺ cells associated with crypt epithelium were counted. Crypt epithelial areas were identified using a combination of support vector machines and genetic programming, within which individual cell nuclei were segmented, and then scored immunohistochemistry positive if ≥25% of their total area colocalized with 3,3"-diaminobenzidine.

### Results of PD Biomarker Assessments in Peripheral Blood

To conduct PD biomarker assessments, we first determined parameters to subdivide populations of lymphocytes as described in Table 2 and elsewhere above. Fig. 3A shows the phenotypic subdivision of peripheral blood CD3+CD4+ T cells based on surface expression of CD45RA and β7. CD3+CD4+ T cells were phenotypically subdivided according to their homing properties into subsets of CD3⁺CD4⁺CD45RA⁻Beta7^{ligh} T cells (mucosal-homing), CD4⁺CD45RA⁻Beta7^{low} T cells (peripheral homing), and CD4⁺CD45RA⁺ T cells (naive T cells that traffic well to both intestinal and peripheral lymph nodes and tissues). Similar phenotypic subdivisions were also evaluated on total CD3+ and CD3+CD4-T cells (not shown). These phenotypic subdivisions are consistent with those reported previously in the scientific literature. *See, e.g.,* Rott et al., J. Immunol 156:3727-3736 (1996); Rott et al., J. Clin Invest 100:1204-1208 (1997); Williams et al., J. Immunol. 161:4227-4235 (1998); Rose et al., J. Virol. 72:726-730 (1998); Williams et al., J. Immunol 159:1746-1752 (1997); and Butcher et al., Adv. Immunol. 72:209-253 (1999). We also subdivided populations B lymphocytes as shown in Fig. 3B. Here, phenotypic subdivision of peripheral blood CD19+ B cells was based on surface expression of IgD and β7. CD19+ B cells were phenotypically subdivided according to their homing properties into subsets of CD19⁺IgD⁻Beta7^{high} B cells (mucosal-homing), CD19⁺IgD⁻Beta7^{low} B cells (peripheral homing), and CD19⁺IgD⁺ B cells (naive B cells that traffic well to both intestinal and peripheral lymph nodes and tissues). After having established the parameters for subdividing populations of lymphocytes, we then proceeded to conduct PD biomarker assessments on patient samples from the Phase II study of etrolizumab described above.

Using the occupancy assay, we examined occupancy of beta7 integrin on peripheral blood T and B lymphocytes pre-dose and post-dose with etrolizumab or placebo according to the clinical study design described above. Fig. 4A shows the cohort mean (± SD) occupancy of β7 Integrin on CD3⁺CD4⁺CD45RA⁻Beta7^{high} T lymphocytes expressed as a percentage of baseline following subcutaneous (SC) administration of 100mg or 300mg+loading dose (LD) etrolizumab or placebo. Fig. 4B shows the cohort mean (± SD) occupancy of β7 Integrin on CD3⁺CD4⁻CD45RA⁻Beta7^{ligh} T lymphocytes expressed as a percentage of baseline following SC administration of 100mg or 300mg+LD etrolizumab or placebo. And Fig. 4C shows the cohort mean (± SD) occupancy of β7 Integrin on CD19+ IgD⁻Beta7^{high} B lymphocytes expressed as a percentage of baseline following SC administration of 100mg or 300mg+LD etrolizumab or placebo. Taken together, these data show that the mean absolute numbers of subsets of mucosal homing phenotype T cells (CD3+ CD4+ CD45RA- β7^{high} and CD3+ CD4- CD45RA- β7^{high} and mucosal homing phenotype B cells (CD19+ IgD- b7^{high}) decreased in all study cohorts following the once every 4 weeks (Q4W) administration of 100 mg or 300 mg+LD of etrolizumab, indicating occupancy of etrolizumab on the target cells. There was no apparent β7 occupancy in patients treated with placebo. In addition, etrolizumab given at the lower 100mg Q4W dose was sufficient to maintain maximal/near maximal occupancy of β7 receptors during the entire dosing phase.

Next, using the expression assay, we evaluated the numbers of beta7 expressing T and B lymphocytes circulating in the peripheral blood. The results are shown in Figs. 5A (gut homing CD3+ CD4+ T cells), 5B (gut homing CD3+ CD4- T cells) and 5C (gut homing CD19+ B cells). As can be seen, the median absolute numbers of subsets of peripheral blood T cells (CD3+CD4⁺CD45RA⁻β7^{high} and CD3+ CD4- CD45RA- b7^{high} "mucosal" homing phenotypes) and B cells (CD19+ IgD- b7^{high} 'mucosal' homing phenotype) increased in all study cohorts following the administration 100mg Q4W or 300mg+LD of etrolizumab, indicating that etrolizumab bound the target cells. There was no substantial increase in patients treated with placebo. When evaluating %baseline, the difference between etrolizumab vs. placebo for the mucosal-homing CD4+ and CD19+ cell subsets was statistically significant on days 29, 43, and 71 (p < 0.05, Kruskal Wallis ANOVA). Moreover, we observed that the elevation of mucosal homing T and B cell subsets in peripheral blood was similar in the 100mg Q4W compared to the 300mg+LD cohorts; there was no statistical difference between the 300 mg + LD and 100 mg dose groups for the CD4+, CD4-, and CD19+ subsets with the exception of CD19+ mucosal homing B cells (p < 0.03 on day 5, Kruskal Wallis ANOVA). We also observed similar elevations of the subsets described above in TNF-IR and TNF-naïve patients (data not shown) and when the data was plotted as absolute counts %BL rather than absolute counts change from baseline (data not shown). Together, these data suggest that the maximal increase may have been reached at the lower 100mg Q4W dose.

### Results of PD Biomarker Assessments in Colonic Tissue

We first established parameters for assessing αEβ7 expression on T lymphocytes obtained from colonic biopsy samples. Representative results are shown in Fig. 6. Fig. 6 shows, in a pre-dose sample, the phenotypic subdivision of colonic tissue CD45+CD3+CD4-T lymphocytes based on cell surface expression of αE and β7 (upper right quadrant, boxed section of plot). Similar phenotypic subdivision was also conducted on colonic tissue CD45+CD3+ and CD45+CD3+CD4+ T lymphocyte populations and similar results were observed (not shown), indicating that we could observe αEβ7 expression in all lymphocyte subsets.

We next assessed occupancy of αEβ7 receptors on CD45+CD3+CD4- T lymphocytes obtained from colonic biopsies taken from a representative patient treated with etrolizumab a single dose of etrolizumab, 100 mg, or from a representative patient treated with placebo. For each treatment arm, samples were obtained at pre-dose, at day 43 and at day 71. The results are shown in Fig. 7. Compared to predose levels, there was an observable decrease in the percentages of αEβ7+ cells at days 43 and 71 after treatment with etrolizumab (left-hand set of panels), confirming maximal receptor occupancy and consistent with the results described above. No such decrease was observed in the cells from the placebo-treated patient (right-hand set of panels) and consistent with the results described above. These data demonstrate that etrolizumab was present in the gut at the disease site where it remained bound to β7 receptors for the duration of this study, 71 days. We believe this is the first demonstration of αEβ7 receptor occupancy on T cells in colonic tissue.

In addition to the single patient analyses described above, we also assessed both αEβ7 occupancy and α4β7 occupancy in CD45+ CD3+ CD4- T lymphocytes obtained from colonic biopsies from each of 23 patients from the clinical study. Seven patients received 100 mg etrolizumab, seven patients received 300 mg + LD etrolizumab, and nine patients received placebo according to the administration schedule described above. The FACS plots of cohort medians at screen (pre-dose), at day 43 and at day 71 are shown in Figs. 8A (occupancy of αEβ7) and 8B (occupancy of α4β7). In each of Figs. 8A and 8B, the percentages of receptor positive T cells decreased in all patients following administration of either 100 mg etrolizumab q4w or 300 mg q4w + LD but there was no such decrease in placebo-treated patients. In addition, each of αEβ7 and α4β7 were maximally occupied, or nearly so, at both dose levels and at both time points examined after etrolizumab administration. We observed no apparent difference in occupancy in TNF-IR compared to TNF-naïve patients (data not shown). Accordingly, target engagement by etrolizumab in colonic tissue was confirmed.

### Additional PD Biomarker Assessments

As discussed above, etrolizumab maximally occupied β7 receptors on CD4⁺β7⁺ T lymphocytes at both the 100 mg and the 300 mg + LD doses, with a corresponding specific increase in CD4'07' T lymphocytes in the peripheral blood. Similar results were observed with CD19⁺β7⁺ B lymphocytes. In the colonic mucosa, maximal occupancy of β7 receptors also was observed at both doses. However, there was no difference in the overall relative frequencies of β7 expressing CD4⁻ T cells observed in etrolizumab-treated patients compared with placebo.

We also assessed the expression of integrins β7 (Fig. 9A), β1 (Fig. 9B), α4 (Fig. 9C) and αE (Fig. 9D) in colonic mucosa by qPCR. As shown in Fig. 9A, there was no apparent change in β7 gene expression observed by qPCR between etrolizumab-treated patients and placebo nor was there a change in expression between those etrolizumab-treated patients who achieved clinical remission compared to those who did not. Figs. 9B-9D show that there were minimal to no changes in β1-, α4-, and αE-integrin expression in colonic biopsy post etrolizumab in remitters.

On further assessment of the intestinal lymphocyte compartments, a decrease in the percentage of αE⁺ cells in the intestinal crypt epithelium was observed in etrolizumab-treated patients compared with placebo (Figs. 10A-B), with no apparent decrease in αE⁺ cells in the lamina propria (Figs. 11A-B). In addition, the number of epithelial cell-associated αE⁺ cells were reduced (Fig. 12A), and expression of E-cadherin was increased (Fig. 12B), in biopsies from etrolizumab-treated patients who achieved clinical remission compared with those who did not. An analysis of MAdCAM-1 expression, lymphocyte gene expression and inflammatory cytokine gene expression revealed reduced expression of the genes indicated in Fig. 13 in biopsies from etrolizumab-treated patients who achieved clinical remission compared with those who did not (Figs. 13A-M).

We interpret these results of the additional PD biomarker assessments as follows. Consistent with the role of β7 receptors in mediating lymphocyte trafficking to the intestine, there was a corresponding increased frequency of β7-expressing lymphocytes in the peripheral blood but not in the colonic mucosa. Although maximal β7 occupancy was observed for a minimum of 10 weeks in all etrolizumab-treated patients, clinical benefit was not observed in all patients, suggesting that the inflammatory process continues in some patients despite blockade of the β7 receptor. This could be explained by proinflammatory activity of immune cells already resident in the gut or β7-independent mechanisms of leukocyte trafficking to the intestinal mucosa. In support of this, in contrast to etrolizumab-treated patients who achieved clinical remission, no decrease was observed in lymphocyte gene expression in those who did not achieve clinical remission. Further understanding of alternative mechanisms of inflammation will require exploration in patients undergoing long-term therapy with etrolizumab.

While mucosal proinflammatory cytokine expression decreased in etrolizumab-treated patients who achieved clinical remission, expression of E-cadherin increased. E-cadherin has been shown to be expressed at lower levels in patients with inflammatory bowel disease compared with healthy controls (Arijs et al., Am J Gastroenterol 106:748-61 (2011)), suggesting that the observed increase in E-cadherin is related to mucosal healing in these patients. This observation is supported by the improvement in histologic disease activity score in patients who received etrolizumab.

### Dose and Dose Regimen Rationale

The rationale for the dosing regimen tested in the Phase II study was established following the Phase I study and was based on PK analysis of Phase I patient samples and population PK modeling as previously described. See, e.g., Intn'l Patent Pub. No. WO 2012/135589. Here, we wished to refine that analysis based on data obtained from patients in the Phase II study, particularly with respect to beta7 occupancy in colonic tissue.

Two of the patients in the study received only a single dose of etrolizumab and had provided colonic biopsy samples at predose and at certain time points following the dose as described further below. These patients are shown in Fig. 8C (dotted lines). When we analyzed receptor occupancy in colonic tissue as described above, we found that one of the two patients showed occupancy on day 43 but that occupancy was lost at day 71. In this patient, the serum drug levels were 4.7 µg/ml on day 43 and 0.5 µg/ml on day 71. In the second patient, we were unable to analyze occupancy on day 43 as sample was not available, but as for the first patient, the receptors were unoccupied on day 71. The serum drug level in this patient was below the limit of detection on day 71.

This result led us to compare the serum concentrations of etrolizumab in each of the 23 patients from whom colonic biopsies had been obtained to the level of beta7 occupancy on T cells obtained from those colonic biopsies at day 43 and at day 71. Those results are shown in Fig. 14. Occupancy of beta7 receptors on colonic tissue lymphocytes was observed in all patients whose serum etrolizumab concentration was 1.7 µg/ml or higher. The two patients who received only a single dose of etrolizumab and whose receptors were not occupied on day 71 had serum concentrations lower than 1 µg/ml. For each patient showing occupancy in colonic tissue, there was corresponding occupancy in the blood. Thus, we compared the relationship between serum drug level and receptor occupancy on lymphocytes in colonic tissue to the PK/PD relationships observed in peripheral blood in the Phase I trial. In the phase I trial, the predicted IC90 for receptor occupancy was determined to be 1.3 µg/ml, as previously described. This predicted IC90 is consistent with and essentially the same as the serum concentration required for occupancy in colonic tissue, as described here. Using these and other data from the Phase II study, we determined using population PK modeling (see, e.g., Intn'l Patent Pub. No. WO 2012/135589) that a dose of 100 mg every four weeks or a dose of 50 mg every two weeks is predicted to maintain a minimum drug serum concentration of 1.7 µg/mL in the majority of the tested patients population, which would be sufficient to maintain colonic tissue beta7 occupancy.

A surprising and unexpected finding from the results described above is that the level of occupancy of beta7 receptors on lymphocytes in the peripheral blood at a particular time following initiation of etrolizumab treatment (100 mg every 4 weeks) was essentially the same as the level of occupancy of beta7 receptors on lymphocytes in colonic tissue. This was surprising and unexpected because the large molecular weight of monoclonal antibodies (mAbs) limits their volume of distribution. Typically, the distribution of the mAbs is confined to vascular and interstitial spaces due to their large size and hydrophilicity. Hence the volume of distribution in the central compartment (Vc) for a mAb is usually equal to or slightly larger than the plasma volume (Mascelli et al., J. Clin Pharmacol 47; 553-565 (2007); Lobo et al., J. of Pharmaceutical Sci. Bol 93, 2645-2668 (2004)). Therefore, it is generally considered that the mAb concentration in the serum is not representative of the mAb concentration at the site of the action in the tissue.

Antibody distribution has been investigated more directly by assessing the concentrations of mAbs in tissues, where tissue samples are obtained by biopsy or necropsy. The distribution of mAbs has also been studied using radio-labeled antibody. For most antibodies reported in the literature, tissue to blood concentration ratios were found to be in the range of 0.1-0.5 (Lin et al., The J. of Pharmacol and Experi Thera Vol 288, 371-378 (1999); Lobo et al., J. of Pharmaceutical Sci. Bol 93, 2645-2668 (2004)). This means that, in most cases, the mAb concentration in the tissues (except brain tissue) is approximately 10-50% of that in the blood, while the mAb concentration in the brain is much less, ranging from 0.05-0.2% of those in the blood due to the protection by the blood brain barrier. Because it is generally considered that the mAb concentration in the serum is not representative of the mAb concentration at the site of the action, it may require up to 10-fold higher serum concentration to provide sufficient exposure to saturate the receptors inside the tissue. Yet, here we discovered that the serum concentration needed to saturate β7 receptors in the blood (1-3 µg/mL) was very similar to that needed to saturate the β7 receptors in the gut tissue (1.7 - 4 µg/mL) in UC patients (based on data from N=23 patients). We believe this is the first time that such a near one-to-one relationship between the blood and tissue compartments has been observed and described. There are a few possible explanations for this relationship. It is possible that the total amount of β7 receptors available in the blood (e.g. number of cells expressing β7 antigen) may be much greater than those in the tissue. Alternatively, it may be that the gut tissue in UC patients is "leakier" than normal tissue leading to more mAb distribution into the tissue under the same serum concentration.

In sum, based on the work described herein, beta7 receptor occupancy in the peripheral blood (an easily accessible site) can serve as a surrogate indicator of beta7 receptor occupancy in colonic tissue (a far less accessible site). This can be applied to the selection of dose and the design of dosing regimens for etrolizumab and other integrin beta7 antagonists. PK/PD relationships can be established by assessing serum drug concentrations and beta7 receptor occupancy in the blood to identify serum drug target concentrations sufficient for saturating receptors in the blood which can then be used to select doses or design dosing regimens with the knowledge that the same or nearly the same relationship will apply to the disease site in the colon thereby providing greater confidence in dose/dosing regimen selection.

Although the foregoing invention has been described in some detail by way of illustration and example for purposes of clarity of understanding, the descriptions and examples should not be construed as limiting the scope of the invention. The disclosures of all patent and scientific literature cited herein are expressly incorporated in their entirety by reference.

### Embodiments of the present invention are as follows:

1. A method of determining or monitoring the efficacy or, or of aiding in determining or monitoring the efficacy of, an integrin beta7 antagonist for treatment of a gastrointestinal inflammatory disorder in a patient, the method comprising comparing the amount of a biomarker in a sample obtained from the patient after or during treatment with the antagonist, to an amount of the biomarker in a sample obtained from the patient before the treatment, wherein a change in the amount of the biomarker after or during the treatment, as compared to before the treatment, is indicative of the efficacy of, or in combination with one or more additional biomarkers of efficacy is indicative of the efficacy of, the antagonist for treatment of the gastrointestinal disorder in the patient, and wherein the biomarker is integrin beta7 subunit-containing receptor occupancy by the antagonist on colonic lymphocytes, or gene expression levels of one or more integrin receptor ligands, or gene expression levels of one or more lymphocyte genes, or gene expression levels of one or more cytokines, or the number of alphaE-positive cells in intestinal crypt epithelium.
2. A method of determining or monitoring the responsiveness of, or of aiding in determining or monitoring the responsiveness of, a patient having a gastrointestinal inflammatory disorder to treatment with an integrin beta7 antagonist, the method comprising comparing the amount of a biomarker in a sample obtained from the patient after or during treatment with the antagonist, to the amount of the biomarker in a sample obtained from the patient before the treatment, wherein a change in the amount of the biomarker after or during the treatment, as compared to before the treatment is indicative of the responsiveness of, or in combination with one or more additional biomarkers of responsiveness is indicative of the responsiveness of, the patient to treatment with the antagonist, and wherein the biomarker is integrin beta7 subunit-containing receptor occupancy by the antagonist on colonic lymphocytes, or gene expression levels of one or more integrin receptor ligands, or gene expression levels of one or more lymphocyte genes, or gene expression levels of one or more cytokines, or the number of alphaE-positive cells in intestinal crypt epithelium.
3. A method of determining or monitoring the efficacy of an integrin beta7 antagonist for treatment of a gastrointestinal inflammatory disorder in an antagonist-treated patient in a placebo-controlled clinical trial, the method comprising comparing the amount of a biomarker in a sample obtained from the patient after or during treatment with the antagonist, to an amount of the biomarker in a sample obtained from a placebo-treated patient, wherein a change in the amount of the biomarker in the antagonist-treated patient after or during treatment, as compared to the amount of the biomarker in the placebo-treated patient, is indicative of the efficacy of the antagonist for treatment of the gastrointestinal disorder in the antagonist-treated patient, and wherein the biomarker is integrin beta7 subunit-containing receptor occupancy by the antagonist on colonic lymphocytes, or gene expression levels of one or more integrin receptor ligands, or gene expression levels of one or more lymphocyte genes, or gene expression levels of one or more cytokines, or the number of alphaE-positive cells in intestinal crypt epithelium.
4. A method of determining or monitoring the responsiveness of a patient having a gastrointestinal inflammatory disorder to treatment with an integrin beta7 antagonist, wherein the patient is in a placebo-controlled clinical trial, the method comprising comparing the amount of a biomarker in a sample obtained from the patient after or during treatment with the antagonist, to an amount of the biomarker in a sample obtained from a placebo-treated patient, wherein a change in the amount of the biomarker in the antagonist-treated patient after or during treatment, as compared to the amount of the biomarker in the placebo-treated patient, is indicative of the responsiveness of the patient to treatment with the antagonist, and wherein the biomarker is integrin beta7 subunit-containing receptor occupancy by the antagonist on colonic lymphocytes, or gene expression levels of one or more integrin receptor ligands, or gene expression levels of one or more lymphocyte genes, or gene expression levels of one or more cytokines, or the number of alphaE-positive cells in intestinal crypt epithelium.
5. A method of determining the dosing regimen of an integrin beta7 antagonist for treatment of a gastrointestinal inflammatory disorder in a patient, the method comprising adjusting the dose regimen of the antagonist based on a comparison of the amount of a biomarker in a sample obtained from the patient after or during treatment with a dosing regimen of the antagonist, to an amount of the biomarker in a sample obtained from the patient before the treatment, wherein a change in the amount of the biomarker after or during the treatment, as compared to before the treatment, is indicative of the efficacy of or responsiveness to the dose or dosing regimen of the antagonist for treatment of the gastrointestinal disorder in the patient, and wherein the biomarker is integrin beta7 subunit-containing receptor occupancy by the antagonist on colonic lymphocytes.
6. The method of any one of embodiments 1-5, wherein the biomarker is integrin beta7 subunit-containing receptor occupancy by the antagonist on colonic lymphocytes and the integrin beta7 subunit-containing receptor is αEβ7 receptor.
7. The method of any one of embodiments 1-5, wherein the biomarker is integrin beta7 subunit-containing receptor occupancy by the antagonist on colonic lymphocytes and the integrin beta7 subunit-containing receptor is α4β7 receptor.
8. The method of any one of embodiments 1-7, wherein the biomarker is integrin beta7 subunit-containing receptor occupancy by the antagonist on colonic lymphocytes and the integrin beta7 subunit-containing receptor occupancy on colonic lymphocytes is determined by measuring integrin beta7 subunit-containing receptor occupancy on peripheral blood lymphocytes, wherein the integrin beta7 subunit-containing receptor occupancy on peripheral blood lymphocytes was previously determined to be essentially the same as the integrin beta7 subunit-containing receptor occupancy on colonic lymphocytes.
9. The method of any one of embodiments 1-8, wherein the biomarker is integrin beta7 subunit-containing receptor occupancy by the antagonist on colonic lymphocytes and the occupancy of the integrin beta7 subunit-containing receptor is determined by a method comprising incubating the lymphocytes with labeled anti-beta7 antibody, wherein the labeled anti-beta7 antibody binds to the same epitope as the integrin beta7 antagonist, washing the lymphocytes, and measuring the percentage of labeled lymphocytes by flow cytometry.
10. The method of embodiment 9, wherein the label is selected from fluorescein isothiocyanate (FITC), rhodamine, phycoerythrin (PE), allophycocyanin (APC), peridinin chlorophyll protein (PerCP), PE-Cy7, APC-Cy7 and APC-H7.
11. The method of embodiment 9, wherein the integrin beta7 antagonist is etrolizumab and the labeled anti-beta7 antibody is etrolizumab or FIB504.
12. The method of any one of embodiments 1-9, wherein the change in the occupancy is an increase or decrease.
13. The method of any one of embodiments 1-4, wherein the biomarker is gene expression levels of one or more integrin receptor ligands and the integrin receptor ligand is MadCAM-1.
14. The method of embodiment 13, wherein the change in gene expression is a decrease.
15. The method of any one of embodiments 1-4, wherein the biomarker is gene expression levels of one or more integrin receptor ligands and the integrin receptor ligand is E-Cadherin.
16. The method of embodiment 15, wherein the change in gene expression is an increase.
17. The method of any one of embodiments 1-4, wherein the biomarker is gene expression levels of one or more lymphocyte genes and the one or more lymphocyte genes are selected from CD19, CD8, and CD3epsilon.
18. The method of embodiment 17, wherein the change in gene expression is a decrease.
19. The method of any one of embodiments 1-4, wherein the biomarker is gene expression levels of one or more cytokines and the one or more cytokines is selected from IL-1β, IL-6, IL-12-p40, IL-17A, IL-17-F, IL-23A, IPNγ and TNFα.
20. The method of embodiment 19, wherein the change in gene expression is a decrease.
21. The method of any one of embodiments 13-20, wherein the gene expression level is measured in colonic biopsy tissue.
22. The method of embodiment 21, wherein the gene expression level is measured by qPCR.
23. The method of any one of embodiments 1-4, wherein the biomarker is the number of alphaE-positive cells in the intestinal crypt epithelium and wherein the change in the number of alphaE-positive cells is a decrease.
24. The method of any one embodiments 1-23, wherein the biomarker is measured within 100 days after receiving a first dose of the antagonist.
25. The method of embodiment 24, wherein the biomarker is measured: (a) at day 43 and at day 71 or (b) at week 6 and at week 10.
26. The method of any one of embodiments 1-25, wherein the gastrointestinal inflammatory disorder is an inflammatory bowel disease.
27. The method of embodiment 26, wherein the inflammatory bowel disease is Crohn's disease (CD) or ulcerative colitis (UC).
28. The method of embodiment 27, wherein the patient is a human.
29. The method of any one of embodiments 1-10 or 12-28, wherein the integrin beta7 antagonist is an anti-beta7 antibody.
30. The method of embodiment 29, wherein the antibody is monoclonal.
31. The method of embodiment 30, wherein the antibody is a chimeric, human or humanized antibody.
32. The method of embodiment 31, wherein the antibody is an antibody fragment.
33. The method of embodiment 31, wherein the anti-beta7 antibody comprises six hypervariable regions (HVRs), wherein:
   (i) HVR-L1 comprises amino acid sequence A1-A11, wherein A1-A11 is RASESVDTYLH (SEQ ID NO:1); RASESVDSLLH (SEQ ID NO:7), RASESVDTLLH (SEQ ID NO:8), or RASESVDDLLH (SEQ ID NO:9) or a variant of SEQ ID NOs:1, 7, 8 or 9 (SEQ ID NO:26) wherein amino acid A2 is selected from the group consisting of A, G, S, T, and V and/or amino acid A3 is selected from the group consisting of S, G, I, K, N, P, Q, R, and T, and/or A4 is selected from the group consisting of E, V, Q, A, D, G, H, I, K, L, N, and R, and/or amino acid A5 is selected from the group consisting of S, Y, A, D, G, H, I, K, N, P, R, T, and V, and/or amino acid A6 is selected from the group consisting of V, R, I, A, G, K, L, M, and Q, and/or amino acid A7 is selected from the group consisting of D, V, S, A, E, G, H, I, K, L, N, P, S, and T, and/or amino acid A8 is selected from the group consisting of D, G, N, E, T, P and S, and/or amino acid A9 is selected from the group consisting of L, Y, I and M, and/or amino acid A10 is selected from the group consisting of L, A, I, M, and V and/or amino acid A11 is selected from the group consisting of H, Y, F, and S;
   (ii) HVR-L2 comprises amino acid sequence B1-B8, wherein B1-B8 is KYASQSIS (SEQ ID NO:2), RYASQSIS (SEQ ID NO:20), or XaaYASQSIS (SEQ ID NO:21, where Xaa represents any amino acid) or a variant of SEQ ID NOs:2, 20 or 21 (SEQ ID NO:27) wherein amino acid B1 is selected from the group consisting of K, R, N, V, A, F, Q, H, P, I, L, Y and Xaa (where Xaa represents any amino acid), and/or amino acid B4 is selected from the group consisting of S and D, and/or amino acid B5 is selected from the group consisting of Q and S, and/or amino acid B6 is selected from the group consisting of S, D, L, and R, and/or amino acid B7 is selected from the group consisting of I, V, E, and K;
   (iii) HVR-L3 comprises amino acid sequence C1-C9, wherein C1-C9 is QQGNSLPNT (SEQ ID NO:3) or a variant of SEQ ID NO:3 (SEQ ID NO:28) wherein amino acid C8 is selected from the group consisting of N, V, W, Y, R, S, T, A, F, H, I L, and M;
   (iv) HVR-H1 comprises amino acid sequence D1-D10 wherein D1-D10 is GFFITNNYWG (SEQ ID NO:4);
   (v) HVR-H2 comprises amino acid sequence E1-E17 wherein E1-E17 is GYISYSGSTSYNPSLKS (SEQ ID NO:5), or a variant of SEQ ID NO:5 (SEQ ID NO:29) wherein amino acid E2 is selected from the group consisting of Y, F, V, and D, and/or amino acid E6 is selected from the group consisting of S and G, and/or amino acid E10 is selected from the group consisting of S and Y, and/or amino acid E12 is selected from the group consisting of N, T, A, and D, and/or amino acid 13 is selected from the group consisting of P, H, D, and A, and/or amino acid E15 is selected from the group consisting of L and V, and/or amino acid E17 is selected from the group consisting of S and G; and
   (vi) HVR-H3 comprises amino acid sequence F2-F11 wherein F2 -F11 is MTGSSGYFDF (SEQ ID NO:6) or RTGSSGYFDF (SEQ ID NO:19); or comprises amino acid sequence F1-F11, wherein F1-F11 is AMTGSSGYFDF (SEQ ID NO:16), ARTGSSGYFDF (SEQ ID NO:17), or AQTGSSGYFDF (SEQ ID NO:18), or a variant of SEQ ID NOs:6, 16, 17, 18, or 19 (SEQ ID NO:30) wherein amino acid F2 is R, M, A, E, G, Q, S, and/or amino acid F11 is selected from the group consisting of F and Y.
34. The method of embodiment 33, wherein the anti-beta7 antibody comprises three heavy chain hypervariable region (HVR-H1-H3) sequences and three light chain hypervariable region (HVR-L1-L3) sequences, wherein:
   (i) HVR-L1 comprises SEQ ID NO:7, SEQ ID NO:8 or SEQ ID NO:9;
   (ii) HVR-L2 comprises SEQ ID NO:2;
   (iii) HVR-L3 comprises SEQ ID NO:3;
   (iv) HVR-H1 comprises SEQ ID NO:4;
   (v) HVR-H2 comprises SEQ ID NO:5; and
   (vi) HVR-H3 comprises SEQ ID NO:6 or SEQ ID NO:16 or SEQ ID NO:17 or SEQ ID NO:19.
35. The method of embodiment 34, wherein the anti-beta7 antibody comprises a variable light chain comprising the amino acid sequence of SEQ ID NO:31 and a variable heavy chain comprising the amino acid sequence of SEQ ID NO:32.
36. The method of any one of embodiments 29-35, wherein the anti-beta7 antibody is etrolizumab.
37. The method of embodiment 5, wherein the antagonist is an anti-beta7 antibody and the dosing regimen determined as indicative of the efficacy of or responsiveness to the dose or dosing regimen comprises subcutaneous administration of a first loading dose of 420 mg anti-beta7 antibody followed two weeks later by subcutaneous administration of a first maintenance dose of 315 mg anti-beta7 antibody followed by subcutaneous administration of one or more subsequent maintenance doses of 315 mg anti-beta7 antibody, wherein each subsequent maintenance dose is administered four weeks after the prior maintenance dose.
38. The method of embodiment 5, wherein the antagonist is an anti-beta7 antibody and the dosing regimen determined as indicative of the efficacy of or responsiveness to the dose or dosing regimen comprises subcutaneous administration of 105 mg anti-beta7 antibody every four weeks.
39. The method of embodiment 5, wherein the antagonist is an anti-beta7 antibody and the dosing regimen determined as indicative of the efficacy of or responsiveness to the dose or dosing regimen comprises subcutaneous administration of 50 mg anti-beta7 antibody every two weeks.
40. The method of any one of embodiments 37-39, wherein the anti-beta7 antibody is etrolizumab.
41. The method of embodiment 1 or embodiment 3, further comprising one or more clinical biomarkers of efficacy selected from clinical remission at week 6, clinical remission at week 10, clinical response at week 6, clinical response at week 10, endoscopy score and rectal bleeding score of 0 at week 6, endoscopy and rectal bleeding score of 0 at week 10, and time to flare of UC after achieving response or remission.
42. The method of embodiment 2 or embodiment 4, further comprising one or more clinical biomarkers of responsiveness selected from clinical remission at week 6, clinical remission at week 10, clinical response at week 6, clinical response at week 10, endoscopy score and rectal bleeding score of 0 at week 6, endoscopy and rectal bleeding score of 0 at week 10, and time to flare of UC after achieving response or remission.

## Claims

1. A method of determining or monitoring the efficacy or, or of aiding in determining or monitoring the efficacy of, an integrin beta7 antagonist for treatment of a gastrointestinal inflammatory disorder in a patient, the method comprising comparing the amount of a biomarker in a sample obtained from the patient after or during treatment with the antagonist, to an amount of the biomarker in a sample obtained from the patient before the treatment, wherein a change in the amount of the biomarker after or during the treatment, as compared to before the treatment, is indicative of the efficacy of, or in combination with one or more additional biomarkers of efficacy is indicative of the efficacy of, the antagonist for treatment of the gastrointestinal disorder in the patient, and wherein the biomarker is integrin beta7 subunit-containing receptor occupancy by the antagonist on colonic lymphocytes, or gene expression levels of one or more integrin receptor ligands, or gene expression levels of one or more lymphocyte genes, or gene expression levels of one or more cytokines, or the number of alphaE-positive cells in intestinal crypt epithelium.

2. A method of determining or monitoring the responsiveness of, or of aiding in determining or monitoring the responsiveness of, a patient having a gastrointestinal inflammatory disorder to treatment with an integrin beta7 antagonist, the method comprising comparing the amount of a biomarker in a sample obtained from the patient after or during treatment with the antagonist, to the amount of the biomarker in a sample obtained from the patient before the treatment, wherein a change in the amount of the biomarker after or during the treatment, as compared to before the treatment is indicative of the responsiveness of, or in combination with one or more additional biomarkers of responsiveness is indicative of the responsiveness of, the patient to treatment with the antagonist, and wherein the biomarker is integrin beta7 subunit-containing receptor occupancy by the antagonist on colonic lymphocytes, or gene expression levels of one or more integrin receptor ligands, or gene expression levels of one or more lymphocyte genes, or gene expression levels of one or more cytokines, or the number of alphaE-positive cells in intestinal crypt epithelium.

3. A method of determining or monitoring the efficacy of an integrin beta7 antagonist for treatment of a gastrointestinal inflammatory disorder in an antagonist-treated patient in a placebo-controlled clinical trial, the method comprising comparing the amount of a biomarker in a sample obtained from the patient after or during treatment with the antagonist, to an amount of the biomarker in a sample obtained from a placebo-treated patient, wherein a change in the amount of the biomarker in the antagonist-treated patient after or during treatment, as compared to the amount of the biomarker in the placebo-treated patient, is indicative of the efficacy of the antagonist for treatment of the gastrointestinal disorder in the antagonist-treated patient, and wherein the biomarker is integrin beta7 subunit-containing receptor occupancy by the antagonist on colonic lymphocytes, or gene expression levels of one or more integrin receptor ligands, or gene expression levels of one or more lymphocyte genes, or gene expression levels of one or more cytokines, or the number of alphaE-positive cells in intestinal crypt epithelium.

4. A method of determining or monitoring the responsiveness of a patient having a gastrointestinal inflammatory disorder to treatment with an integrin beta7 antagonist, wherein the patient is in a placebo-controlled clinical trial, the method comprising comparing the amount of a biomarker in a sample obtained from the patient after or during treatment with the antagonist, to an amount of the biomarker in a sample obtained from a placebo-treated patient, wherein a change in the amount of the biomarker in the antagonist-treated patient after or during treatment, as compared to the amount of the biomarker in the placebo-treated patient, is indicative of the responsiveness of the patient to treatment with the antagonist, and wherein the biomarker is integrin beta7 subunit-containing receptor occupancy by the antagonist on colonic lymphocytes, or gene expression levels of one or more integrin receptor ligands, or gene expression levels of one or more lymphocyte genes, or gene expression levels of one or more cytokines, or the number of alphaE-positive cells in intestinal crypt epithelium.

5. The method of any one of claims 1-4, wherein the biomarker is integrin beta7 subunit-containing receptor occupancy by the antagonist on colonic lymphocytes and the integrin beta7 subunit-containing receptor is αEβ7 receptor.

6. The method of any one of claims 1-4, wherein the biomarker is integrin beta7 subunit-containing receptor occupancy by the antagonist on colonic lymphocytes and the integrin beta7 subunit-containing receptor is α4β7 receptor.

7. The method of any one of claims 1-6, wherein the biomarker is integrin beta7 subunit-containing receptor occupancy by the antagonist on colonic lymphocytes and the integrin beta7 subunit-containing receptor occupancy on colonic lymphocytes is determined by measuring integrin beta7 subunit-containing receptor occupancy on peripheral blood lymphocytes, wherein the integrin beta7 subunit-containing receptor occupancy on peripheral blood lymphocytes was previously determined to be essentially the same as the integrin beta7 subunit-containing receptor occupancy on colonic lymphocytes.

8. The method of any one of claims 1-7, wherein the biomarker is integrin beta7 subunit-containing receptor occupancy by the antagonist on colonic lymphocytes and the occupancy of the integrin beta7 subunit-containing receptor is determined by a method comprising incubating the lymphocytes with labeled anti-beta7 antibody, wherein the labeled anti-beta7 antibody binds to the same epitope as the integrin beta7 antagonist, washing the lymphocytes, and measuring the percentage of labeled lymphocytes by flow cytometry.

9. The method of claim 8, wherein the label is selected from fluorescein isothiocyanate (FITC), rhodamine, phycoerythrin (PE), allophycocyanin (APC), peridinin chlorophyll protein (PerCP), PE-Cy7, APC-Cy7 and APC-H7.

10. The method of claim 8, wherein the integrin beta7 antagonist is etrolizumab and the labeled anti-beta7 antibody is etrolizumab or FIB504.

11. The method of any one of claims 1-8, wherein the change in the occupancy is an increase or decrease.

12. The method of any one of claims 1-4, wherein the biomarker is gene expression levels of one or more integrin receptor ligands and the integrin receptor ligand is MadCAM-1.

13. The method of claim 12, wherein the change in gene expression is a decrease.

14. The method of any one of claims 1-4, wherein the biomarker is gene expression levels of one or more integrin receptor ligands and the integrin receptor ligand is E-Cadherin.

15. The method of claim 14, wherein the change in gene expression is an increase.

16. The method of any one of claims 1-4, wherein the biomarker is gene expression levels of one or more lymphocyte genes and the one or more lymphocyte genes are selected from CD19, CD8, and CD3epsilon.

17. The method of claim 16, wherein the change in gene expression is a decrease.

18. The method of any one of claims 1-4, wherein the biomarker is gene expression levels of one or more cytokines and the one or more cytokines is selected from IL-1β, IL-6, IL-12-p40, IL-17A, IL-17-F, IL-23A, IPNγ and TNFα.

19. The method of claim 18, wherein the change in gene expression is a decrease.

20. The method of any one of claims 12-19, wherein the gene expression level is measured in colonic biopsy tissue.

21. The method of claim 20, wherein the gene expression level is measured by qPCR.

22. The method of any one of claims 1-4, wherein the biomarker is the number of alphaE-positive cells in the intestinal crypt epithelium and wherein the change in the number of alphaE-positive cells is a decrease.

23. The method of any one claims 1-22, wherein the biomarker is measured within 100 days after receiving a first dose of the antagonist.

24. The method of claim 23, wherein the biomarker is measured: (a) at day 43 and at day 71 or (b) at week 6 and at week 10.

25. The method of any one of claims 1-24, wherein the gastrointestinal inflammatory disorder is an inflammatory bowel disease.

26. The method of claim 25, wherein the inflammatory bowel disease is Crohn's disease (CD) or ulcerative colitis (UC).

27. The method of claim 26, wherein the patient is a human.

28. The method of any one of claims 1-9 or 11-27, wherein the integrin beta7 antagonist is an anti-beta7 antibody.

29. The method of claim 28, wherein the antibody is monoclonal.

30. The method of claim 29, wherein the antibody is a chimeric, human or humanized antibody.

31. The method of claim 30, wherein the antibody is an antibody fragment.

32. The method of claim 31, wherein the anti-beta7 antibody comprises six hypervariable regions (HVRs), wherein:
(i) HVR-L1 comprises amino acid sequence A1-A11, wherein A1-A11 is RASESVDTYLH (SEQ ID NO:1); RASESVDSLLH (SEQ ID NO:7), RASESVDTLLH (SEQ ID NO:8), or RASESVDDLLH (SEQ ID NO:9) or a variant of SEQ ID NOs:1, 7, 8 or 9 (SEQ ID NO:26) wherein amino acid A2 is selected from the group consisting of A, G, S, T, and V and/or amino acid A3 is selected from the group consisting of S, G, I, K, N, P, Q, R, and T, and/or A4 is selected from the group consisting of E, V, Q, A, D, G, H, I, K, L, N, and R, and/or amino acid A5 is selected from the group consisting of S, Y, A, D, G, H, I, K, N, P, R, T, and V, and/or amino acid A6 is selected from the group consisting of V, R, I, A, G, K, L, M, and Q, and/or amino acid A7 is selected from the group consisting of D, V, S, A, E, G, H, I, K, L, N, P, S, and T, and/or amino acid A8 is selected from the group consisting of D, G, N, E, T, P and S, and/or amino acid A9 is selected from the group consisting of L, Y, I and M, and/or amino acid A10 is selected from the group consisting of L, A, I, M, and V and/or amino acid A11 is selected from the group consisting of H, Y, F, and S;
(ii) HVR-L2 comprises amino acid sequence B1-B8, wherein B1-B8 is KYASQSIS (SEQ ID NO:2), RYASQSIS (SEQ ID NO:20), or XaaYASQSIS (SEQ ID NO:21, where Xaa represents any amino acid) or a variant of SEQ ID NOs:2, 20 or 21 (SEQ ID NO:27) wherein amino acid B1 is selected from the group consisting of K, R, N, V, A, F, Q, H, P, I, L, Y and Xaa (where Xaa represents any amino acid), and/or amino acid B4 is selected from the group consisting of S and D, and/or amino acid B5 is selected from the group consisting of Q and S, and/or amino acid B6 is selected from the group consisting of S, D, L, and R, and/or amino acid B7 is selected from the group consisting of I, V, E, and K;
(iii) HVR-L3 comprises amino acid sequence C1-C9, wherein C1-C9 is QQGNSLPNT (SEQ ID NO:3) or a variant of SEQ ID NO:3 (SEQ ID NO:28) wherein amino acid C8 is selected from the group consisting of N, V, W, Y, R, S, T, A, F, H, I L, and M;
(iv) HVR-H1 comprises amino acid sequence D1-D10 wherein D1-D10 is GFFITNNYWG (SEQ ID NO:4);
(v) HVR-H2 comprises amino acid sequence E1-E17 wherein E1-E17 is GYISYSGSTSYNPSLKS (SEQ ID NO:5), or a variant of SEQ ID NO:5 (SEQ ID NO:29) wherein amino acid E2 is selected from the group consisting of Y, F, V, and D, and/or amino acid E6 is selected from the group consisting of S and G, and/or amino acid E10 is selected from the group consisting of S and Y, and/or amino acid E12 is selected from the group consisting of N, T, A, and D, and/or amino acid 13 is selected from the group consisting of P, H, D, and A, and/or amino acid E15 is selected from the group consisting of L and V, and/or amino acid E17 is selected from the group consisting of S and G; and
(vi) HVR-H3 comprises amino acid sequence F2-F11 wherein F2 -F11 is MTGSSGYFDF (SEQ ID NO:6) or RTGSSGYFDF (SEQ ID NO: 19); or comprises amino acid sequence F1-F1 1, wherein F1-F1 1 is AMTGSSGYFDF (SEQ ID NO:16), ARTGSSGYFDF (SEQ ID NO:17), or AQTGSSGYFDF (SEQ ID NO:18), or a variant of SEQ ID NOs:6, 16, 17, 18, or 19 (SEQ ID NO:30) wherein amino acid F2 is R, M, A, E, G, Q, S, and/or amino acid F11 is selected from the group consisting of F and Y.

33. The method of claim 32, wherein the anti-beta7 antibody comprises three heavy chain hypervariable region (HVR-H1-H3) sequences and three light chain hypervariable region (HVR-L1-L3) sequences, wherein:
(i) HVR-L1 comprises SEQ ID NO:7, SEQ ID NO:8 or SEQ ID NO:9;
(ii) HVR-L2 comprises SEQ ID NO:2;
(iii) HVR-L3 comprises SEQ ID NO:3;
(iv) HVR-H1 comprises SEQ ID NO:4;
(v) HVR-H2 comprises SEQ ID NO:5; and
(vi) HVR-H3 comprises SEQ ID NO:6 or SEQ ID NO:16 or SEQ ID NO:17 or SEQ ID NO:19.

34. The method of claim 33, wherein the anti-beta7 antibody comprises a variable light chain comprising the amino acid sequence of SEQ ID NO:31 and a variable heavy chain comprising the amino acid sequence of SEQ ID NO:32.

35. The method of any one of claims 28-34, wherein the anti-beta7 antibody is etrolizumab.

36. The method of claim 1 or claim 3, further comprising one or more clinical biomarkers of efficacy selected from clinical remission at week 6, clinical remission at week 10, clinical response at week 6, clinical response at week 10, endoscopy score and rectal bleeding score of 0 at week 6, endoscopy and rectal bleeding score of 0 at week 10, and time to flare of UC after achieving response or remission.

37. The method of claim 2 or claim 4, further comprising one or more clinical biomarkers of responsiveness selected from clinical remission at week 6, clinical remission at week 10, clinical response at week 6, clinical response at week 10, endoscopy score and rectal bleeding score of 0 at week 6, endoscopy and rectal bleeding score of 0 at week 10, and time to flare of UC after achieving response or remission.


SEQUENCE LISTING

<110> GENENTECH, INC. ET AL.

<120> USE OF BIOMARKERS FOR ASSESSING TREATMENT OF GASTROINTESTINAL
      INFLAMMATORY DISORDERS WITH BETA7 INTEGRIN ANTAGONISTS

<130> P5599R1-WO

<140>
<141>

<150> 61/914,619
<151> 2013-12-11

<150> 61/805,860
<151> 2013-03-27

<160> 32

<170> PatentIn version 3.5

<210> 1
<211> 11
<212> PRT
<213> Artificial Sequence

<220>
<223> Description of Artificial Sequence: Synthetic
      peptide

<400> 1
Arg Ala Ser Glu Ser Val Asp Thr Tyr Leu His
1               5                   10


<210> 2
<211> 8
<212> PRT
<213> Artificial Sequence

<220>
<223> Description of Artificial Sequence: Synthetic
      peptide

<400> 2
Lys Tyr Ala Ser Gln Ser Ile Ser
1               5


<210> 3
<211> 9
<212> PRT
<213> Artificial Sequence

<220>
<223> Description of Artificial Sequence: Synthetic
      peptide

<400> 3
Gln Gln Gly Asn Ser Leu Pro Asn Thr
1               5

<210> 4
<211> 10
<212> PRT
<213> Artificial Sequence

<220>
<223> Description of Artificial Sequence: Synthetic
      peptide

<400> 4
Gly Phe Phe Ile Thr Asn Asn Tyr Trp Gly
1               5                   10


<210> 5
<211> 17
<212> PRT
<213> Artificial Sequence

<220>
<223> Description of Artificial Sequence: Synthetic
      peptide

<400> 5
Gly Tyr Ile Ser Tyr Ser Gly Ser Thr Ser Tyr Asn Pro Ser Leu Lys
1               5                   10                  15


Ser




<210> 6
<211> 10
<212> PRT
<213> Artificial Sequence

<220>
<223> Description of Artificial Sequence: Synthetic
      peptide

<400> 6
Met Thr Gly Ser Ser Gly Tyr Phe Asp Phe
1               5                   10


<210> 7
<211> 11
<212> PRT
<213> Artificial Sequence

<220>
<223> Description of Artificial Sequence: Synthetic
      peptide

<400> 7
Arg Ala Ser Glu Ser Val Asp Ser Leu Leu His
1               5                   10


<210> 8
<211> 11

<212> PRT
<213> Artificial Sequence

<220>
<223> Description of Artificial Sequence: Synthetic
      peptide

<400> 8
Arg Ala Ser Glu Ser Val Asp Thr Leu Leu His
1               5                   10


<210> 9
<211> 11
<212> PRT
<213> Artificial Sequence

<220>
<223> Description of Artificial Sequence: Synthetic
      peptide

<400> 9
Arg Ala Ser Glu Ser Val Asp Asp Leu Leu His
1               5                   10


<210> 10
<211> 108
<212> PRT
<213> Rattus sp.

<400> 10
Asp Val Val Met Thr Gln Ser Pro Ala Thr Leu Ser Val Thr Pro Gly
1               5                   10                  15

Glu Arg Ile Ser Leu Ser Cys Arg Ala Ser Glu Ser Val Asp Thr Tyr
            20                  25                  30

Leu His Trp Tyr Gln Gln Lys Pro Asn Glu Ser Pro Arg Leu Leu Ile
        35                  40                  45

Lys Tyr Ala Ser Gln Ser Ile Ser Gly Ile Pro Ser Arg Phe Ser Gly
    50                  55                  60

Ser Gly Ser Gly Thr Asp Phe Thr Leu Ser Ile Asn Gly Val Glu Leu
65                  70                  75                  80

Glu Asp Leu Ser Ile Tyr Tyr Cys Gln Gln Gly Asn Ser Leu Pro Asn
                85                  90                  95

Thr Phe Gly Ala Gly Thr Lys Leu Glu Leu Lys Arg
            100                 105


<210> 11
<211> 117
<212> PRT

<213> Rattus sp.

<400> 11
Glu Val Gln Leu Gln Glu Ser Gly Pro Gly Leu Val Lys Pro Ser Gln
1               5                   10                  15

Ser Leu Ser Leu Thr Cys Ser Val Thr Gly Phe Phe Ile Thr Asn Asn
            20                  25                  30

Tyr Trp Gly Trp Ile Arg Lys Phe Pro Gly Asn Lys Met Glu Trp Met
        35                  40                  45

Gly Tyr Ile Ser Tyr Ser Gly Ser Thr Ser Tyr Asn Pro Ser Leu Lys
    50                  55                  60

Ser Arg Ile Ser Ile Thr Arg Asp Thr Ser Lys Asn Gln Phe Phe Leu
65                  70                  75                  80

Gln Leu Asn Ser Val Thr Thr Glu Asp Thr Ala Thr Tyr Tyr Cys Ala
                85                  90                  95

Met Thr Gly Ser Ser Gly Tyr Phe Asp Phe Trp Gly Pro Gly Thr Met
            100                 105                 110

Val Thr Val Ser Ser
        115


<210> 12
<211> 108
<212> PRT
<213> Homo sapiens

<400> 12
Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Ser Ile Ser Asn Tyr
            20                  25                  30

Leu Ala Trp Tyr Gln Gln Lys Pro Gly Lys Ala Pro Lys Leu Leu Ile
        35                  40                  45

Tyr Ala Ala Ser Ser Leu Glu Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Asn Ser Leu Pro Trp
                85                  90                  95

Thr Phe Gly Gln Gly Thr Lys Val Glu Ile Lys Arg
            100                 105

<210> 13
<211> 113
<212> PRT
<213> Homo sapiens

<400> 13
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Val Ile Ser Gly Asp Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Gly Phe Asp Tyr Trp Gly Gln Gly Thr Leu Val Thr Val Ser
            100                 105                 110

Ser

<210> 14
<211> 108
<212> PRT
<213> Artificial Sequence

<220>
<223> Description of Artificial Sequence: Synthetic
      polypeptide

<400> 14
Asp Ile Gln Met Thr Gln Ser Pro Ser Ser Leu Ser Ala Ser Val Gly
1               5                   10                  15

Asp Arg Val Thr Ile Thr Cys Arg Ala Ser Glu Ser Val Asp Thr Tyr
            20                  25                  30

Leu His Trp Tyr Gln Gln Lys Pro Gly Lys Ala Pro Lys Leu Leu Ile

35                  40                  45

Lys Tyr Ala Ser Gln Ser Ile Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Gly Asn Ser Leu Pro Asn
                85                  90                  95

Thr Phe Gly Gln Gly Thr Lys Val Glu Ile Lys Arg
            100                 105

<210> 15
<211> 117
<212> PRT
<213> Artificial Sequence

<220>
<223> Description of Artificial Sequence: Synthetic
      polypeptide

<400> 15
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Phe Ile Thr Asn Asn
            20                  25                  30

Tyr Trp Gly Trp Val Arg Gln Ala Pro Gly Lys Gly Leu Glu Trp Val
        35                  40                  45

Gly Tyr Ile Ser Tyr Ser Gly Ser Thr Ser Tyr Asn Pro Ser Leu Lys
    50                  55                  60

Ser Arg Phe Thr Ile Ser Ala Asp Thr Ser Lys Asn Thr Ala Tyr Leu
65                  70                  75                  80

Gln Met Asn Ser Leu Arg Ala Glu Asp Thr Ala Val Tyr Tyr Cys Ala
                85                  90                  95

Met Thr Gly Ser Ser Gly Tyr Phe Asp Phe Trp Gly Gln Gly Thr Leu
            100                 105                 110

Val Thr Val Ser Ser
        115

<210> 16
<211> 11

<212> PRT
<213> Artificial Sequence

<220>
<223> Description of Artificial Sequence: Synthetic
      peptide

<400> 16
Ala Met Thr Gly Ser Ser Gly Tyr Phe Asp Phe
1               5                   10


<210> 17
<211> 11
<212> PRT
<213> Artificial Sequence

<220>
<223> Description of Artificial Sequence: Synthetic
      peptide

<400> 17
Ala Arg Thr Gly Ser Ser Gly Tyr Phe Asp Phe
1               5                   10


<210> 18
<211> 11
<212> PRT
<213> Artificial Sequence

<220>
<223> Description of Artificial Sequence: Synthetic
      peptide

<400> 18
Ala Gln Thr Gly Ser Ser Gly Tyr Phe Asp Phe
1               5                   10


<210> 19
<211> 10
<212> PRT
<213> Artificial Sequence

<220>
<223> Description of Artificial Sequence: Synthetic
      peptide

<400> 19
Arg Thr Gly Ser Ser Gly Tyr Phe Asp Phe
1               5                   10


<210> 20
<211> 8
<212> PRT
<213> Artificial Sequence

<220>
<223> Description of Artificial Sequence: Synthetic
      peptide

<400> 20
Arg Tyr Ala Ser Gln Ser Ile Ser
1               5

<210> 21
<211> 8
<212> PRT
<213> Artificial Sequence

<220>
<223> Description of Artificial Sequence: Synthetic
      peptide

<220>
<221> MOD_RES
<222> (1)..(1)
<223> Any amino acid

<400> 21
Xaa Tyr Ala Ser Gln Ser Ile Ser
1               5

<210> 22
<211> 108
<212> PRT
<213> Artificial Sequence

<220>
<223> Description of Artificial Sequence: Synthetic
      polypeptide

<400> 22
Asp Ile Gln Met Thr Gln Ser Pro Ser Ser Leu Ser Ala Ser Val Gly
1               5                   10                  15

Asp Arg Val Thr Ile Thr Cys Arg Ala Ser Glu Ser Val Asp Ser Leu
            20                  25                  30

Leu His Trp Tyr Gln Gln Lys Pro Gly Lys Ala Pro Lys Leu Leu Ile
        35                  40                  45

Lys Tyr Ala Ser Gln Ser Ile Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Gly Asn Ser Leu Pro Asn
                85                  90                  95

Thr Phe Gly Gln Gly Thr Lys Val Glu Ile Lys Arg
            100                 105

<210> 23
<211> 108
<212> PRT
<213> Artificial Sequence

<220>
<223> Description of Artificial Sequence: Synthetic
      polypeptide

<400> 23
Asp Ile Gln Met Thr Gln Ser Pro Ser Ser Leu Ser Ala Ser Val Gly
1               5                   10                  15

Asp Arg Val Thr Ile Thr Cys Arg Ala Ser Glu Ser Val Asp Thr Leu
            20                  25                  30

Leu His Trp Tyr Gln Gln Lys Pro Gly Lys Ala Pro Lys Leu Leu Ile
        35                  40                  45

Lys Tyr Ala Ser Gln Ser Ile Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Gly Asn Ser Leu Pro Asn
                85                  90                  95

Thr Phe Gly Gln Gly Thr Lys Val Glu Ile Lys Arg
            100                 105


<210> 24
<211> 108
<212> PRT
<213> Artificial Sequence

<220>
<223> Description of Artificial Sequence: Synthetic
      polypeptide

<400> 24
Asp Ile Gln Met Thr Gln Ser Pro Ser Ser Leu Ser Ala Ser Val Gly
1               5                   10                  15

Asp Arg Val Thr Ile Thr Cys Arg Ala Ser Glu Ser Val Asp Asp Leu
            20                  25                  30

Leu His Trp Tyr Gln Gln Lys Pro Gly Lys Ala Pro Lys Leu Leu Ile
        35                  40                  45

Lys Tyr Ala Ser Gln Ser Ile Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Gly Asn Ser Leu Pro Asn
                85                  90                  95

Thr Phe Gly Gln Gly Thr Lys Val Glu Ile Lys Arg
            100                 105

<210> 25
<211> 117
<212> PRT
<213> Artificial Sequence

<220>
<223> Description of Artificial Sequence: Synthetic
      polypeptide

<400> 25
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Phe Ile Thr Asn Asn
            20                  25                  30

Tyr Trp Gly Trp Val Arg Gln Ala Pro Gly Lys Gly Leu Glu Trp Val
        35                  40                  45

Gly Tyr Ile Ser Tyr Ser Gly Ser Thr Ser Tyr Asn Pro Ser Leu Lys
    50                  55                  60

Ser Arg Phe Thr Ile Ser Arg Asp Thr Ser Lys Asn Thr Phe Tyr Leu
65                  70                  75                  80

Gln Met Asn Ser Leu Arg Ala Glu Asp Thr Ala Val Tyr Tyr Cys Ala
                85                  90                  95

Met Thr Gly Ser Ser Gly Tyr Phe Asp Phe Trp Gly Gln Gly Thr Leu
            100                 105                 110

Val Thr Val Ser Ser
        115

<210> 26
<211> 11
<212> PRT
<213> Artificial Sequence

<220>
<223> Description of Artificial Sequence: Synthetic
      peptide

<220>
<221> MOD_RES
<222> (2)..(2)
<223> Ala, Gly, Ser, Thr or Val

<220>
<221> MOD_RES
<222> (3)..(3)
<223> Ser, Gly, Ile, Lys, Asn, Pro, Gln, Arg or Thr

<220>
<221> MOD_RES
<222> (4)..(4)
<223> Glu, Val, Gln, Ala, Asp, Gly, His, Ile, Lys, Leu, Asn
      or Arg

<220>
<221> MOD_RES
<222> (5)..(5)
<223> Ser, Tyr, Ala, Asp, Gly, His, Ile, Lys, Asn, Pro, Arg,
      Thr or Val

<220>
<221> MOD_RES
<222> (6)..(6)
<223> Val, Arg, Ile, Ala, Gly, Lys, Leu, Met or Gln

<220>
<221> MOD_RES
<222> (7)..(7)
<223> Asp, Val, Ser, Ala, Glu, Gly, His, Ile, Lys, Leu, Asn,
      Pro, Ser or Thr

<220>
<221> MOD_RES
<222> (8)..(8)
<223> Asp, Gly, Asn, Glu, Thr, Pro or Ser

<220>
<221> MOD_RES
<222> (9)..(9)
<223> Leu, Tyr, Ile or Met

<220>
<221> MOD_RES
<222> (10)..(10)
<223> Leu, Ala, Ile, Met or Val

<220>
<221> MOD_RES
<222> (11)..(11)
<223> His, Tyr, Phe or Ser

<400> 26
Arg Xaa Xaa Xaa Xaa Xaa Xaa Xaa Xaa Xaa Xaa
1               5                   10


<210> 27
<211> 8
<212> PRT

<213> Artificial Sequence

<220>
<223> Description of Artificial Sequence: Synthetic
      peptide

<220>
<221> MOD_RES
<222> (1)..(1)
<223> Any amino acid

<220>
<221> MOD_RES
<222> (4)..(4)
<223> Ser or Asp

<220>
<221> MOD_RES
<222> (5)..(5)
<223> Gln or Ser

<220>
<221> MOD_RES
<222> (6)..(6)
<223> Ser, Asp, Leu or Arg

<220>
<221> MOD_RES
<222> (7)..(7)
<223> Ile, Val, Glu or Lys

<220>
<223> see specification as filed for detailed description of
      substitutions and certain embodiments

<400> 27
Xaa Tyr Ala Xaa Xaa Xaa Xaa Ser
1               5

<210> 28
<211> 9
<212> PRT
<213> Artificial Sequence

<220>
<223> Description of Artificial Sequence: Synthetic
      peptide

<220>
<221> MOD_RES
<222> (8)..(8)
<223> Asn, Val, Trp, Tyr, Arg, Ser, Thr, Ala, Phe, His, Ile,
      Leu or Met

<400> 28
Gln Gln Gly Asn Ser Leu Pro Xaa Thr
1               5

<210> 29

<211> 17
<212> PRT
<213> Artificial Sequence

<220>
<223> Description of Artificial Sequence: Synthetic
      peptide

<220>
<221> MOD_RES
<222> (2)..(2)
<223> Tyr, Phe, Val or Asp

<220>
<221> MOD_RES
<222> (6)..(6)
<223> Ser or Gly

<220>
<221> MOD_RES
<222> (10)..(10)
<223> Ser or Tyr

<220>
<221> MOD_RES
<222> (12)..(12)
<223> Asn, Thr, Ala or Asp

<220>
<221> MOD_RES
<222> (13)..(13)
<223> Pro, His, Asp or Ala

<220>
<221> MOD_RES
<222> (15)..(15)
<223> Leu or Val

<220>
<221> MOD_RES
<222> (17)..(17)
<223> Ser or Gly

<400> 29
Gly Xaa Ile Ser Tyr Xaa Gly Ser Thr Xaa Tyr Xaa Xaa Ser Xaa Lys
1               5                   10                  15

Xaa

<210> 30
<211> 11
<212> PRT
<213> Artificial Sequence

<220>
<223> Description of Artificial Sequence: Synthetic
      peptide

<220>
<221> MOD_RES
<222> (1)..(1)
<223> May or may not be present

<220>
<221> MOD_RES
<222> (2)..(2)
<223> Arg, Met, Ala, Glu, Gly, Gln or Ser

<220>
<221> MOD_RES
<222> (11)..(11)
<223> Phe or Tyr

<400> 30
Ala Xaa Thr Gly Ser Ser Gly Tyr Phe Asp Xaa
1               5                   10


<210> 31
<211> 108
<212> PRT
<213> Artificial Sequence

<220>
<223> Description of Artificial Sequence: Synthetic
      polypeptide

<400> 31
Asp Ile Gln Met Thr Gln Ser Pro Ser Ser Leu Ser Ala Ser Val Gly
1               5                   10                  15


Asp Arg Val Thr Ile Thr Cys Arg Ala Ser Glu Ser Val Asp Asp Leu
            20                  25                  30


Leu His Trp Tyr Gln Gln Lys Pro Gly Lys Ala Pro Lys Leu Leu Ile
        35                  40                  45


Lys Tyr Ala Ser Gln Ser Ile Ser Gly Val Pro Ser Arg Phe Ser Gly
    50                  55                  60


Ser Gly Ser Gly Thr Asp Phe Thr Leu Thr Ile Ser Ser Leu Gln Pro
65                  70                  75                  80


Glu Asp Phe Ala Thr Tyr Tyr Cys Gln Gln Gly Asn Ser Leu Pro Asn
                85                  90                  95


Thr Phe Gly Gln Gly Thr Lys Val Glu Ile Lys Arg
            100                 105


<210> 32
<211> 117
<212> PRT
<213> Artificial Sequence

<220>
<223> Description of Artificial Sequence: Synthetic
      polypeptide

<400> 32